(19) Europäisches Patentamt
European Patent Office
Office européen des brevets

(11) Veröffentlichungsnummer: **0 115 473**
**A2**

(12) # EUROPÄISCHE PATENTANMELDUNG

(21) Anmeldenummer: **84810041.8**

(22) Anmeldetag: **23.01.84**

(51) Int. Cl.³: **C 07 C 103/52**
**A 61 K 37/02**

(30) Priorität: **27.01.83 CH 454/83**

(43) Veröffentlichungstag der Anmeldung:
**08.08.84 Patentblatt 84/32**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Bencze, William, Dr.**
**Scheltenstrasse 6**
**CH-4106 Therwil(CH)**

(72) Erfinder: **Kamber, Bruno, Dr.**
**Sonnenweg 9**
**CH-4144 Arlesheim(CH)**

(72) Erfinder: **Storni, Angelo, Dr.**
**Im Feuerbusch 3**
**CH-4310 Rheinfelden(CH)**

(54) **Substituierte Pyrrolidinonderivate und Verfahren zu ihrer Herstellung.**

(57) Die Erfindung betrifft Lactampeptide der Formel I und ihre Salze,

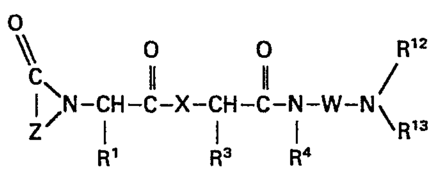

(I)

die zur Behandlung von zerebraler Leistungsinsuffizienz verwendet werden können, Verfahren und neue Zwischenprodukte zur Herstellung dieser Verbindungen, pharmazeutische Präparate, die diese Verbindungen, pharmazeutische Präparate, die diese Verbindungen enthalten, und ihre Verwendung zur Herstellung pharmazeutischer Präparate oder als pharmakologisch wirksame Verbindungen. In Formel I bedeuten Z einen Alkylenrest, der unsubstituiert oder durch freies oder funktionell abgewandeltes Hydroxy und/ oder durch Aryl substituiert ist und der zusammen mit der Amidgruppierung einen fünfgliedrigen Ring bildet,

$R^1$ Wasserstoff, unsubstituiertes oder durch Aryl oder durch freies oder funktionell abgewandeltes Hydroxy substituiertes Niederalkyl oder Aryl,

X Sauerstoff oder die Gruppe $NR^2$, worin $R^2$ für Wasserstoff oder Niederalkyl steht,

$R^3$ Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl oder Aryl oder $R^2$ and $R^3$ zusammen unsubstituiertes oder durch freies oder funktionell abgewandeltes Hydroxy substituiertes Trimethylen,

$R^4$ Wasserstoff oder Niederalkyl,

W einen jeweils mit dem Carbonyl-C-Atom an den Rest der Formel II

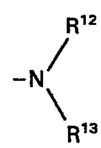

(II)

gebundenen Rest der Formeln III, IV, V oder VI,

./..

EP 0 115 473 A2

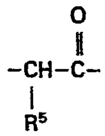

(III)

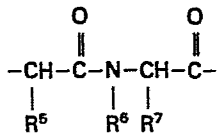

(IV)

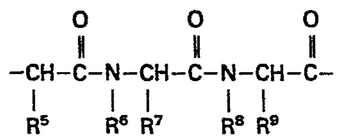

(V)

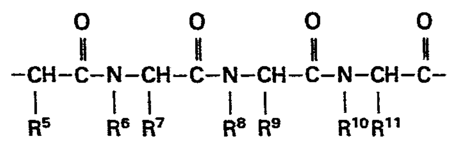

(VI)

worin $R^6$, $R^8$ und $R^{10}$ unabhängig voneinander für Wasserstoff oder Niederalkyl, $R^5$, $R^7$, $R^9$ und $R^{11}$ unabhängig voneinander für Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl oder Aryl oder $R^4$ und $R^5$ zusammen, $R^6$ und $R^7$ zusammen, $R^8$ und $R^9$ zusammen und/oder $R^{10}$ und $R^{11}$ zusammen jeweils unabhängig voneinander für unsubstituiertes oder durch freies oder funktionell abgewandeltes Hydroxy substituiertes Trimethylen stehen, und $R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen.

Nicht zum Gegenstand der Erfindung gehören Verbindungen der Formel I, worin Z unsubstituiertes Alkylen mit 3-C-Atomen und zugleich

- $R^1$ Wasserstoff, unsubstituiertes Alkyl mit 1-4 C-Atomen, Aryl oder Arylnierderalkyl,
- X den Rest NH,
- $R^3$ Aryl, unsubstituiertes Alkyl oder Wasserstoff
- $R^4$ Wasserstoff,
- W einen Rest der Formel III, worin $R^5$ eine mit dem Rest $R^3$ identische Bedeutung hat, und
- $R^{12}$ und $R^{13}$ Wasserstoff

bedeuten.

0115473



CIBA-GEIGY AG

Basel (Schweiz)

4-14299/+

Substituierte Pyrrolidinonderivate und Verfahren zu ihrer Herstellung

Die Erfindung betrifft Lactampeptide der Formel I,

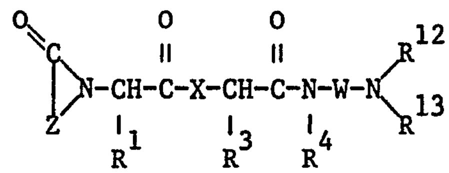

(I)

worin Z einen Alkylenrest bedeutet, der unsubstituiert oder durch freies oder funktionell abgewandeltes Hydroxy und/oder durch Aryl substituiert ist und der zusammen mit der Amidgruppierung einen fünfgliedrigen Ring bildet,

$R^1$ Wasserstoff, unsubstituiertes oder durch Aryl oder durch freies oder funktionell abgewandeltes Hydroxy substituiertes Niederalkyl oder Aryl,

X Sauerstoff oder die Gruppe $NR^2$, worin $R^2$ für Wasserstoff oder Niederalkyl steht,

$R^3$ Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl oder Aryl oder $R^2$ und $R^3$ zusammen unsubstituiertes oder durch freies oder funktionell abgewandeltes Hydroxy substituiertes Trimethylen,

$R^4$ Wasserstoff oder Niederalkyl,

W einen jeweils mit dem Carbonyl-C-Atom an den Rest der Formel II

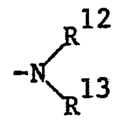

(II)

gebundenen Rest der Formeln III, IV, V oder VI,

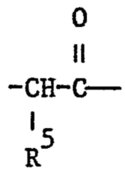

(III)

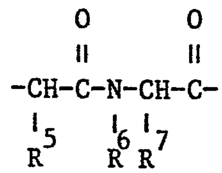

(IV)

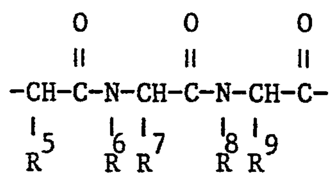

(V)

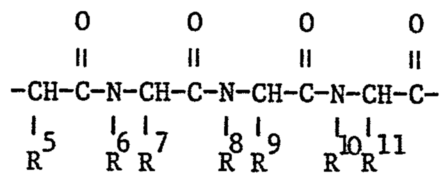

(VI)

worin $R^6$, $R^8$ und $R^{10}$ unabhängig voneinander für Wasserstoff oder Niederalkyl, $R^5$, $R^7$, $R^9$ und $R^{11}$ unabhängig voneinander für Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl, oder Aryl oder $R^4$ und $R^5$ zusammen, $R^6$ und $R^7$ zusammen, $R^8$ und $R^9$ zusammen und/oder $R^{10}$ und $R^{11}$ zusammen jeweils unabhängig voneinander für unsubstituiertes oder durch freies oder funktionell abgewandeltes Hydroxy substituiertes Trimethylen stehen, und $R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, und Salze von solchen Verbindungen mit mindestens einer salzbildenden Gruppe, mit Ausnahme von solchen Verbindungen der Formel I, worin

Z unsubstituiertes Alkylen mit 3 C-Atomen und zugleich

$R^1$ Wasserstoff, unsubstituiertes Alkyl mit 1-4 C-Atomen, Aryl oder Arylniederalkyl,

X den Rest NH,
$R^3$ Aryl, unsubstituiertes Alkyl oder Wasserstoff,
$R^4$ Wasserstoff,
W einen Rest der Formel III, worin $R^5$ eine mit dem Rest $R^3$ identische Bedeutung hat, und
$R^{12}$ und $R^{13}$ Wasserstoff
bedeuten, und von Salzen der letztgenannten Verbindungen mit mindestens einer salzbildenden Gruppe.

Verfahren und neue Zwischenprodukte zur Herstellung dieser Verbindungen, pharmazeutische Präparate, die diese Verbindungen enthalten, und ihre Verwendung zur Herstellung pharmazeutischer Präparate oder als pharmakologisch wirksame Verbindungen.

Der unsubstituierte Alkylenrest Z hat mindestens 3 und vorzugsweise nicht mehr als 17 C-Atome und ist Trimethylen oder Trimethylen, das durch mindestens eine und bis zu vier, bevorzugterweise aber nur 1 oder 2, Alkylgruppen, substituiert ist, und zwar bevorzugterweise in 1-Stellung, 1,1-Stellung oder 3-Stellung, wobei die 1-Stellung des Trimethylenrestes das an das Stickstoffatom gebundene Ende bezeichnet.

Funktionell abgewandeltes Hydroxy ist verestertes oder verethertes Hydroxy.

Verestertes Hydroxy ist vorzugsweise durch eine organische, aber auch durch eine anorganische Säure verestertes Hydroxy, vor allem Acyloxy, daneben auch Sulfonyloxy oder Halogen. Acyloxy ist vorzugsweise gegebenenfalls substituiertes Hydrocarbylcarbonyloxy oder Hydrocarbyloxycarbonyloxy. Gegebenenfalls substituiertes Hydrocarbyl steht hier und im folgenden insbesondere für einen aliphatischen Rest mit 1-21, in erster Linie 1-11, vor allem 1-7 C-Atomen, einen carbocyclischen Rest, d.h. einen cycloaliphatischen Rest mit 3-8, insbesondere 5 oder 6, Ringgliedern und bis zu 21, vorzugsweise bis zu 11 C-Atomen, oder einen mono- oder bicyclischen aromatischen Rest mit

6 oder 10 Ringgliedern und bis zu 21, vorzugsweise bis zu 15 C-Atomen, oder einen entsprechenden carbocyclisch-aliphatischen Rest. Bei den obengenannten cycloaliphatischen beziehungsweise aromatischen Resten handelt es sich vorzugsweise um unsubstituierte oder substituierte Cycloalkylreste, insbesondere unsubstituierte oder durch Niederalkyl substituierte Cycloalkylreste, beziehungsweise um Phenylreste, z.B. Phenyl.

Sulfonyloxy ist insbesondere aromatisches, vornehmlich monocyclisches aromatisches, Sulfonyloxy mit höchstens 12 Kohlenstoffatomen, z.B. Toluolsulfonyloxy, oder niederaliphatisches, vorzugsweise Niederalkylsulfonyloxy.

Das Präfix "Nieder" bezeichnet hier und im folgenden einen Rest mit 1-7, insbesondere 1-4 C-Atomen.

Verethertes Hydroxy ist insbesondere gegebenenfalls substituiertes, vorzugsweise, jedoch nicht im Falle von Aryloxy, unsubstituiertes Hydrocarbyloxy.

Aryl steht für einen unsubstituierten oder substituierten carbocyclischen aromatischen Rest. In erster Linie steht Aryl in den Resten $R^1$, $R^3$ und W für unsubstituiertes oder substituiertes Phenyl, im Rest Z daneben auch für unsubstituiertes oder substituiertes Naphthyl.

Als Arylsubstituenten sind hauptsächlich zu nennen: Niederalkyl, Halogen und freies oder funktionell abgewandeltes Hydroxy, daneben auch freies oder verestertes Carboxy, Oxo oder unsubstituiertes oder substituiertes Phenyl. Aryl als Substituent des Alkylenrestes Z trägt als Substituenten insbesondere Halogen, vor allem Fluor, oder Halogenphenoxy, vor allem 4-Chlor-phenoxy.

Alkyliertes Amino ist in erster Linie Diniederalkylamino, daneben auch Mononiederalkylamino.
Acyliertes Amino ist unsubstituiertes oder substituiertes Hydrocarbylcarbonylamino, insbesondere Niederalkanoylamino. Verestertes Carboxy ist insbesondere unsubstituiertes oder substituiertes Hydrocarbyloxycarbonyl, vor allem Niederalkoxycarbonyl.

Amidiertes Carboxy ist in erster Linie Carbamoyl, daneben auch durch eine Aminosäure, z.B. eine α-Amino-niederalkancarbonsäure, oder durch Mono- oder Diniederalkylamino amidiertes Carboxy.

Unsubstituiertes Niederalkyl $R^3$, $R^5$, $R^7$, $R^9$ oder $R^{11}$ ist vorzugsweise Methyl, Isopropyl, 1-Methyl-propyl oder Isobutyl. Substituiertes Niederalkyl $R^3$, $R^5$, $R^7$, $R^9$ oder $R^{11}$ ist vorzugsweise Phenylmethyl, Hydroxymethyl, 1-Hydroxy-ethyl, Carboxy-methyl, Carbamoylmethyl, 2-Carboxy-ethyl, 2-Carbamoyl-ethyl oder 4-Amino-butyl, daneben auch 3-Guanidino-propyl, 4-Imidazolyl-methyl, 4-Hydroxyphenylmethyl, Mercaptomethyl oder 2-Methylthio-ethyl. Durch freies oder funktionell abgewandeltes Hydroxy substituiertes Trimethylen ist insbesondere in 2-Stellung substituiert. 3-Aza-3-methyl-1,5-pentylen ist durch Oxo vorzugsweise in 1-, 1- und 2- oder 1- und 4-Stellung oder durch Methyl vorzugsweise in 1- und 5-Stellung substituiert.

Die Konfiguration an gegebenenfalls vorhandenen Asymmetriezentren in einer Verbindung der Formel I kann unabhängig voneinander (D), (L) oder (D,L) sein, ist jedoch in den Aminosäureresten vorzugsweise (L).

Die vor- und nachstehend verwendeten Allgemeinbegriffe haben im Rahmen der vorliegenden Beschreibung vorzugsweise die folgenden Bedeutungen:

Niederalkyl ist vor allem Methyl, Ethyl, n-Propyl, Isopropyl, n-Butyl, und Isobutyl, daneben z.B. auch sek. Butyl oder tert. Butyl, ferner n-Pentyl, Neopentyl, n-Hexyl oder n-Heptyl.
Halogen ist insbesondere Fluor oder Chlor, kann jedoch auch für Brom oder Jod stehen.
Cycloalkyl ist z.B. Cyclohexyl oder Cyclopentyl.
Diniederalkylamino ist z.B. Dimethylamino oder Diethylamino.
Mononiederalkylamino ist z.B. Methylamino oder Ethylamino. Niederalkanoylamino ist z.B. Acetylamino oder Propionylamino. Niederalkoxycarbonyl ist z.B. Methoxy- oder Ethoxycarbonyl.

Die Erfindung betrifft auch die insbesondere als Zwischenprodukte verwendbaren Verbindungen der Formel I mit geschützten funktionellen Gruppen, insbesondere geschütztem Hydroxy, Carboxy oder Amino.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxymethyl, 1-Methoxyethyl, 1-Ethoxy-ethyl, 1-Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Jodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe kann z.B. in Form einer leicht spaltbaren Acylamino-, Arylmethylamino-, verätherten Mercaptoamino-, 2-Acylniederalk-1-en-1-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl, oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Jodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Salze der erfindungsgemässen Verbindungen mit salzbildenden Gruppen sind in erster Linie pharmazeutisch verwendbare, nicht-toxische Salze, wie diejenigen von Verbindungen der Formel I mit sauren Gruppen, z.B. mit einer freien Carboxyl- und Sulfogruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxyethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Aminobenzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin. Verbindungen der Formel I mit mindestens einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer sauren, z.B. freien Carboxylgruppe, und einer freien basischen, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt werden.

Die Verbindungen der Formel I, worin die funktionellen Gruppen in freier, d.h. nicht in geschützter Form vorliegen, wobei jedoch Carboxyl- und Hydroxylgruppen gegebenenfalls in physiologisch spaltbarer, veresterter Form vorliegen, und ihre pharmazeutisch verwendbaren, nicht-toxischen Salze sind wertvolle, pharmakologisch wirksame Substanzen. Insbesondere bewirken sie einen Schutz vor der amnesiogenen Wirkung von cerebralem Elektroschock und eine Verbesserung der Gedächtnisleistung. Befunde der genannten Art können in folgenden Lerntests ermittelt werden:
Einer dieser Lerntests ist ein passiver Vermeidungstest, der sogenannte two-compartment passive avoidance test, modifiziert von Jarvik und Koop, Psychol. Rep. 21, 221-224 (1967).

Die Testvorrichtung besteht aus einer grossen Box (35 x 20 x 10 cm), die durch eine Schiebetür mit einer kleinen Box (10 x 10 x 18 cm) verbunden ist. Während die kleine Box durch eine 100 Watt Lampe von oben hell erleuchtet wird, ist die grosse Box dunkel. Der Boden beider Abteile besteht aus einem elektrisch leitenden Gitterrost, dessen Gitterstäbe (6 mm Durchmesser) in einem Abstand von jeweils 13 mm angeordnet sind. Zum Training werden Gruppen von jeweils 10 männlichen Mäusen mit einem Körpergewicht von 20-22 g einzeln in die hell erleuchtete kleine Box gesetzt. Da Mäuse eine Spontanpräferenz für Dunkel haben, gehen die meisten innerhalb von 30 Sekunden ins dunkle Abteil. Sobald sie dieses vollständig betreten haben, wird die Schiebetür geschlossen und ein Fussschock (1 mA, 50 Hz, 5 Sekunden) verabreicht. Die Tiere werden darauf sofort aus dem Versuchsapparat entfernt.

Zur Prüfung des Lerneffektes (Retest) werden die Mäuse nach 24 Stunden wiederum einzeln ins helle Abteil gesetzt und die Zeitspanne bis sie sich ganz im dunklen Abteil befinden, gemessen. Ueblicherweise bleiben nun die meisten Tiere über die ganze Beobachtungszeit von 150 Sekunden

im hellen Abteil. Der Lerneffekt wird weitgehend aufgehoben bzw. die Erinnerung an den Fussschock mindestens partiell ausgelöscht, wenn als amnesiogene Behandlung unmittelbar anschliessend an den Fussschock des Lerndurchgangs eine temporale Elektroschockbehandlung angeschlossen wird. Parameter des Elektroschocks: 50 mA, 0,4 Sekunden, 50 Hz , 0,6 Millisekunden (Pulsdauer).

Zur Prüfung und zum Vergleich ihrer Schutzwirkung gegenüber der amnesiogenen Wirkung des Elektroschocks werden die Testsubstanzen 30 Minuten vor dem Lerndurchgang in Dosen von 0,1 mg/kg, 1 mg/kg, 10 mg/kg und 100 mg/kg (für jede Dosis werden 10 Mäuse verwendet) als Lösung in physiologischer NaCl-Lösung oder physiologische Kochsalzlösung alleine (= Placebo) intraperitoneal verabreicht und die Tiere unmittelbar nach dem Lerndurchgang der Elektroschockbehandlung unterzogen. 24 Stunden später wird anhand der Verweildauer in der beleuchteten Box das Ausmass des noch erhalten gebliebenen Lerneffektes gemessen und mit demjenigen der anderen Testsubstanzen wie auch von Kontrolltieren mit alleiniger Verabreichung des jeweils verwendeten Lösungsmittels und Training ohne, wie auch solchen mit anschliessender Elektroschockbehandlung, verglichen. Eine mit dem Placebo behandelte Gruppe ohne Elektroschockbehandlung dient zur Kontrolle des Lernens im Vermeidungstest, eine zweite mit Placebo behandelte Kontrollgruppe mit anschliessender Elektroschockbehandlung dient zur Ueberwachung des Ausmasses der amnesiogenen Wirkung des Elektroschocks.

Die oben erwähnten Wirkungen lassen sich beispielsweise nach intraperitonealer Verabreichung von N-[(2-Oxo-1-pyrrolidinyl)-acetyl]-glycyl-(L)-alaninamid in einem Dosisbereich von 0,1 bis 100 mg/kg nachweisen.

Zur unmittelbaren Messung der Verbesserung der Gedächtnisleistung wird ein anderer passiver Vermeidungstest (step down passive avoidance test, Psychopharmacol. 63, 297-300 [1979]) verwendet:

Die Testvorrichtung besteht aus einer mit Tageslicht normal beleuchteten Box (50 x 50 x 50 cm) mit einem elektrisch leitenden Gitterrost, dessen Gitterstäbe (6 mm Durchmesser) in einem Abstand von jeweils 13 mm angeordnet sind. In der Mitte des Gitterrostes ist eine hölzerne Plattform (10 mm Höhe, 67 mm Durchmesser) angebracht, welche von einer Plastikröhre (20 cm hoch, 68 mm innerer Durchmesser) umgeben ist. Zur Durchführung des Trainings werden Gruppen von jeweils 30 männlichen Mäusen mit einem Körpergewicht von 20-22g verwendet, indem jeweils ein Tier auf die von der Plastikröhre umgebene Plattform gesetzt, nach 10 Sekunden die Plastikröhre entfernt und die Zeitdauer gemessen wird, welche das Tier zum Hinabsteigen und Berühren des Gitterrostes mit allen 4 Pfoten benötigt. Wenn alle 4 Pfoten den Gitterrost berühren, wird ein Fussschock (1 mA, 50 Hz, 1 Sekunde) verabreicht. Innerhalb von 10 Sekunden nach Verabreichung des Fussschocks werden die Testsubstanzen in Dosen von 3 mg/kg, 10 mg/kg und 30 mg/kg (für jede Dosis werden 30 Mäuse verwendet) als Lösung in physiologischer NaCl-Lösung oder deren Lösungsmittel (physiologische Natriumchloridlösung = Placebo) intraperitoneal verabreicht. 24 Stunden später wird anhand der Verweildauer jedes einzelnen Tieres auf der Plattform das Ausmass der Verbesserung oder Verschlechterung des Lerneffekts im Vergleich zu den Tieren, denen zur Kontrolle lediglich das Lösungsmittel appliziert worden war, ermittelt. So lässt sich eine Verbesserung der Gedächtnisleistung beispielsweise schon nach i.p. Verabreichung von 10 mg/kg N-[(2-Oxo-1-pyrrolidinyl)-acetyl]-glycyl-(L)-alaninamid zeigen.

Aufgrund dieser Eigenschaften erscheinen die neuen Verbindungen zur Behandlung von zerebraler Leistungsinsuffizienz, insbesondere von Gedächtnisstörungen verschiedener Genese, wie senile Demenz, Multiinfarkt-Demenz oder Demenz vom Alzheimer-Typ, ferner von Folgeerscheinungen von Hirntraumen oder Apoplexie geeignet.

Die neuen Verbindungen besitzen ausserdem eine ausgeprägte tumorhemmende Wirkung. Sie hemmen nicht nur das Wachstum der Tumoren und verringern die Tumoranzahl, sondern sie erhöhen auch die Ueberlebenszeit. Dabei liegen zwei Vorteile der erfindungsgemässen Verbindungen gegenüber herkömmlichen cytostatischen oder cytotoxischen Drogen in der guten Verträglichkeit und darin begründet, dass sie als Nootropika die Lebensqualität der Tumorpatienten nicht mindern, sondern, im Gegenteil, diesen Patienten bei der Ueberwindung ihrer psychischen Probleme helfen. Die erfindungsgemässen Verbindungen können somit auch zur Therapie von Tumorerkrankungen, z.B. des Respirationstraktes, bei Warmblütern einschliesslich des Menschen verwendet werden.

Die tumorhemmende Wirkung der neuen Verbindungen kann z.B. durch folgenden Versuch gezeigt werden:

Bei syrischen Hamstern erzeugt man durch Applikation von Diethylnitrosamin in den Magen papilläre, epidermoide und adenocarcinomatöse Tumoren des Kehlkopfes, der Luftröhre und der Lungen (G. Papadopoulo und K.H. Schmidt-Ruppin, Oncology 33, 40-43 (1976)). Ein Teil dieser an Tumoren erkrankten Hamster wird mit einer Verbindung der Formel I (z.B. 4 Wochen lang jeweils zweimal täglich an 5 Tagen pro Woche in einer Einzeldosis von je 20 mg/kg), der restliche Teil der Hamster mit einem Placebo behandelt. Drei oder 4 Tage nach dem Ende der Behandlung werden sämtliche Hamster getötet. Der Respirationstrakt wird herausgeschnitten und nach geeigneter Präparation auf die Grösse, Anzahl und Art der darin enthaltenen Tumoren mikroskopisch untersucht. Dabei findet man, dass die Grösse und Anzahl der Tumoren bei den mit einer Verbindung der Formel I behandelten Hamstern im Vergleich zu den unbehandelten Hamstern signifikant geringer ist.

Insbesondere betrifft die Erfindung Verbindungen der Formel I, worin Z einen Alkylenrest mit 3 bis 17 C-Atomen bedeutet, der unsubstituiert oder durch Hydroxy, Niederalkoxy, Niederalkanoyloxy und/oder einen unsubstituierten oder durch Halogen, Niederalkyl, Halogenphenoxy, Hydroxy oder Niederalkoxy substituierten Phenyl- oder Naphthylrest substituiert ist und der zusammen mit der Amidgruppierung einen fünfgliedrigen Ring bildet,
$R^1$ Wasserstoff, unsubstituiertes oder durch einen Phenylrest, Hydroxy, Niederalkoxy oder Niederalkanoyloxy substituiertes Niederalkyl oder Phenyl, X Sauerstoff oder die Gruppe $NR^2$, worin $R^2$ für Wasserstoff oder Niederalkyl steht,
$R^3$ Wasserstoff, unsubstituiertes oder durch Hydroxy, Niederalkanoyloxy, einen Phenylrest, Aminocarbonyl, Mercapto, Methylthio, Amino, Di- oder Mononiederalkylamino, Niederalkanoylamino, Guanidino, Carboxy, Niederalkoxycarbonyl oder Imidazol-4-yl substituiertes Niederalkyl oder einen Phenylrest, oder $R^2$ und $R^3$ zusammen unsubstituiertes oder durch Hydroxy, Niederalkoxy oder Niederalkanoyloxy substituiertes Trimethylen,
$R^4$ Wasserstoff oder Niederalkyl,
W einen jeweils mit dem Carbonyl-C-Atom an den Rest der Formel II gebundenen Rest der Formeln III, IV, V oder VI, worin
$R^6$, $R^8$ und $R^{10}$ unabhängig voneinander für Wasserstoff oder Niederalkyl, $R^5$, $R^7$, $R^9$ und $R^{11}$ unabhängig voneinander für Wasserstoff, unsubstituiertes oder durch Hydroxy, Niederalkoxy, Niederalkanoyloxy, einen Phenylrest, Aminocarbonyl, Mercapto, Methylthio, Amino, Di- oder Mononiederalkylamino, Niederalkanoylamino, Guanidino, Carboxy, Niederalkoxycarbonyl oder Imidazol-4-yl substituiertes Niederalkyl oder einen Phenylrest, oder $R^4$ und $R^5$ zusammen, $R^6$ und $R^7$ zusammen, $R^8$ und $R^9$ zusammen oder $R^{10}$ und $R^{11}$ zusammen jeweils unabhängig voneinander für unsubstituiertes oder durch Hydroxy, Niederalkoxy oder Niederalkanoyloxy substituiertes Trimethylen stehen, und
$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen

oder unsubstituiertes oder durch Oxo oder Methyl substituiertes 3-Aza-3-methyl-1,5-pentylen bedeuten, und Salze von solchen Verbindungen mit mindestens einer salzbildenden Gruppe, mit Ausnahme der bereits eingangs ausgenommenen Verbindungen der Formel I, die nicht zum Gegenstand der vorliegenden Erfindung gehören.

In erster Linie betrifft die Erfindung Verbindungen der Formel I, worin
Z einen Alkylenrest mit 3 bis 14 C-Atomen bedeutet, der unsubstituiert oder durch Hydroxy, Niederalkanoyloxy und/oder Naphthyl oder einen unsubstituierten oder durch Halogen oder Halogenphenoxy substituierten Phenylrest substituiert ist, und der zusammen mit der Amidgruppierung einen fünfgliedrigen Ring bildet,
$R^1$ Wasserstoff oder unsubstituiertes oder durch Hydroxy oder Niederalkanoyloxy substituiertes Niederalkyl,
X Sauerstoff oder die Gruppe $NR^2$, worin $R^2$ für Wasserstoff oder $C_{1-4}$-Alkyl steht,
$R^3$ Wasserstoff, unsubstituiertes oder durch Hydroxy, Niederalkanoyloxy, Phenyl, 4-Hydroxy-phenyl, Aminocarbonyl, Amino, Niederalkanoylamino, Carboxy, Niederalkoxycarbonyl oder Imidazol-4-yl substituiertes Niederalkyl, oder Phenyl, oder
$R^2$ und $R^3$ zusammen unsubstituiertes oder durch Hydroxy substituiertes Trimethylen,
$R^4$ Wasserstoff oder $C_{1-4}$-Alkyl,
W einen jeweils mit dem Carbonyl-C-Atom an den Rest der Formel II gebundenen Rest der Formeln III, IV, V oder VI, worin
$R^6$, $R^8$ und $R^{10}$ unabhängig voneinander für Wasserstoff oder $C_{1-4}$-Alkyl,
$R^5$, $R^7$, $R^9$ und $R^{11}$ unabhängig voneinander für Wasserstoff, unsubstituiertes oder durch Hydroxy, Niederalkanoyloxy, Phenyl, 4-Hydroxyphenyl, Aminocarbonyl, Amino, Niederalkanoylamino, Carboxy, Niederalkoxycarbonyl oder Imidazol-4-yl substituiertes Niederalkyl, oder Phenyl, oder
$R^4$ und $R^5$ zusammen, $R^6$ und $R^7$ zusammen, $R^8$ und $R^9$ zusammen und/oder
$R^{10}$ und $R^{11}$ zusammen jeweils unabhängig voneinander für unsubstituiertes oder durch Hydroxy oder Niederalkanoyloxy substituiertes Trimethylen stehen, und

$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, und Salze von solchen Verbindungen mit mindestens einer salzbildenden Gruppe.

Hauptsächlich betrifft die Erfindung Verbindungen der Formel I, worin

Z einen Rest der Formel VII bedeutet,

$$\begin{array}{ccccccc} & R^{14} & & R^{16} & & R^{17} & \\ & | & & | & & | & \\ - & C & - & C & - & C & - \\ & | & & | & & | & \\ & R^{15} & & H & & H & \end{array} \qquad \text{(VII)}$$

der mit dem $C(R^{14})$-Atom an das Stickstoffatom gebunden ist, und worin

$R^{14}$ und $R^{15}$ unabhängig voneinander Wasserstoff oder $C_{1-4}$-Alkyl,

$R^{16}$ Wasserstoff, Naphthyl, unsubstituiertes oder durch Fluor, Chlor, Fluorphenoxy oder Chlorphenoxy substituiertes Phenyl, Hydroxy oder Niederalkanoyloxy und

$R^{17}$ Wasserstoff, Hydroxy oder Niederalkanoyloxy bedeuten,

$R^1$ Wasserstoff, unsubstituiertes oder durch Hydroxy substituiertes $C_{1-4}$-Alkyl,

X Sauerstoff oder die Gruppe $NR^2$, worin $R^2$ für Wasserstoff oder geradkettiges $C_{1-3}$-Alkyl steht,

$R^3$ Wasserstoff oder unsubstituiertes oder durch Hydroxy, Phenyl, p-Hydroxy-phenyl, Aminocarbonyl oder Imidazol-4-yl substituiertes Niederalkyl,

$R^4$ Wasserstoff oder geradkettiges $C_{1-3}$-Alkyl,

W einen jeweils mit dem Carbonyl-C-Atom an den Rest der Formel II

gebundenen Rest der Formeln III, IV, V oder VI,
worin
$R^6$, $R^8$ und $R^{10}$ unabhängig voneinander für Wasserstoff oder geradkettiges $C_{1-3}$-Alkyl,
$R^5$, $R^7$, $R^9$ und $R^{11}$ unabhängig voneinander für Wasserstoff oder unsubstituiertes oder durch Hydroxy, Phenyl, 4-Hydroxy-phenyl, Aminocarbonyl oder Imidazol-4-yl substituiertes Niederalkyl, oder, wenn W für Formel III steht, $R^4$ und $R^5$ zusammen, oder, wenn W für Formel IV steht, $R^6$ und $R^7$ zusammen, oder, wenn W für Formel V steht, $R^8$ und $R^9$ zusammen, oder, wenn W für Formel VI steht, $R^{10}$ und $R^{11}$ zusammen für einen unsubstituierten oder durch Hydroxy oder Niederalkanoyloxy substituierten Trimethylenrest stehen, und
$R^{12}$ und $R^{13}$ unabhängig voneinander Wasserstoff oder $C_{1-4}$-Alkyl oder $R^{12}$ und $R^{13}$ zusammen 3-Oxa-1,5-pentylen oder 3-Aza-3-methyl-1,5-pentylen bedeuten, und Salze von solchen Verbindungen mit mindestens einer salzbildenden Gruppe.

Vor allem betrifft die Erfindung Verbindungen der Formel I, worin Z einen mit dem $C(R^{14})$-Atom an das Stickstoffatom gebundenen Rest der Formel VII, worin $R^{14}$ und $R^{15}$ unabhängig voneinander Wasserstoff oder $C_{1-4}$-Alkyl, $R^{16}$ Wasserstoff, Naphthyl, 4-Fluor-phenyl, 4-Chlorphenoxyphenyl oder Hydroxy und $R^{17}$ Wasserstoff bedeuten,
$R^1$ Wasserstoff,
X Sauerstoff oder die Gruppe NH,
$R^3$ Wasserstoff oder $C_1$-$C_4$-Alkyl,
$R^4$ Wasserstoff,
W einen Rest der Formeln III, IV, V oder VI, worin
$R^5$, $R^7$, $R^9$ und $R^{11}$ unabhängig voneinander für Wasserstoff oder $C_{1-4}$-Alkyl,
$R^6$, $R^8$ und $R^{10}$ für Wasserstoff oder $R_{10}$ und $R_{11}$ zusammen für Trimethylen stehen, $R^{12}$ Wasserstoff und $R^{13}$ $C_1$-$C_4$-Alkyl bedeuten.

Besonders betrifft die Erfindung die obengenannten Verbindungen der Formel I, worin X die Gruppe NH und/oder W einen Rest der Formeln III oder IV und/oder $R^7$, $R^9$ und $R^{11}$, daneben auch $R^5$, unabhängig voneinander Wasserstoff oder $C_{1-4}$-Alkyl und/oder $R^2$, $R^4$, $R^6$, $R^8$ und $R^{10}$ Wasserstoff bedeuten.

Ganz besonders betrifft die Erfindung die Verbindungen der Formel I, worin Z Trimethylen oder 2-Hydroxy-trimethylen, $R^1$, $R^3$, $R^4$ und $R^{12}$ Wasserstoff, X die Gruppe NH, W einen Rest der Formeln III oder IV, worin $R^5$ für Wasserstoff oder Methyl und $R^6$ und $R^7$ für Wasserstoff stehen, und $R^{13}$ Wasserstoff oder $C_{1-3}$-Alkyl bedeuten, wobei die Konfiguration am $\underline{C}$-$R^5$, wenn $R^5$ für Methyl steht, (D) oder (L) sein kann, insbesondere sämtliche obengenannten Verbindungen, worin Z Trimethylen bedeutet, und Salze von solchen Verbindungen mit mindestens einer salzbildenden Gruppe, vor allem die Verbindungen der Formel I, worin Z Trimethylen, $R^1$ Wasserstoff, X die Gruppe $NR^2$, $R^2$ und $R^3$ Wasserstoff oder $R^2$ und $R^3$ zusammen Trimethylen, $R^4$ Wasserstoff, W einen Rest der Formeln III, IV, V oder VI, worin $R^5$ für Wasserstoff oder $C_{1-3}$-Alkyl oder $R^4$ und $R^5$ zusammen für Trimethylen, $R^6$, $R^7$, $R^8$, $R^9$, $R^{10}$ und $R^{11}$ für Wasserstoff oder $R^6$ und $R^7$ zusammen für Trimethylen stehen, $R^{12}$ Wasserstoff oder $C_{1-3}$-Alkyl und $R^{13}$ Wasserstoff bedeuten.

In allererster Linie betrifft die Erfindung die in den Beispielen beschriebenen Verbindungen, vor allem N-[(2-Oxo-1-pyrrolidinyl)-acetyl]-L-prolyl-glycyl-glycinamid, aber auch N-[(2-Oxo-1-pyrrolidinyl)-acetyl]-glycyl-(L)-alaninamid und N-[(2-Oxo-1-pyrrolidinyl)-acetyl]-glycyl-(L)-alanin-N-monoethylamid.

Die erfindungsgemässen Verbindungen der Formel I und Salze von solchen Verbindungen mit mindestens einer salzbildenden Gruppe werden nach an sich bekannten Verfahren erhalten.

Selbstverständlich ist auch bei allen vorstehend genannten Verbindungsgruppen die eingangs erwähnte Verbindungsgruppe der Formel I, worin Z unsubstituiertes Alkylen mit 3 C-Atomen und zugleich $R^1$ Wasserstoff, unsubstituiertes Alkyl mit 1-4 C-Atomen, Aryl oder Arylniederalkyl, X den Rest NH, $R^3$ Aryl, unsubstituiertes Alkyl oder Wasserstoff, $R^4$ Wasserstoff, W einen Rest der Formel III, worin $R^5$ eine mit dem Rest $R^3$ identische Bedeutung hat, und $R^{12}$ und $R^{13}$ Wasserstoff bedeuten, und Salze von Verbindungen dieser Verbindungsgruppe mit mindestens einer salzbildenden Gruppe ausgenommen, das heisst die Erfindung betrifft die letztgenannten Verbindungen nicht.

Verbindungen der Formel I und Salze von solchen Verbindungen, die mindestens eine salzbildende Gruppe enthalten, werden beispielsweise hergestellt, indem man

a) eine Verbindung der Formel VIII,

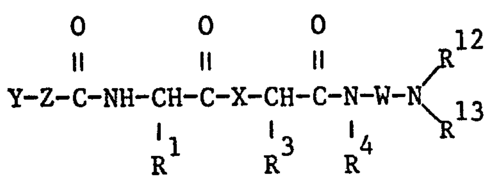

(VIII)

worin Y eine nucleophile Abgangsgruppe bedeutet und die übrigen Substituenten die obengenannten Bedeutungen haben, mit der Massgabe, dass in den Resten Z, $R^1$, $R^3$ oder W gegebenenfalls vorhandene funktionelle Gruppen gegebenenfalls durch leicht abspaltbare Schutzgruppen geschützt sind, intramolekular unter Ausbildung des Pyrrolidonringes zyklisiert, oder

b) eine Verbindung der Formel IX,

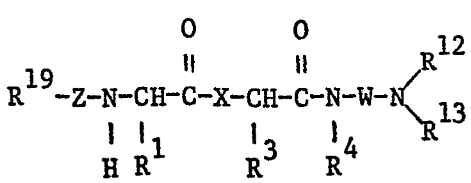

(IX)

worin $R^{19}$ eine Carboxylgruppe oder ein aktiviertes Derivat derselben bedeutet und auch die an der Reaktion teilnehmende Aminogruppe in aktivierter Form vorliegen kann und die übrigen Substituenten die obengenannten Bedeutungen haben, mit der Massgabe, dass in den Resten Z, $R^1$, $R^3$ oder W gegebenenfalls vorhandene funktionelle Gruppen gegebenenfalls durch leicht abspaltbare Schutzgruppen geschützt sind, intramolekular unter Ausbildung des Pyrrolidonringes zyklisiert oder

c) eine Verbindung der Formel X,

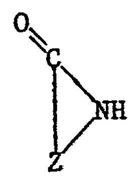

(X)

worin Z die obengenannte Bedeutung hat, mit der Massgabe, dass darin vorhandene funktionelle Gruppen gegebenenfalls durch leicht abspaltbare Schutzgruppen geschützt sind, oder eine reaktionsfähige Form dieser Verbindung mit einer Verbindung der Formel XI,

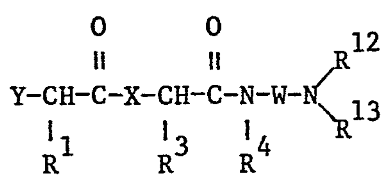

(XI)

worin Y eine nucleophile Abgangsgruppe bedeutet und die übrigen Substituenten die obengenannten Bedeutungen haben, mit der Massgabe, dass in den Resten Z, $R^1$, $R^3$ oder W vorhandene funktionelle Gruppen gegebenenfalls durch leicht abspaltbare Schutzgruppen geschützt sind, umsetzt, oder

d) eine Verbindung der Formel XII,

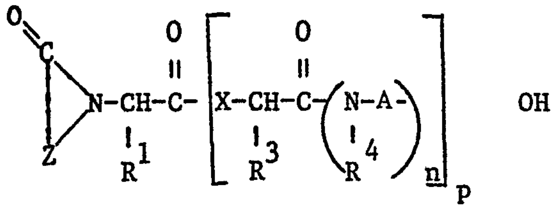

(XII)

worin n und p unabhängig voneinander die Zahl 0 oder 1 und A den Rest W oder $W_a$ bedeuten, wobei $W_a$ einen Rest der Formeln III, IV oder V bedeutet, und die übrigen Substituenten die obengenannten Bedeutungen haben, mit der Massgabe, dass in den Resten Z, $R^1$, $R^3$ und A gegebenenfalls vorhandene funktionelle Gruppen gegebenenfalls durch leicht abspaltbare Schutzgruppen geschützt sind, oder ein reaktionsfähiges Carbonsäurederivat der Verbindung der Formel XII mit einer Verbindung der Formel

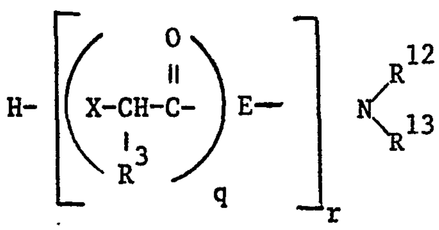

(XIII)

worin E für den Rest der Formel $-N(R^4)-W-$ oder für den Rest $W_b$, der so definiert ist, dass bei Verknüpfung von $W_a$ und $W_b$ der Rest W resultiert, und q und r unabhängig voneinander für die Zahl 0 oder 1 stehen, mit der Massgabe, dass r und q jeweils für 1 und E für den Rest $-N(R^4)-W-$ stehen, wenn im Reaktionspartner der Formel XII p für 0 steht, oder dass r für 1, q für 0 und E für den Rest $-N(R^4)-W-$ stehen, wenn p für 1 und n für 0 stehen, oder dass r für 1, q für 0 und E für $W_b$ stehen, wenn p für 1, n für 1 und A für $W_a$ stehen, oder dass r für 0 steht, wenn p und n jeweils für 1 und A für W stehen, und worin die übrigen Substituenten die obengenannten Bedeutungen haben, mit der Massgabe, dass in den Resten $R^3$ und E

gegebenenfalls vorhandene funktionelle Gruppen gegebenenfalls durch leicht abspaltbare Schutzgruppen geschützt sind, oder einem Derivat der Verbindung der Formel XIII, in dem die Gruppe H-X-, H-E- oder $H-\underset{R^{12}}{N}-$ in reaktionsfähiger Form vorliegt, umsetzt, oder

e) eine Verbindung der Formel I, worin mindestens einer der Reste $R^2$, $R^4$, $R^6$, $R^8$, $R^{10}$, $R^{12}$ und $R^{13}$ für Wasserstoff steht und die übrigen Substituenten die obengenannten Bedeutungen haben, mit der Massgabe, dass in einer Verbindung der Formel I vorhandene funktionelle Gruppen mit Ausnahme der reagierenden Amidgruppe(n) gegebenenfalls durch leicht abspaltbare Schutzgruppen geschützt sind, oder ein reaktionsfähiges Derivat dieser Verbindung mit einem die obengenannten Reste $R^2$, $R^4$, $R^6$, $R^8$, $R^{10}$, $R^{12}$ und/oder $R^{13}$ einführenden Alkylierungsmittel umsetzt, oder

f) in einer Verbindung der Formel I, worin Z für einen mit dem $C(R^{14})$-Atom an das Stickstoffatom gebundenen Rest der Formel XV oder XVI steht,

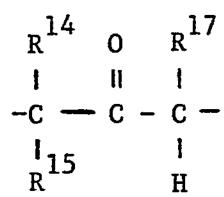

(XV)

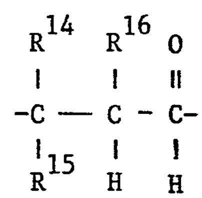

(XVI)

wobei alle Substituenten die obengenannten Bedeutungen haben, mit der Massgabe, dass in den Resten $R^1$, $R^3$, W, $R^{16}$ und $R^{17}$ gegebenenfalls vorhandene funktionelle Gruppen gegebenenfalls durch leicht abspaltbare Schutzgruppen geschützt sind, die Ketogruppe in den Formeln XV oder XVI, mit einem regioselektiven Reduktionsmittel in eine Hydroxygruppe überführt, oder

g) die Cyanogruppe in einer Verbindung der Formel XVII,

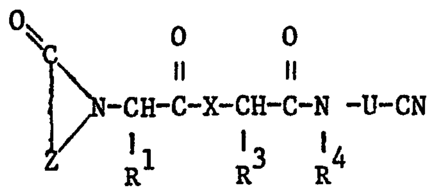

(XVII)

worin U einen Rest der Formel XVIII, XIX, XX oder XXI bedeutet

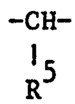

(XVIII)

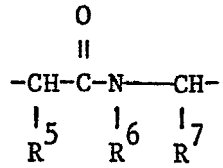

(XIX)

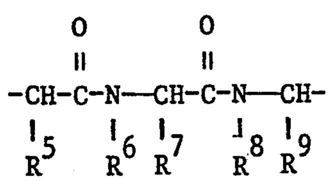

(XX)

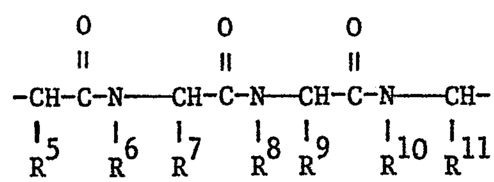

(XXI)

und worin alle Substituenten die obengenannten Bedeutungen haben, in eine Amidgruppe überführt oder

h) die Oximgruppe in einer Verbindung der Formel XXII,

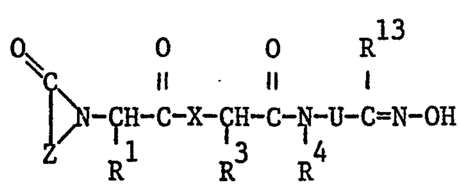

(XXII)

worin die Substituenten die obengenannten Bedeutungen haben, durch eine Beckmann-Umlagerung in eine Amidgruppe überführt, oder

i) in einer Verbindung der Formel I, die mindestens eine freie Hydroxy-, Carboxy- oder Aminogruppe aufweist, freies Hydroxy verestert oder verethert, freies Carboxy verestert oder amidiert oder

freies Amino alkyliert oder acyliert und nach Ausführung der unter a) bis h) beschriebenen Verfahren gegebenenfalls vorhandene Schutzgruppen abspaltet und, wenn erwünscht, erhaltene Stereoisomerengemische auftrennt, und, wenn erwünscht, eine erhaltene Verbindung mit mindestens einer salzbildenden Gruppe in ein Salz oder ein erhaltenes Salz in die freie Verbindung überführt.

Verfahren a) (intramolekulare Zyklisierung einer Verbindung der Formel VIII): Eine nucleophile Abgangsgruppe Y ist insbesondere eine mit geeigneten Säuren veresterte Hydroxygruppe, z.B. ein Sulfonat, wie Mesylat oder Tosylat, vor allem Halogen, wie Brom oder Jod, besonders aber Chlor.

Die Reaktion wird mit Hilfe einer Base durchgeführt, wobei man vorzugsweise äquimolare Mengen dieser Base verwendet. Als Basen verwendet man insbesondere z.B. Metallhydroxide, -carbonate oder -alkoholate, wie Alkalimetall- oder Erdalkalimetallhydroxide oder -alkoholate, z.B. Natrium- oder Kaliumhydroxid oder Natrium-tert.butylat, oder Alkalimetallcarbonate oder Salze, insbesondere Alkalimetallsalze, sekundärer Amide, z.B. 2-Oxo-pyrrolidin-natrium, oder starkbasische Ionenaustauscher, z.B. Dowex 1 (eingetragenes Warenzeichen), daneben auch Hydride oder Amide, z.B. Alkalimetallhydride oder -amide, wie Natriumhydrid oder -amid oder Lithiumdiisopropylamid.

Die Ringschlussreaktion wird vorzugsweise in einem inerten organischen Lösungsmittel, wenn erwünscht oder notwendig, unter Kühlen oder Erwärmen durchgeführt, z.B. in einem Temperaturbereich von etwa -80°C bis etwa +150°C, hauptsächlich zwischen 0°C und 100°C.

Generell sind die Reaktionsbedingungen so zu wählen, dass die intramolekular ablaufende Ringschlussreaktion gegenüber intermolekularen Reaktionen des gleichen Reaktionstyps, die zur Bildung von Dimeren oder ähnlichem führen würde, begünstigt ist. Zu diesem Zwecke kann man z.B., wenn es erforderlich ist, in grösserer Verdünnung arbeiten.

Die Aktivierung mit Hilfe der Base kann bei der gleichen oder einer anderen Temperatur erfolgen als die eigentliche Ringschlussreaktion.

Insbesondere bei Verwendung einer sehr starken Base, z.B. Lithiumdiisopropylamid, erfolgt die Aktivierung mit der Base ("Metallierung") bei tieferer Temperatur (z.B. -80°C) als der Ringschluss. In diesem Fall kann man z.B. das Reaktionsgefäss nach erfolgter Metallierung sich langsam aufwärmen lassen.

Bei der Wahl des Lösungsmittels muss auch die Art der zu verwendenden Base berücksichtigt werden. Reaktionen unter Verwendung von Alkalimetallhydroxiden werden vorzugsweise in Dimethylsulfoxid oder in Alkoholen, wie Niederalkanolen, z.B. wasserfreiem Ethanol bei Siedetemperatur, Reaktionen unter Verwendung von Alkoholaten werden vorzugsweise in Ethern, insbesondere cyclischen Ethern, durchgeführt, bei Verwendung von Natrium-tert.butylat z.B. in Dioxan bei Raumtemperatur. Ringschlussreaktionen mit z.B. 2-Oxo-pyrrolidin-natrium werden unter anderem in einem Gemisch aus einem cyclischen Ether und einem aromatischen Kohlenwasserstoff, z.B. einem Dioxan-Toluol-Gemisch, bei einer Temperatur zwischen Raumtemperatur und 60°C durchgeführt.

Die erfindungsgemässe Zyklisierung mit Hilfe eines Ionenaustauschers nimmt man insbesondere in einem Alkohol, z.B. Niederalkanol, z.B. Aethanol, bei Raumtemperatur vor.

Die Ausgangsstoffe der Formel VIII können nach an sich bekannten Methoden hergestellt werden, z.B. durch Acylierung der freien Aminogruppe eines Peptidamids der Formel XXIII,

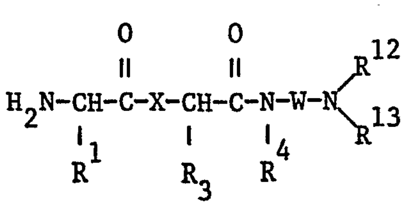

(XXIII)

worin die Substituenten die obengenannten Bedeutungen haben, mit der Massgabe, dass darin vorhandene freie funktionelle Gruppen, wenn

erwünscht, in geschützter Form vorliegen, mit einem Säurechlorid der Formel XXIV,

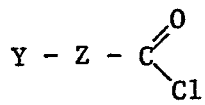

(XXIV)

worin Y und Z die obengenannten Bedeutungen haben, z.B. Y für Chlor steht, mit der Massgabe, dass im Rest Z vorhandene freie funktionelle Gruppen gegebenenfalls durch leicht abspaltbare Schutzgruppen geschützt sind, und nachfolgende Abspaltung der Schutzgruppen.

Verfahren b) (intramolekulare Zyklisierung einer Verbindung der Formel IX):

Aktivierte Carbonsäurederivate einer Verbindung der Formel IX sind in erster Linie reaktionsfähige aktivierte Ester oder reaktionsfähige Anhydride, ferner reaktionsfähige cyclische Amide; dabei können reaktionsfähige Derivate von Säuren der Formel IX auch in situ gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronenanziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenyl-thioester (die man z.B. durch Behandeln der entsprechenden Säure mit gegebenenfalls, z.B. durch Nitro, substituierten Thiophenolen, u.a. mit Hilfe der Anhydrid- oder Carbodiimid-Methode, erhalten kann; Methode der aktivierten Thiolester), Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann).

Anhydride von Säuren der Formel IX können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten

kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Die an der Reaktion teilnehmende Aminogruppe in einem Ausgangsmaterial der Formel IX liegt bevorzugterweise in freier Form vor, insbesondere wenn die damit reagierende Carboxygruppe in reaktionsfähiger Form vorliegt, sie kann aber auch selbst derivatisiert sein, d.h. z.B. durch Reaktion mit einem Phosphit, wie Diäthylchlorphosphit, 1,2-Phenylen-chlorphosphit, Ethyl-dichlor-phosphit, Ethylen-chlor-phosphit oder Tetraethylpyrophosphit, aktiviert worden sein.

Schutzgruppen von gegebenenfalls vorhandenen funktionellen Gruppen sind z.B. die obengenannten Schutzgruppen.

Die Ringschlussreaktion kann in an sich bekannter Weise durchgeführt werden, wobei die Reaktionsbedingungen in erster Linie davon abhängen, ob und wie die an der Reaktion teilnehmende Carboxylgruppe aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +200°C, in einem geschlossenen Reaktionsgefäss und/oder in der Atmosphäre eines Inertgases, z.B. Stickstoff.

Generell sind die Reaktionsbedingungen so zu wählen, dass die intramolekular ablaufende Ringschlussreaktion gegenüber intermolekularen Reaktionen des gleichen Reaktionstyps, die zur Bildung von Dimeren oder ähnlichem führen würde, begünstigt ist. Zu diesem Zwecke kann man z.B., wenn es erforderlich ist, in grösserer Verdünnung arbeiten.

Die Reaktion kann z.B. durchgeführt werden, indem man eine Verbindung der Formel IX, worin $R^{19}$ eine unaktivierte Carboxylgruppe bedeutet, und die übrigen gegebenenfalls in geschützter Form vorliegenden Reste die obengenannten Bedeutungen haben, gegebenenfalls in Gegenwart eines wasserentziehenden Mittels, z.B. eines N,N'-disubstituierten Carbodiimids, wie Dicyclohexylcarbodiimid, und gegebenenfalls zusätzlich in Gegenwart eines N-Hydroxy-amins oder N-Hydroxy-amids, z.B. N-Hydroxy-succinimid, in einem wasserfreien inerten Lösungsmittel erwärmt, z.B. auf 50°C-180°C.

Verbindungen der Formel IX, worin $R^{19}$ eine aktivierte Carboxylgruppe bedeutet, reagieren in einem wasserfreien Lösungsmittel schon bei tieferen Temperaturen, z.B. -20° bis +50°C, unter Ausbildung des 2-Oxo-pyrrolidin-Ringes. Vorzugsweise geht man von solchen

aktivierten Carbonsäureverbindungen der Formel IX aus, worin die dem Rest Z benachbarte Aminogruppe in geschützter Form vorliegt und setzt dann die Aminogruppe in situ frei, worauf die Zyklisierung eintritt.

Eine bevorzugte Ausführungsform des Verfahrens b) ist das Erhitzen einer Carbonsäure der Formel IX in Hexamethyldisilazan.

Die Verbindungen der Formel IX, worin die Carboxylgruppe $R^{19}$ in geschützter Form vorliegt, d.h. als Rest $R_a^{19}$, z.B. als 2-Trimethylsilylethyl-carbonyl, können z.B. durch N-Alkylierung einer Verbindung der Formel XXIII oder eines geeigneten Derivats davon, z.B. eines Azomethins oder des Alkalimetallsalzes eines Benzolsulfonamids, mit einer Verbindung der Formel XXV,

$$R_a^{19} - Z - Y \qquad \text{(XXV)}$$

worin $R_a^{19}$ eine geschützte Carboxylgruppe bedeutet und Z und Y die obengenannten Bedeutungen haben, hergestellt werden.

Verfahren c) (N-Alkylierung des 2-Oxo-pyrrolidins der Formel X):

Eine reaktionsfähige Form einer Verbindung der Formel X ist insbesondere eine Metallverbindung, in erster Linie eine Alkalimetallverbindung, z.B. eine Natrium-, Kalium- oder Lithiumverbindung derselben, die man z.B. durch Einwirkung einer entsprechenden, geeigneten Base, z.B. eines Alkalimetallhydroxids, z.B. Natriumhydroxid, eines Alkalimetallalkoholats, z.B. -niederalkanolats, wie -methylats, -ethylats oder tert.butylats, eines -hydrids, -amids, z.B. Natriumhydrid, Natriumamid oder Lithiumdiisopropylamid, oder einer Alkalimetall-Kohlenwasserstoffverbindung, z.B. Butyllithium, herstellen kann.

Die nucleophile Abgangsgruppe Y entspricht der bei Verfahren a) genannten Gruppe Y und ist vor allem Chlor oder Brom.

Die Reaktion wird vorzugsweise in einem wasserfreien oder, abhängig von der verwendeten Base, aprotischen Lösungsmittel bei Temperaturen zwischen etwa -80°C und +150°C und ähnlich wie bei Verfahren a) oder e) durchgeführt.

Die Ausgangsverbindungen der Formel XI sind durch Acylierung einer Verbindung der Formel XIII, worin q und r beide für 1 stehen, mit einem Acylierungsmittel der Formel XXVI,

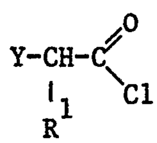

(XXVI)

worin Y und $R^1$ die obengenannte Bedeutung haben, mit der Massgabe, dass im Rest $R^1$ vorhandene freie funktionelle Gruppen, wenn nötig, in geschützter Form vorliegen, und, wenn erwünscht, nachfolgende Abspaltung der Schutzgruppe(n) zugänglich.

Die Ausgangsverbindungen der Formel X sind bekannt oder können z.B. durch Zyklisierung einer Verbindung der Formel XXVII oder einem aktivierten Carbonsäurederivat davon,

$$HOOC\text{-}Z\text{-}NH_2 \qquad (XXVII)$$

worin Z die obengenannte Bedeutung hat, mit der Massgabe, dass darin vorhandene freie funktionelle Gruppen, wenn nötig, geschützt sind, analog Verfahren b) und Abspaltung der Schutzgruppen erhalten werden.

Die Zyklisierung einer freien Säure der Formel XXVII gelingt in vielen Fällen durch Erhitzen auf 200-250° ohne Lösungsmittel und Kugelrohrdestillation.

Verfahren d) (Knüpfung einer Peptid- bzw. Esterbindung):

Reaktionsfähige Carbonsäurederivate einer Verbindung der Formel XII sind reaktionsfähige Ester, Anhydride oder Amide analog den bei Verfahren b) genannten.

Wie bei b) erwähnt können Derivate von Säuren der Formel XII auch in situ gebildet werden. So kann man z.B. N,N'-disubstituierte Amidinoester in situ bilden, indem man das Gemisch des Ausgangsmaterials der Formel XIII und der Säure der Formel XII in Gegenwart eines geeigneten N,N'-disubstituierten Carbodiimids, z.B. N,N'-Dicyclohexylcarbodiimid, zur Reaktion bringt. Ferner kann man Amino- oder Amidoester von Säuren der Formel XII in Gegenwart des zu acylierenden Ausgangsmaterials der Formel XIII bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Ein Derivat der Verbindung der Formel XIII, worin die an der Reaktion teilnehmende Aminogruppe in reaktionsfähiger Form vorliegt, kann z.B. durch Umsetzung mit einem Phosphit, z.B. einem der bei Verfahren b) genannten, hergestellt werden. Eine reaktionsfähige Form einer Verbindung der Formel XIII ist z.B. auch ein Carbaminsäurehalogenid oder ein Isocyanat, wobei die an der Reaktion teilnehmende Aminogruppe in einer Verbindung der Formel XIII an Halogencarbonyl, z.B. Chlorocarbonyl, gebunden ist beziehungsweise als Isocyanatogruppe vorliegt, wobei im letzteren Falle nur Verbindungen der Formel I zugänglich sind, die am Stickstoffatom der durch die Reaktion gebildeten Amidgruppe ein Wasserstoffatom tragen. Eine weitere reaktionsfähige Form einer Verbindung der Formel XIII, worin r für 0 steht, ist z.B. ein Harnstoff der Formel XXVIII,

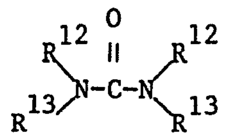
(XXVIII)

worin $R^{12}$ und $R^{13}$ die obengenannten Bedeutungen haben, oder, wenn $R^{12}$ und $R^{13}$ Methyl bedeuten, Dimethylformamid.

Beispielsweise erhält man beim Erhitzen äquimolarer Mengen solcher Harnstoffe und den freien Säuren der Formel XII die Verbindungen der Formel I in guter Ausbeute.

Die Acylierung einer Verbindung der Formel XIII oder einem reaktionsfähigen Derivat davon mit einer Verbindung der Formel XII oder einem reaktionsfähigen Säurederivat davon kann in an sich bekannter Weise durchgeführt werden, wobei die Reaktionsbedingungen in erster Linie von der Art des verwendeten Acylierungsmittels abhängen, üblicherweise in Gegenwart eines geeigneten Lösungs- oder Verdünnungsmittels oder eines Gemisches von solchen, und, falls notwendig, in Gegenwart eines Kondensationsmittels, das z.B. bei Verwendung eines Anhydrids als Acylierungsmittel gegebenenfalls auch ein Säure-bindendes Mittel sein kann, unter Kühlen oder Erwärmen, z.B. in einem Temperaturbereich von etwa -30°C bis etwa +150°C, in einem geschlossenen Reaktionsgefäss und/oder in der Atmosphäre eines Inertgases, z.B. Stickstoff.

Die Ausgangsstoffe der Formeln XII und XIII sind bekannt oder können nach an sich bekannten Verfahren, z.B. analog den in dieser Anmeldung beschriebenen Verfahren, hergestellt werden.

Verfahren d) umfasst auch die Ausführungsform, wonach man zunächst eine Carbonsäure der Formel XII, worin n und p für 1 stehen, mit einer Verbindung der Formel XIII, worin r für 0 steht, unter Bildung eines Ammoniumsalzes der Carbonsäure der Formel XII umsetzt, und das auf diese oder eine andere Art erhaltene Ammoniumsalz unter Bildung einer Verbindung der Formel I thermisch dehydratisiert, z.B. analog der im Britischen Patent 1 309 692 beschriebenen Art.

Verfahren e) (N-Alkylierung einer Verbindung der Formel I):

Ein die Reste $R^2$, $R^4$, $R^6$, $R^8$, $R^{10}$, $R^{12}$ oder $R^{13}$ einführendes Alkylierungsmittel ist insbesondere eine Verbindung, worin einer dieser Reste an die obengenannte nucleophile Abgangsgruppe Y gebunden ist.

Die Verbindung der Formel I wird in der Regel zunächst mit Hilfe einer Base in eine reaktionsfähige Form gebracht. Vorzugsweise verwendet man äquimolare Mengen Base, z.B. eine der bei Verfahren a) genannten Basen. Grundsätzlich, aber in der Regel unnötig, kann man auch noch stärkere Basen, z.B. Metallhydrocarbylverbindungen, wie Alkalimetallniederalkylverbindungen, z.B. Butyllithium, verwenden, oder die Aktivierung kann mit Hilfe von schwächeren Basen unter Phasentransferbedingungen oder in Gegenwart geeigneter Komplexbildner für das Basenkation, wie Kronenethern, z.B. 18-Krone-6, durchgeführt werden.

Die Reaktion wird vorzugsweise in einem inerten organischen Lösungsmittel, wenn erwünscht oder notwendig, unter Kühlen oder Erwärmen durchgeführt, z.B. in einem Temperaturbereich von etwa -80°C bis etwa +150°C, hauptsächlich zwischen 0°C und 100°C.

Bei der Wahl des Lösungsmittels muss insbesondere auch die Art der zu verwendenden Base berücksichtigt werden. Reaktionen unter Verwendung von Alkalimetallhydroxiden werden vorzugsweise in Dimethylsulfoxid oder in Alkoholen, wie Niederalkanolen, z.B. wasserfreiem Ethanol bei Siedetemperatur, Reaktionen unter Verwendung von Alkoholaten werden vorzugsweise in Ethern, insbesondere cyclischen Ethern durchgeführt, bei Verwendung von Natrium-tert.butylat z.B. in Dioxan bei Raumtemperatur. Alkylierungsreaktionen mit Hilfe von z.B. 2-Oxo-pyrrolidinnatrium können unter anderem in einem Gemisch aus einem cyclischen Ether und einem aromatischen Kohlenwasserstoff, z.B. einem Dioxan-Toluol-Gemisch, bei einer Temperatur zwischen Raumtemperatur und 60°C durchgeführt werden. Die eigentliche Alkylierungsreaktion kann bei der gleichen oder einer anderen Temperatur erfolgen als die Aktivierung

mit der Base. Insbesondere bei Verwendung von sehr starken Basen, wie Lithiumdiisopropylamid oder Butyllithium, erfolgt die Aktivierung der Ausgangsverbindung der Formel I mit der Base ("Metallierung") bei tieferer Temperatur (z.B. -80°C) als die eigentliche Alkylierung. In diesem Fall kann man das Alkylierungsmittel erst nach beendeter Metallierung in das Reaktionsgefäss geben und dieses langsam aufwärmen lassen. Derartige Metallierungsreaktionen mit sehr starken Basen werden in einem inerten, aprotischen Lösungsmittel und unter Schutzgas durchgeführt, beispielsweise in einem Kohlenwasserstoff, z.B. Hexan, Benzol, Toluol oder Xylol, einem schwach polaren Ether, z.B. Diethylether, Tetrahydrofuran oder Dioxan, oder einem Säureamid, z.B. Hexamethylphosphorsäuretriamid, oder Mischungen davon. Die Reaktionstemperatur liegt zwischen etwa -80° und etwa Raumtemperatur, in Abhängigkeit vornehmlich vom Schmelzpunkt des Lösungsmittels oder Lösungsmittelgemisches und von der Reaktivität des jeweiligen Metallierungsreagenz in dem gewählten Lösungsmittel, aber auch von der Löslichkeit und der Reaktivität des Substrats.

Amidgruppen -CO-NH- in einer Verbindung der Formel I, die nicht alkyliert werden sollen, können in an sich bekannter Weise, z.B. in Form des N-Diphenyl-methyl-Derivats oder eines N-Methoxy-benzyl-Derivats, z.B. eines 2,4-Dimethoxy- oder 2,4,6-Trimethoxy-benzyl-Derivats, geschützt werden.

Weitere Einzelheiten zur Durchführung von Metallierungs- und Alkylierungsreaktionen, die den oben beschriebenen analog sind, sind z.B. beschrieben in Houben-Weyl, Methoden der Organischen Chemie, Band XIII/1, Thieme Verlag, Stuttgart 1970.

Verfahren f) (Reduktion einer Ketogruppe im Rest Z):

Solche regioselektiven Reduktionsmittel können verwendet werden, die unter den Reaktionsbedingungen eine isolierte Ketogruppe genügend schneller als Amidgruppen reduzieren.

In erster Linie zu nennen sind viele Borhydride, wie Alkalimetallborhydride, insbesondere Natriumborhydrid, Lithiumborhydrid oder Natriumcyanborhydrid, oder einige Aluminiumhydride, wie sterisch gehinderte Alkalimetallniederalkoxyaluminiumhydride, z.B. Lithium-tris-tert.butoxy-aluminiumhydrid.

Die Reduktion kann auch mit Wasserstoff in Gegenwart bestimmter Schwermetallkatalysatoren, z.B. Raney-Nickel, Platin- oder Palladiumkatalysatoren, oder nach Meerwein-Ponndorf-Verley mit Hilfe von Aluminiumalkanolaten, bevorzugt Aluminium-2-propanolat oder -ethanolat, durchgeführt werden.

Die Reduktion wird vorzugsweise mit stöchiometrischen Mengen oder, wenn es aufgrund unerwünschter Nebenreaktionen, z.B. mit dem Lösungsmittel, notwendig ist, einem sinnvoll bemessenen Ueberschuss des Reduktionsmittels in einem inerten Lösungsmittel bei Temperaturen zwischen -80°C und +180°C, vorzugsweise zwischen -20°C und +100°C, wenn nötig, unter Schutzgas, z.B. Argon, durchgeführt.

Die Ausgangsverbindungen können nach an sich bekannten Methoden, z.B. den in dieser Anmeldung beschriebenen, hergestellt werden. Dabei kann der 2,3- bzw. 2,4-Dioxo-pyrrolidinring z.B. durch eine Dieckmann-Kondensation ausgehend von einer Verbindung der Formel XXXI bzw. XXXII hergestellt werden,

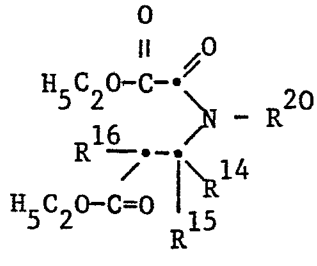

(XXXI)

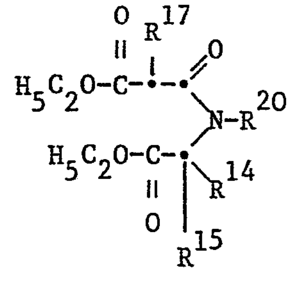

(XXXII)

worin $R^{20}$ einen Rest der Formel XXXIII

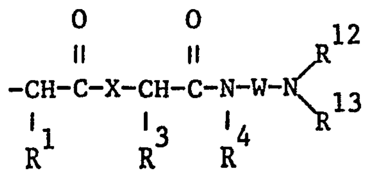

(XXXIII)

oder einen geeignet vervollständigten Teil desselben bedeutet, und alle übrigen Substituenten die obengenannten Bedeutungen haben.

Wenn $R^{14}$ und $R^{15}$ für Wasserstoff stehen, ist ein geeignet vervollständigter Teil eines Restes der Formel XXXIII bevorzugterweise Ethoxycarbonylmethyl.

Verfahren g) (Ueberführung einer Cyanogruppe ins Amid):
Die Ueberführung kann durch partielle Hydrolyse, im Sinne einer Graf-Ritter-Reaktion oder auf dem Weg über Carbonsäureesterimid-Salze erfolgen. Die Bedingungen zur Hydrolyse einer Verbindung der Formel XVII müssen so gewählt werden, dass die Reaktion auf der Stufe des Amids angehalten werden kann und nicht die freie Carbonsäure gebildet wird. Zu diesem Zweck am generellsten geeignet ist die Hydrolyse mit Säuren, wobei man je nach den in einer Verbindung der Formel XVII vorhandenen Substituenten wählen kann zwischen 80%iger Schwefelsäure (unter Erwärmen), Polyphosphorsäure (bei 110-150°), Bromwasserstoff/Eisessig (Raumtemperatur), Ameisensäure (ohne Lösungsmittel), Chlorwasserstoffgas in ätherischer Lösung gefolgt von Zugabe von Wasser oder wässeriger Salzsäure, oder Borhalogeniden/1 Aequivalent Wasser.

Bevorzugterweise verwendet man ein Ionenaustauscherharz, z.B. Lewatite® M 504 (vgl. Deutsche Offenlegungsschrift 2 507 576) oder Amberlite® IRA-400.

In einigen Fällen gelingt auch die alkalische Hydrolyse, jedoch ist die Methode von Radziszewski mit Wasserstoffperoxid in Gegenwart von Alkalien bei mässiger Temperatur meist erfolgreicher.

Mit Hilfe der Graf-Ritter-Reaktion gelingt die Herstellung N-substituierter Amide aus den Nitrilen der Formel XVII.

Hierzu setzt man die Nitrile in Gegenwart einer starken Säure, vornehmlich 85-90%iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen oder Alkoholen.

Statt mit einer starken Säure kann man auch mit Quecksilber(II)-nitrat katalysieren und anschliessend mit Natriumborhydrid reduzieren.

Die Carbonsäureesterimide erhält man z.B. durch säurekatalysierte Anlagerung von Alkoholen an die Nitrile. Aus den Esterimiden erhält man die Amide im Sinne einer Pinner-Spaltung.

Nitrile der Formel XVII können beispielsweise durch eine Kolbe-Synthese aus den entsprechenden primären Halogeniden mit Cyanidionen im Sinne einer nucleophilen Substitution hergestellt werden.

Bevorzugterweise setzt man z.B. ein primäres Halogenid mit Kupfercyanid in Dioxan um (vgl. Deutsche Offenlegungsschrift 2 507 576). Die primären Halogenide erhält man z.B. aus den primären Alkoholen mittels Thionylchlorid (vgl. Deutsche Offenlegungsschrift 2 507 576). Bevorzugterweise erhält man Nitrile der Formel XVII mit endständiger Cyanomethylengruppe durch Reaktion einer Verbindung der Formel XII, worin p für 1 steht und, falls n ebenfalls für 1 steht, A den Rest $W_a$ bedeutet, mit Aminoacetonitril, welches im Handel erhältlich ist, nach den für Verfahren d) beschriebenen Methoden.

Verfahren h) (Beckmann-Umlagerung):

Die Umlagerung von Verbindungen der Formel XXII, worin $R^{13}$ Niederalkyl bedeutet, kann unter den bekannten Bedingungen der Beckmann-Umlagerung durchgeführt werden, z.B. mit konzentrierter Schwefelsäure, Halogenwasserstoffen, Polyphosphorsäure, Thionylchlorid, Ameisensäure, Tosylchlorid in Pyridin, Kupfer, Alkalimetallchloriden, Eisen(III)- oder Aluminiumchlorid, Trifluoressigsäure oder "Japanese acid earth". Vorzugsweise geht man jedoch von Oximen aus, worin $R^{13}$ trans-ständig zur Hydroxygruppe der Oximgruppierung steht, und verwendet in diesem Falle Katalysatoren, die möglichst geringe Isomerisierung verursachen, wie Phosphor(V)-chlorid in Ether oder Benzol bei tiefer Temperatur.

Die Umlagerung von Verbindungen der Formel XXII, worin $R^{13}$ Wasserstoff bedeutet, führt man am besten mit Polyphosphorsäure oder Bortrifluorid durch. In diesem Fall spielt die Konfiguration des Oxims keine Rolle.

Die Oxime der Formel XXII sind nach an sich bekannten Methoden, z.B. durch Umsetzung der entsprechenden Aldehyde oder Ketone mit Hydroxylaminen zugänglich.

Verfahren i) (Umwandlungen innerhalb der Definition der Endstoffe):

Die erfindungsgemäss erhältlichen Verbindungen der Formel (I) können in an sich bekannter Weise in andere Verbindungen der Formel (I) überführt werden.

In den Verbindungen der Formel (I), die mindestens eine freie Hydroxylgruppe aufweisen, kann diese nach an sich bekannten Methoden verethert oder verestert (acyliert) werden. Freies Carboxy kann verestert oder amidiert werden. Freies Amino kann alkyliert oder acyliert werden, wobei die Alkylierung analog der Veretherung und die Acylierung analog der Veresterung einer Hydroxylgruppe durchgeführt wird.

Geeignete Mittel zur Veretherung einer Hydroxylgruppe oder Veresterung einer Carboxylgruppe sind beispielsweise entsprechende Diazoverbindungen, wie gegebenenfalls substituierte Diazoniederalkane, z.B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Ethers,wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel zur Veretherung einer Hydroxylgruppe oder zur Veresterung einer Carboxylgruppe in einer Verbindung der Formel I sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure, oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise

sterisch gehinderte Triniederalkylamine, z.B. N,N-Diisopropyl-N-ethyl-amin (vorzugsweise zusammen mit Halogensulfonsäure-niederalkylestern oder gegebenenfalls halogensubstituierten Methansulfonsäure-niederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre, gearbeitet wird.

Durch Phasentransfer-Katalyse [siehe z.B. Dehmlow, Angewandte Chemie, 86, 187 (1974)] kann die oben beschriebene Veretherungsreaktion wesentlich beschleunigt werden. Als Phasentransfer- Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyl-triethylammoniumchlorid, in katalytischen oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten,niederaliphatischen, cycloaliphatischen oder aromatischen Kohlenwasserstoffe, wie Tri- oder Tetrachlorethylen, Tetrachlorethan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können bei basenempfindlichen Verbindungen der Reaktionsmischung titrimetrisch, z.B. mittels eines Titrierautomaten, zugesetzt werden, damit der pH-Wert während der Veretherung zwischen etwa 7 und etwa 8,5 bleibt.

Weitere Mittel zur Veretherung einer Hydroxylgruppe oder zur Veresterung einer Carboxylgruppe in einer Verbindung der Formel I sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Veretherungsmittel vorzugsweise in einem inerten Lösungsmittel, wie einem Ether oder einem halogenierten Kohlenwasserstoff, z.B. Diethylether, Tetrahydrofuran oder Methylenchlorid,oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere geeignete Veretherungsmittel sind schliesslich entsprechende 1-substituierte 3-Aryltriazenverbindungen, worin der Substituent den verethernden Rest, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyltriazene, z.B. 3-(4-Methylphenyl)-1-methyl-triazen, 3-(4-Methylphenyl)-1-ethyl-triazen oder 3-(4-Methylphenyl)-1-isopropyl-triazen. Diese Reagentien werden üblicherweise in Gegenwart von inerten Lösungsmitteln, wie gegebenenfalls halogenierten Kohlenwasserstoffen oder Ethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig in einem geschlossenen Gefäss und/oder in einer Inertgas-, z.B. Stickstoffatmosphäre, verwendet.

Die Umwandlung von freiem Carboxyl in einer Verbindung der Formel (I) in verestertes Carboxyl, kann weiterhin beispielsweise erfolgen, indem man eine Verbindung der Formel (I), worin andere, gegebenenfalls vorhandene funktionelle Gruppen gegebenenfalls in geschützter Form vorliegen, oder ein reaktionsfähiges funktionelles Carboxyderivat, inkl. ein Salz davon, mit einem entsprechenden Alkohol oder einem reaktionsfähigen funktionellen Derivat davon umsetzt.

Die Veresterung von freiem Carboxyl mit dem gewünschten Alkohol wird in Gegenwart eines geeigneten Kondensationsmittels durchgeführt. Uebliche Kondensationsmittel sind z.B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel (I) sind z.B. Anhydride, insbesondere gemischte Anhydride, und aktivierte Ester.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner Stickstoffwasserstoffsäuren, d.h. die entsprechenden Säureazide, sowie phosphorhaltigen Säuren, z.B. Phosphorsäure, Diethylphosphorsäure oder phosphorige Säure, oder schwefelhaltigen Säuren, z.B. Schwefelsäure, oder Cyanwasserstoffsäure. Gemischte Anhydride sind weiter z.B. diejenigen

mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie Ethyl- oder Isobutylhalbestern der Kohlensäure, oder mit organischen, insbesondere aliphatischen oder aromatischen Sulfonsäuren, z.B. p-Toluolsulfonsäure.

Zur Reaktion mit einem Alkohol geeignete aktivierte Ester einer Verbindung der Formel I mit mindestens einer Carboxylgruppe sind z.B. Ester mit vinylogen Alkoholen (d.h. Enolen), wie vinylogen Niederalkenolen, oder Iminomethylesterhalogeniden, wie Dimethyliminomethylesterchlorid (hergestellt aus der Carbonsäure und Dimethylchlormethyliden-iminium-chlorid der Formel $[(CH_3)_2N{=}CHCl]^{\oplus}Cl^{\ominus}$), oder Arylester, wie Pentachlorphenyl-, 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Reaktion zwischen einem solchen Säurederivat der Formel I, wie einem Anhydrid, insbesondere einem Säurehalogenid, und einem Alkohol wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triethylamin oder Ethyldiisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Ethylen- oder Propylenoxid, durchgeführt.

Ein reaktionsfähiges funktionelles Derivat des veresternden Alkohols ist in erster Linie ein entsprechender Ester, vorzugsweise mit einer starken anorganischen oder organischen Säure und stellt insbesondere ein entsprechendes Halogenid, z.B. Chlorid, Bromid oder Jodid, oder eine entsprechende Niederalkyl- oder Aryl-, wie Methyl- oder 4-Methylphenylsulfonyloxyverbindung dar.

Ein solcher reaktionsfähiger Ester eines Alkohols kann mit der freien Carboxylverbindung der Formel (I) oder einem Salz, wie einem Alkalimetall- oder Ammoniumsalz, davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureethylester, oder einem Nitril, z.B.Acetonitril, oder Mischungen davon, wenn notwendig unter Kühlen oder Erwärmen, z.B. in einem Temperaturbereich von etwa -40°C bis etwa +100°C, vorzugsweise bei etwa -10°C bis etwa +40°C, und/oder in einer Inertgas-, z.B. Stickstoffatmosphäre.

Ferner kann das Säurederivat, wenn erwünscht, <u>in situ</u> gebildet werden. So erhält man z.B. ein gemischtes Anhydrid durch Behandeln der Carbonsäureverbindung mit entsprechend geschützten funktionellen Gruppen oder eines geeigneten Salzes davon, wie eines Ammoniumsalzes, z.B. mit einem organischen Amin, wie Piperidin oder 4-Methylmorpholin, oder eines Metall-, z.B. Alkalimetallsalzes mit einem geeigneten Säurederivat, wie dem entsprechenden Säurehalogenid einer gegebenenfalls substituierten Niederalkancarbonsäure, z.B. Trichloracetylchlorid, mit einem Halbester eines Kohlensäure-halbhalogenids, z.B. Chlorameisensäurehalbester oder -isobutylester, oder mit einem Halogenid einer Diniederalkylphosphorsäure, z.B. Diethylphosphorbromidat, und verwendet das so erhältliche gemischte Anhydrid ohne Isolierung.

Zur Veresterung (Acylierung) einer Hydroxygruppe in einer Verbindung der Formel I behandelt man das Ausgangsmaterial der Formel (I) mit einem, den gewünschten Acylrest einer organischen Carbonsäure einführenden Acylierungsmittel. Dabei verwendet man die entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure,oder dann diejenigen mit geeigneten Kohlensäurehalbderivaten, wie entsprechenden -halbestern (wie die z.B. mit einem Halogen-ameisensäure-niederalkyl, wie Chlorameisensäure-ethylester oder -isobutylester, gebildeten gemischten Anhydride) oder mit gegebenenfalls substituierten, z.B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Niederalkyl-, z.B. Cyanmethylester, oder geeignet substituierte Phenyl-, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren, z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triethylamin, oder heterocyclischen Basen, z.B. Pyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre, durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Eine durch eine organische Sulfonsäure, z.B. Niederalkansulfonsäure, wie Methansulfonsäure, oder eine aromatische Sulfonsäure, wie p-Toluolsulfonsäure, veresterte Hydroxygruppe kann man vorzugsweise durch Behandeln des Ausgangsmaterials der Formel (I) mit einem reaktionsfähigen Sulfonsäurederivat, wie einem entsprechenden Halogenid, z.B. Chlorid, wenn notwendig, in Gegenwart eines Säure-neutralisierenden basischen Mittels, z.B. einer anorganischen oder organischen Base, z.B. in analoger Weise wie die entsprechenden Ester mit organischen Carbonsäuren, bilden.

In einer Verbindung der Formel I, die mindestens eine Carboxylgruppe oder ein reaktives Derivat derselben aufweist, kann diese analog wie bei Verfahren d) für die Säuren der XII oder deren Säurederivate beschrieben mit einem Amin amidiert werden.

Die zur Ausführung der vorstehend beschriebenen Verfahren a) bis i) benötigten Ausgangsstoffe sind bekannt oder können nach an sich bekannten Verfahren, z.B. nach oder analog den in dieser Anmeldung beschriebenen hergestellt werden.

Funktionelle Gruppen in Ausgangsstoffen können mit den gleichen Schutzgruppen geschützt werden, wie sie für entsprechende funktionelle Gruppen in den Endstoffen der Formel I in dieser Anmeldung genannt sind. Auch die Einführung und Abspaltung dieser Schutzgruppen kann in der Weise geschehen, wie sie in den zitierten Literaturstellen geschildert ist.

In einer erhaltenen Verbindung der Formel (I), worin eine oder mehrere funktionelle Gruppen geschützt sind, können diese, z.B. geschützte Carboxyl-, und/oder Hydroxygruppen, in an sich bekannter Weise, mittels Solvolyse, insbesondere Hydrolyse, Alkoholyse oder Acidolyse, oder in einigen Fällen auch mittels vorsichtiger Reduktion, gegebenenfalls stufenweise oder gleichzeitig freigesetzt werden. Silylschutzgruppen werden vorteilhafterweise mit Fluoriden, z.B. Tetraethylammoniumfluorid, abgespalten.

Salze von Verbindungen der Formel (I) mit salzbildenden Gruppen können in an sich bekannter Weise hergestellt werden. So kann man Salze von Verbindungen der Formel (I) mit sauren Gruppen, z.B. durch Behandeln mit Metallverbindungen, wie Alkalimetallsalzen von geeigneten organischen Carbonsäuren, z.B. dem Natriumsalz der α-Ethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel (I) mit mindestens einer basischen Gruppe, z.B. einer freien Aminogruppe, erhält man in üblicher Weise, z.B. durch Behandeln mit einer Säure oder einem geeigneten Anionenaustauschreagens. Innere Salze von Verbindungen der Formel (I), welche z.B. eine freie Carboxylgruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie etc. in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie oralen oder rektalen sowie parenteralen, wie intraperitonealen, intramuskulären oder intravenösen, Verabreichung an Warmblüter, welche den pharmakologischen Wirkstoff allein oder zusammen mit einem pharmazeutisch anwendbaren Trägermaterial enthalten.

Die Dosierung des Wirkstoffes hängt von der Warmblüter-Spezies, dem Körpergewicht, Alter und dem individuellen Zustand, der zu behandelnden Krankheit sowie von der Applikationsweise ab.

Die Dosierung liegt zwischen etwa 0,1 und etwa 100 mg/kg täglich, vorzugsweise zwischen etwa 0,1 und etwa 40 mg/kg täglich, z.B. bei etwa 10 mg/kg täglich.

Die an Warmblüter, z.B. Menschen, von etwa 70 kg Körpergewicht zu verabreichenden Dosen liegen zwischen etwa 10 mg und etwa 10 g, vorzugsweise zwischen etwa 100 mg und 2 g, z.B. bei 600 mg, pro Warmblüter und Tag, verteilt auf bis zu etwa 12, vorzugsweise 2 bis 4, z.B. 3, Einzeldosen, die z.B. gleich gross sein können. Die Dosierung bei enteraler und parenteraler Applikation ist im wesentlichen gleich. Die Abhängigkeit der Dosierung vom Körpergewicht ist nicht linear, sondern eher schwach ausgeprägt, so dass man die obengenannte Dosierung auch bei Warmblütern mit geringerem oder höherem Körpergewicht als 70 kg, z.B. 35 kg bzw. 100 kg, anwenden kann. Ueblicherweise jedoch erhalten Kinder die halbe Erwachsenendosis.

Die neuen pharmazeutischen Präparate enthalten eine signifikante Menge, insbesondere von etwa 1 % bis etwa 99 %, hauptsächlich von etwa 10 % bis etwa 95 %, vorzugsweise von etwa 20 % bis etwa 90 % des Wirkstoffes. Erfindungsgemässe pharmazeutische Präparate können z.B. in Dosiseinheitsform, wie Dragées, Tabletten, Kapseln, Suppositorien oder Ampullen, vorliegen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren, hergestellt. Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw.

Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Poylvinylpyyrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die Herstellung der Injektionspräparate erfolgt in üblicher Weise unter antimikrobiellen Bedingungen, ebenso das Abfüllen in Ampullen oder Vials sowie das Verschliessen der Behälter.

Zur Herstellung der Injektionspräparate verwendet man vorzugsweise Lösungen des Wirkstoffes, daneben auch Suspensionen, und zwar insbesondere isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natrium-Carboxymethylcellulose, Carboxymethylcellulose, Dextran, Polyvinylpyrrolidon oder Gelatine, enthalten.

Die folgenden Beispiele dienen zur Illustration der Erfindung. Temperaturen werden in Celsiusgraden angegeben. $R_f$-Werte werden, wenn nicht anders angegeben, auf Kieselgeldünnschichtplatten (Merck, Darmstadt, Deutschland) ermittelt.

Beispiel 1: 36 g (0,088 Mol) 2-(4-Hydroxy-2-oxo-1-pyrrolidinyl)-essigsäurepentachlorophenylester und 14,7 g (0,088 Mol) Glycyl-glycinamid-hydrochlorid werden in 250 ml Dimethylformamid eingerührt und mit 13 ml (0,088 Mol) Triethylamin versetzt. Die Suspension wird 20 Stunden lang bei Raumtemperatur gerührt und dann abgesaugt. Das klare Filtrat wird mit 1,5 Liter Ethylether verrührt, worauf ein Oel ausfällt, das nach einer Stunde Rühren durchkristallisiert. Die Kristalle werden abgesaugt, in 100 ml Wasser gelöst und dann durch eine Säule mit 400 g Amberlite IR 120 ($H^+$-Form, starksaurer Kationenaustauscher) gegeben. Das wässrige Eluat wird am Rotationsverdampfer eingedampft (60°/12 Min.). Der halbfeste Rückstand wird in 300 ml Ethanol heiss gelöst, filtriert und im Eisbad kristallisieren lassen.

Das Rohprodukt mit dem Schmelzpunkt 133-9° wird nochmals aus Ethanol kristallisiert, abgesaugt und bei 120°/12 Torr im Exsikkator getrocknet, worauf man N-[(4-Hydroxy-2-oxo-1-pyrrolidinyl)-acetyl]-glycyl--glycinamid mit Schmelzpunkt 138-141° erhält.

| | | | | | |
|---|---|---|---|---|---|
| $C_{10}H_{16}N_4O_5$ | (272,26) | Ber. | C 44,15 | H 5,93 | N 20,58 |
| | | Gef. | C 44,2 | H 6,1 | N 20,5 |

Den als Ausgangsprodukt verwendeten Ester erhält man folgendermassen:

4,0 g (0,034 Mol) 4-Amino-3-hydroxy-buttersäure werden bei 200-250° geschmolzen. Die Schmelze wird bei 180°/0,02 Torr im Kugelrohr destilliert.

Das Destillat wird in Ethanol heiss gelöst, abgekühlt und mit Ethylether versetzt. Die erhaltenen Kristalle von 4-Hydroxy-2-oxo-pyrrolidin werden abgesaugt und mit Ethylether gewaschen, wonach sie bei 118-120° schmelzen.

1,0 g (0,01 Mol) 4-Hydroxy-2-oxo-pyrrolidin und 1,1 g 0,015 Mol Ethylvinylether in 6 ml Chloroform werden bei Raumtemperatur gerührt. Zu dieser Suspension wird eine kleine Spatelspitze (ca. 30 mg) Trichloressigsäure zugegeben. Man erwärmt die Suspension 10 Minuten bei 50°C, worauf eine klare Lösung entsteht, die bei Raumtemperatur 18 Stunden gerührt wird.

Das Reaktionsgemisch wird mit 10 ml gesättigter wässriger Natriumhydrogencarbonatlösung extrahiert. Die Chloroformphase wird mit gesättigter wässriger Natriumchloridlösung gewaschen, über Natriumsulfat getrocknet, eingedampft und im Kugelrohr destilliert, worauf man 4-(1-Ethoxyethoxy)-2-oxo-pyrrolidin als klares, farbloses Oel mit $R_f$ = 0,40 (Toluol : Ethanol = 8:2; Kieselgel) erhält.

1,1 g (0,0064 Mol) 4-(1-Ethoxy-ethoxy)-2-oxo-pyrrolidin in 10 ml Toluol und 0,3 g einer 50%igen Suspension von Natriumhydrid in Mineralöl werden eine Stunde bei Raumtemperatur gerührt. Dann tropft man unter Kühlung im Eisbad und Rühren innerhalb von 10 Minuten eine Lösung von 2,5 g (0,0064 Mol) Bromessigsäurepentachlorophenylester in 5 ml Toluol zu. Das Reaktionsgemisch wird 18 Stunden bei Raumtemperatur gerührt. Die bräunliche Suspension wird mit Essigester verdünnt, mit gesättigter Natriumchloridlösung gewaschen, über Natriumsulfat getrocknet, filtriert und bei 65°/12 Torr eingedampft, worauf man ein zähflüssiges Oel erhält, das man mit 20 ml Methanol versetzt und filtriert.

Das Filtrat, das 2-[4-(1-Ethoxy-ethoxy)-2-oxo-1-pyrrolidinyl]-essigsäure-pentachlorophenylester enthält, wird mit 0,2 g p-Toluolsulfonsäure-Monohydrat versetzt und 30 Minuten bei Raumtemperatur stehen gelassen. Danach wird eingedampft und der erhaltene Feststoff mit Hexan verkocht, um vom Mineralöl zu befreien. Der im Hexan unlösliche Anteil wird aus Essigester umkristallisiert, worauf man 2-(4-Hydroxy-2-oxo-1-pyrrolidinyl)-essigsäure-pentachlorophenylester mit Schmelzpunkt 182-186° erhält.

Den verwendeten Bromessigsäureester erhält man folgendermassen:

14,0 g (0,1 Mol) Bromessigsäure und 32 g (0,12 Mol) Pentachlorophenol werden in 250 ml absolutem Tetrahydrofuran gelöst. Die gelbliche Lösung wird im Eisbad gerührt und tropfenweise mit einer Lösung von 22,7 g (0,11 Mol) Dicyclohexylcarbodiimid in 75 ml absolutem Tetrahydrofuran versetzt, wobei die Temperatur unterhalb 10° gehalten wird. Anschliessend rührt man noch eine Stunde im Eisbad und 2 Stunden bei Raumtemperatur.

Der ausgefallene Dicyclohexylharnstoff wird abgesaugt, und das Filtrat bei 60°/12 Torr eingeengt. Der Rückstand wird in 500 ml Ethylether gelöst und filtriert. Das klare Filtrat wird mit 50 ml kalter 1 normaler Natriumhydroxidlösung extrahiert, mit Natriumchloridlösung gewaschen, über Natriumsulfat getrocknet, filtriert und eingedampft. Der feste Rückstand wird aus Hexan umkristallisiert, worauf man Bromessigsäurepentachlorophenylester mit Schmelzpunkt 113-115° erhält.

| | | | |
|---|---|---|---|
| $C_8H_2Cl_5BrO_2$ (387,27) | Ber. | C 24,81 | H 0,52 |
| | Gef. | C 25,1 | H 0,7 |

Beispiel 2: 9,0 g (0,04 Mol) Glycyl-glycyl-glycinamid-hydrochlorid und 9,6 g (0,04 Mol) 2-(2-Oxo-1-pyrrolidinyl)-essigsäure-N-hydroxy-succinimidester werden in 400 ml absolutes DMF eingerührt und mit 1 ml Triethylamin versetzt. Das Reaktionsgemisch wird bei Raumtemperatur 24 Stunden lang gerührt. Der grösste Anteil von DMF wird am Rotationsverdampfer bei 12 Torr und 70° abdestilliert. Der Rückstand wird mit absolutem Ethanol aufgeschwemmt und abgesaugt. Das erhaltene Produkt wird zwecks Entfernung von Triethylammoniumchlorid in 100 ml Wasser gelöst und durch eine Säule mit 60 ml Aberlite IR-120 (starksaurer Kationenaustauscher, $H^+$ – Form) gewaschen. Das wässrige Eluat wird am Rotationsverdampfer eingedampft, in 20-22 ml Wasser gelöst, mit 100 ml

Ethanol versetzt und in einen Kühlschrank (5°) gestellt. Die gebildeten farblosen Kristalle von N-[(2-Oxo-1-pyrrolidinyl)-acetyl]-glycyl-glycyl-glycinamid haben nach dem Absaugen und Abziehen des Lösungsmittels bei 80°/13 Torr einen Schmelzpunkt von 230-232°.

| | | | | |
|---|---|---|---|---|
| $C_{12}H_{19}N_5O_5$ (313,31) | Ber. | C 45,98 | H 6,11 | N 22,36 |
| | Gef. | C 45,6 | H 5,9 | N 22,2 |

Die verwendeten Ausgangsprodukte erhält man wie folgt:

Eine Lösung von 14 g (0,08 Mol) N-tert.Butyloxycarbonyl-glycin (bezogen von der Firma Fluka) in 240 ml DMF wird zunächst mit 22 ml (0,16 Mol) Triethylamin versetzt, anschliessend werden 10,4 ml (0,08 Mol) Chlorameisensäureisobutylester zugetropft. Nach 30 Minuten Rühren bei Raumtemperatur fügt man 13,4 g (0,08 Mol) festes Glycyl-glycinamid-hydrochlorid und 10,4 ml (0,08 Mol) Triethylamin zu und rührt 18 Stunden bei Raumtemperatur. Die Lösung wird mit 6normaler alkoholischer Salzsäure bis zum Farbumschlag des Indikators Kongorot angesäuert und am Rotationsverdampfer bei 60°/12 Torr eingeengt. Der Rückstand wird in 300 ml Ethanol aufgekocht und der darin enthaltene Feststoff wird abgesaugt. Dieser Feststoff wird in 400 ml Wasser gelöst, die Lösung wird filtriert, mit 1000 ml Ethanol verdünnt und in einen Kühlschrank gestellt. Die ausgeschiedenen Kristalle von Glycyl-glycyl-glycinamid-hydrochlorid haben nach dem Absaugen und Entfernen des Lösungsmittels bei 100°/12 Torr einen Schmelzpunkt von 240-243°.

| | | | | |
|---|---|---|---|---|
| $C_6H_{12}N_4O_3 \cdot HCl$ (224,65) | Ber. | C 32,08 | H 5,83 | N 24,94 |
| | Gef. | C 32,0 | H 6,0 | N 24,8 |

Zu einer Suspension von 25,6 g (0,18 Mol) 2-(2-Oxo-1-pyrrolidinyl)-essigsäure und 20,6 g (0,18 Mol) N-Hydroxysuccinimid in 450 ml Dioxan werden 37,6 g (0,18 Mol) Dicyclohexylcarbodiimid in Portionen unter Kühlung mit Eiswasser eingerührt. Anschliessend rührt man 18 Stunden bei Raumtemperatur und saugt den ausgefallenen Dicyclohexylharnstoff

ab. Das Filtrat wird am Rotationsverdampfer bei 60°/12 Torr eingeengt. Der Rückstand wird aus Essigester/Diethylether kristallisiert. Der erhaltene 2-(2-Oxo-1-pyrrolidinyl)-essigsäure-N-hydroxy-succinimidester schmilzt bei 128-130°.

| | | | | | |
|---|---|---|---|---|---|
| $C_{10}H_{12}N_2O_5$ | (240,22) | Ber. | C 50,00 | H 5,04 | N 11,66 |
| | | Gef. | C 49,9 | H 5,2 | N 11,8 |

Beispiel 3: 6,6 g (0,02 Mol) 2-(4-[4-Fluoro-phenyl]-2-oxo-1-pyrrolidinyl)-essigsäure-N-hydroxysuccinimidester und 3,35 g (0,02 Mol) Glycylglycinamid-hydrochlorid werden in 400 ml absolutes DMF eingerührt, wonach man 6,11 ml Triethylamin innerhalb von 10 Min. unter Rühren zufliessen lässt. Die Suspension wird 20 Stunden bei Raumtemperatur gerührt und anschliessend im Vakuum (Wasserstrahlpumpe) eingeengt. Der gelbliche feste Rückstand wird mit 200 ml Wasser verrührt, im Eiswasserbad abgekühlt und abgesaugt. Der noch feuchte farblose Filterrückstand wird in 140 ml Wasser bei 90° gelöst. Die Lösung wird filtriert und ins Eisbad gestellt, worauf N-{(4-[4-Fluoro-phenyl]-2-oxo-1-pyrrolidinyl)-acetyl}-glycyl-glycinamid mit dem Schmelzpunkt 200-202° auskristallisiert.

| | | | | | |
|---|---|---|---|---|---|
| $C_{16}H_{19}N_4O_4$ | (350,35) | Ber. | C 54,9 | H 5,50 | N 16,0 |
| | | Gef. | C 55,3 | H 5,6 | N 16,0 |

Die verwendeten Ausgangsstoffe erhält man wie folgt:

In eine Lösung von 35,6 g (0,15 Mol) 2-(4-[4-Fluoro-phenyl]-2-oxo-1-pyrrolidinyl)-essigsäure und 17,25 g (0,15 Mol) N-Hydroxysuccinimid in 570 ml Dioxan rührt man portionenweise innerhalb von 10 Minuten 31,5 g Dicyclohexylcarbodiimid, wobei man die Reaktionstemperatur durch Kühlung mit Eiswasser unter 30° hält. Nach 18stündigem Rühren bei Raumtemperatur saugt man den entstandenen Dicyclohexylharnstoff ab und dampft das klare Filtrat bis zur Trockne ein. Der ölige Rückstand wird in 200 ml Essigester aufgenommen und mit Hexan bis zur Trübung versetzt, worauf farbloser 4-(4-Fluoro-phenyl)-2-oxo-pyrrolidinyl-essigsäure-N-hydroxysuccinimidester mit Schmelzpunkt 120-123°

auskristallisiert.

Beispiel 4: Zu einer Lösung von 4,9 g (0,031 Mol) 5-Methyl-2-oxo-pyrrolidinyl-essigsäure in 75 ml DMF (purissimum) gibt man unter Rühren bei 10° 4,4 ml Triethylamin. Man kühlt auf -15° und fügt unter Rühren tropfenweise 4,23 g (0,031 Mol) Chlorameisensäure-isobutylester zu. Nach 20minütigem Rühren gibt man zunächst bei -10° 5,2 g (0,034 Mol) Glycyl-glycinamid-hydrochlorid und anschliessend 4,4 ml (0,035 Mol) Triethylamin zu. Man rührt eine Stunde bei 0-5° und 22 Stunden bei 20°, wobei eine Suspension entsteht. Man verdünnt die Reaktionslösung mit 250 ml Ether und saugt das kristalline Rohprodukt ab, das man anschliessend in 120 ml absolutem Ethanol verrührt, um Triethylammoniumchlorid auszuwaschen. Man saugt ab, wäscht mit Diethylether, löst den Filterrückstand in 200 ml Methanol in der Hitze, engt die vorher filtrierte Lösung bis auf 50 ml ein, saugt ab, wäscht mit Ethanol und Ether und trocknet das als Filterrückstand erhaltene N-[(5-Methyl-2-oxo-1-pyrrolidinyl)-acetyl]-glycyl-glycinamid mit dem Schmelzpunkt 197-198°.

| | | | | |
|---|---|---|---|---|
| $C_{11}H_{18}N_4O_4$ (270,29) | Ber. | C 48,88 | H 6,71 | N 20,73 |
| | Gef. | C 49,18 | H 6,44 | N 20,96 |

Beispiel 5: 6,0 g (0,042 Mol) 2-Oxo-pyrrolidinyl-essigsäure und 5,3 ml (0,042 Mol) N-Ethylmorpholin werden in 100 ml Dimethylformamid gelöst und bei -15° mit 5,5 ml (0,042 Mol) Chlorameisensäureisobutylester versetzt. Nach 10 Min. bei -15° gibt man tropfenweise eine Lösung von 7,8 g (0,051 Mol) Glycyl-alaninamid-hydrochlorid und 5,3 ml N-Ethylmorpholin in 200 ml Dimethylformamid derart zu, dass die Temperatur -10° nie überschreitet. Nach einer weiteren Stunde bei -10° und 10 Stunden bei 20° wird das Reaktionsgemisch eingedampft, der Rückstand in Wasser aufgenommen und durch eine Säule von stark basischem Ionenaustauscher (in freier Form, d.h. mit freien Dialkylaminogruppen; Merck III) geleitet. Das Eluat wird eingedampft und der Rückstand aus Methanol-Ether umkristallisiert, worauf man N-[(2-Oxo-1-pyrrolidinyl)--acetyl]-glycyl-(L)-alaninamid mit Schmelzpunkt 196-198°, $[\alpha]_D^{20} = -22°$ (c = 1,204 , Wasser) und $R_f$ = 0,40 (Laufmittelsystem A, d.h. n-Butanol/Pyridin/Essigsäure/Wasser = 42/24/4/30) erhält.

Analog erhält man:

N-[(2-Oxo-1-pyrrolidinyl)-acetyl]-glycyl-(D)-alaninamid mit Schmelzpunkt 195-196°, $[\alpha]_D^{20} = +24°$ (c = 0,873, Wasser ) und $R_f$ = 0,40 (System A),

N-[(2-Oxo-1-pyrrolidinyl)-acetyl]-glycyl-(L)-alanin-N-ethyl-amid mit Schmelzpunkt 195-196°, $[\alpha]_D^{20} = -35°$ (c = 0,858, Wasser) und $R_f$ = 0,48 (System A),

N-[(2-Oxo-1-pyrrolidinyl)-acetyl]-glycyl-(D)-alanin-N-ethyl-amid mit Schmelzpunkt 202-203°, $[\alpha]_D^{20} = +38°$ (c = 0,916, Wasser) und $R_f$ = 0,48 (System A), sowie

N-[(2-Oxo-1-pyrrolidinyl)-acetyl]-glycyl-(L)-alanin-N,N-diethyl-amid und

N-[(2-Oxo-1-pyrrolidinyl)-acetyl]-glycyl-(D)-alanin-N,N-diethyl-amid, beide als Oel und mit $R_f$ = 0,52 (System A).

Das als Ausgangssubstanz verwendete Peptid Glycyl-(L)-alaninamid-hydrochlorid erhält man folgendermassen:

Zu 13,51 g (0,065 Mol) N-Benzyloxycarbonylglycin, 8,0 g (0,0645 Mol) Alaninamid-hydrochlorid und 8,15 ml (0,068 Mol) N-Ethylmorpholin in 200 ml Dimethylformamid gibt man bei 0° 14,65 g (0,071 Mol) Dicyclohexylcarbodiimid und 10,88 g (0,08 Mol) 1-Hydroxybenzotriazol. Nach 15 Stunden bei 20° filtriert man, dampft das Filtrat ein, nimmt den Rückstand in Essigester auf und wäscht diese Lösung mit 0,5normaler wässriger Zitronensäurelösung, 0,5normaler wässriger Natriumhydrogencarbonatlösung und Wasser, trocknet die organische Phase über Natriumsulfat und dampft ein. Nach dem Umkristallisieren des Rückstands aus

Methanol/Ether erhält man N-Benzyloxycarbonyl-glycyl-(L)-alaninamid mit Schmelzpunkt 181-182° und $[\alpha]_D^{20} = -10°$ (c = 0,781, Methanol).

In analoger Weise erhält man:

N-Benzyloxycarbonyl-glycyl-(D)-alaninamid mit Schmelzpunkt 182-183° und $[\alpha]_D^{20} = +12°$ (c = 1,283, Methanol),

N-Benzyloxycarbonyl-glycyl-(L)-alanin-N-ethyl-amid mit Schmelzpunkt 134-136° und $[\alpha]_D^{20} = -27°$ (c = 1,126, Methanol),

N-Benzyloxycarbonyl-glycyl-(D)-alanin-N-ethyl-amid mit Schmelzpunkt 138-140° und $[\alpha]_D^{20} = +29°$ (c = 0,896, Methanol),

N-Benzyloxycarbonyl-glycyl-(L)-alanin-N,N-diethyl-amid mit Schmelzpunkt 92-93° und $[\alpha]_D^{20} = -9°$ (c = 1,627, Chloroform) sowie

N-Benzyloxycarbonyl-glycyl-(D)-alanin-N,N-diethyl-amid mit Schmelzpunkt 90-92° und $[\alpha]_D^{20} = +9°$ (c = 1,724, Chloroform).

Eine Lösung von 11,7 g (0,042 Mol) N-Benzyloxycarbonyl-glycyl-alaninamid in 400 ml Methanol und 41,9 ml 1,0 normaler Salzsäure wird in Gegenwart von 1,2 g Palladiumkohle (10% Pd) bei 22°/840 Torr hydriert, wobei man das entstehende $CO_2$ in einem zweiten Hydriergefäss mit 1normaler wässriger Kaliumhydroxidlösung absorbiert. Nach beendeter Wasserstoffaufnahme (ca. 40 Min.) filtriert man vom Katalysator ab und dampft das Filtrat ein.

In analoger Weise spaltet man die Benzyloxycarbonylschutzgruppe in den anderen obengenannten Peptiden ab.

Die erhaltenen Glycyl-alaninamid-hydrochloride verwendet man direkt für die eingangs dieses Beispiels geschilderte Reaktion.

<u>Beispiel 6</u>: 4,4 g (0,011 Mol) 4-(4-Chloro-phenoxy)-phenyl-2-oxo-pyrrolidinyl-essigsäure-N-hydroxy-succinimidester und 1,7 g (0,01 Mol) Glycyl-glycinamid-hydrochlorid in 100 ml DMF werden mit 2,8 ml

Triethylamin versetzt und 20 Stunden bei Raumtemperatur gerührt. Der nach dem Eindampfen am Rotationsverdampfer verbleibende feste Rückstand wird mit 50 ml Wasser bei 50° verrührt, abgesaugt und mit 10 ml Wasser gewaschen. Der feste Filterrückstand, der nach dem Trocknen bei 204-208° schmilzt, wird in 200 ml Wasser aufgekocht, bei Raumtemperatur 3 Stunden stehen gelassen, abgesaugt und getrocknet.

| | | | | | |
|---|---|---|---|---|---|
| $C_{22}H_{23}ClN_4O_4$ | (458,90) | Ber. | C 57,58 | H 5,05 | N 12,21 |
| | | Gef. | C 57,6 | H 5,3 | N 11,7 |

Das erhaltene N-{(4-[4-Chloro-phenoxy]-phenyl-2-oxo-1-pyrrolidinyl)-acetyl}-glycyl-glycinamid schmilzt bei 211-213°.

Den als Ausgangssubstanz verwendeten Succinimidester erhält man folgendermassen:

38,5 g (0,111 Mol) 4-(4-Chloro-phenoxy)-phenyl-2-oxo-pyrrolidinyl-essigsäure, 12,7 g (0,111 Mol) N-Hydroxy-succinimid und 25,1 g (0,122 Mol) Dicyclohexylcarbodiimid in 1500 ml Dioxan werden 20 Stunden bei Raumtemperatur gerührt. Der gebildete Dicyclohexylharnstoff wird abgesaugt und das Filtrat bis zur Trockne eingedampft. Der amorphe feste Rückstand wird aus Essigester-Diethylether kristallisiert. Nach dem Absaugen und Trocknen erhält man 4-(4-Chloro-phenoxy)-phenyl-2-oxo-pyrrolidinyl-essigsäure-N-hydroxy-succinimidester mit Schmelzpunkt 132-134°.

| | | | | | |
|---|---|---|---|---|---|
| $C_{22}H_{19}ClN_2O_6$ | (442,86) | Ber. | C 59,7 | H 4,3 | N 6,3 |
| | | Gef. | C 60,1 | H 4,4 | N 6,3 |

Beispiel 7: 7,2 g (0,027 Mol) 2-(5,5-Dimethyl-2-oxo-1-pyrrolidinyl)-essigsäure-(2,5-dioxo-1-pyrrolidinyl)-ester, 4,5 g (0,027 Mol) Glycyl-glycinamid-hydrochlorid und 7 ml Triethylamin werden in 90 ml absolutem Dimethylformamid 48 Stunden bei Raumtemperatur gerührt.

Das ausgefallene Triethylamin-hydrochlorid wird abgesaugt und das Filtrat bei 70°/14 Torr bis zur Trockne eingedampft. Der als viskoses Oel angefallene Rückstand wird über 200 g Kieselgel 60 (Merck A.G.) chromatographiert. Die erwünschte Substanz wird mit Methanol eluiert, das Eluat eingedampft und aus Ethanol-Ether kristallisiert, worauf man N-[(5,5-Dimethyl-2-oxo-1-pyrrolidinyl)-acetyl]-glycyl-glycinamid mit Schmelzpunkt 138-140° erhält.

| | | | | | |
|---|---|---|---|---|---|
| $C_{12}H_{20}N_4O_4$ | (284,32) | Ber. | C 50,70 | H 7,09 | N 19,71 |
| | | Gef. | C 50,5 | H 7,0 | N 19,3 |

Den als Ausgangsprodukt verwendeten Succinimidester erhält man folgendermassen:
27,8 g (0,162 Mol) 2-(5,5-Dimethyl-2-oxo-1-pyrrolidinyl)-essigsäure und 18,6 g (0,162 Mol) N-Hydroxy-succinimid werden in 400 ml Dioxan gelöst und portionsweise mit 34 g (0,162 Mol) N,N'-Dicyclohexyl-carbodiimid versetzt. Die Temperatur wird bei 27-30° gehalten. Nach 20 Stunden Rühren bei Raumtemperatur wird der entstandene Dicyclohexylharnstoff abgesaugt. Das Filtrat wird bei 60°/14 Torr eingedampft. Das als Rückstand erhaltene, gelbe, viskose Oel wird in 100 ml Essigester gelöst und mit 100 ml Ether versetzt. Im Eisbad kristallisiert 2-(5,5-Dimethyl-2-oxo-1-pyrrolidinyl)-essigsäure-(2,5-dioxo-1-pyrrolidinyl)-ester mit Schmelzpunkt 100-105°.

| | | | | |
|---|---|---|---|---|
| $C_{12}H_{16}N_2O_5$ | (268,27) | Ber. | C 53,73 | H 6,01 |
| | | Gef. | C 53,4 | H 6,1 |

Den Ausgangsstoff erhält man folgendermassen:
53,2 g (0,287 Mol) 2-(5,5-Dimethyl-2-oxo-1-pyrrolidinyl)-essigsäureethylester werden mit 19 g (0,287 Mol) 85%iger wässeriger Kaliumhydroxidlösung in 300 ml Methanol 2 Stunden unter Rückfluss gekocht. Das Reaktionsgemisch wird bei 60°C/14 Torr bis zur Trockene eingedampft. Der Rückstand wird in 500 ml Wasser gelöst, mit konzentrierter Salzsäure auf pH 4 gestellt und eingedampft. Der Rückstand wird in 200 ml Ethanol verrieben. Man saugt ab und dampft das Filtrat bis zur Trockne ein, löst in 100 ml Ethanol und verdünnt mit 400 ml Ether. Beim Stehen im Eisbad kristallisiert 2-(5,5-Dimethyl-2-oxo-1-pyrrolidinyl)-essigsäure mit Schmelzpunkt 128-130°.

| | | | | | |
|---|---|---|---|---|---|
| $C_8H_{13}NO_3$ | (171,30) | Ber. | C 56,13 | H 7,66 | N 8,18 |
| | | Gef. | C 55,9 | H 7,7 | N 8,1 |

Den Ausgangsstoff erhält man folgendermassen:
Aus 11,5 g (0,5 Grammatom) Natrium wird mit 350 ml Methanol eine Natriummethanolatlösung hergestellt. In diese Lösung werden 56,5 g (0,5 Mol) 5,5-Dimethyl-2-pyrrolidon [Org. Synthesis IV, 357 (1963)] eingerührt. Nach 3 Stunden wird das Reaktionsgemisch bis zur Trockne eingedampft. Das trockene Natriumsalz des Pyrrolidons wird in 250 ml Toluol suspendiert und tropfenweise mit 53 ml Bromessigsäuremethylester in 50 ml Toluol versetzt. Die Suspension wird bei Raumtemperatur 20 Stunden gerührt.

Das Reaktionsgemisch wird mit 200 ml Wasser extrahiert. Die organische Schicht wird über Natriumsulfat getrocknet, filtriert und eingedampft. Der Rückstand wird im Hochvakuum fraktioniert. Die Fraktion mit Siedepunkt 104°/0,005 Torr besteht aus 2-(5,5-Dimethyl-2-oxo-1-pyrrolidinyl)-essigsäureethylester.

| | | | | | |
|---|---|---|---|---|---|
| $C_9H_{15}NO_3$ | (185,22) | Ber. | C 58,36 | H 8,16 | N 7,56 |
| | | Gef. | C 58,0 | H 8,5 | N 7,4 |

Beispiel 8: Ein Gemisch von 14,6 g (0,04 Mol) 2-[4-(1-Naphthyl)-2-oxo-1-pyrrolidinyl]-essigsäure-(2,5-dioxo-1-pyrrolidinyl)-ester, 6,7 g (0,04 Mol) Glycyl-glycinamid-hydrochlorid und 8,1 g (11 ml) Triethylamin in 150 ml Dimethylformamid wird bei 50°C 20 Stunden lang gerührt. Der grösste Teil des entstandenen Triethylaminhydrochlorids wird abgesaugt. Das klare Filtrat wird bei 70°/14 Torr eingedampft. Der ölige Rückstand wird in 50 ml Wasser eingerührt und 20 Stunden bei 5°C stehen gelassen. Der kristalline Niederschlag wird abgesaugt und dreimal aus Dimethylformamid/Ether umkristallisiert, worauf man N-{[4-(1-Naphthyl)-2-oxo-1-pyrrolidinyl]-acetyl}-glycyl-glycinamid mit Schmelzpunkt 206-208° erhält.

| | | | | | |
|---|---|---|---|---|---|
| $C_{20}H_{22}N_4O_4$ | (382,42) | Ber. | C 62,82 | H 5,80 | N 14,65 |
| | | Gef. | C 62,3 | H 5,9 | N 14,4 |

Den als Ausgangsprodukt verwendeten Succinimidester erhält man folgendermassen:
Ein Gemisch von 11,5 g (0,043 Mol) 2-[4-(1-Naphthyl)-2-oxo-1-pyrrolidinyl]-essigsäure, 4,9 g (0,043 Mol) N-Hydroxysuccinimid und 8,9 g (0,043 Mol) Dicyclohexylcarbodiimid werden in 200 ml absolutem Dioxan bei Raumtemperatur 20 Stunden lang gerührt.
Der ausgeschiedene Dicyclohexylharnstoff wird abgesaugt. Das klare Filtrat wird bei 60°/14 Torr bis zur Trockne eingedampft, worauf man 2-[4-(1-Naphthyl)-2-oxo-1-pyrrolidinyl]-essigsäure-(2,5-dioxo-1-pyrrolidinyl)-ester in Form eines Oeles erhält, das direkt weiterverwendet wird.

Den Ausgangsstoff erhält man folgendermassen:
19,2g (0,065 Mol) 2-[4-(1-Naphthyl)-2-oxo-1-pyrrolidinyl]-essigsäure-ethylester werden in 100 ml Methanol gelöst und mit 65 ml wässriger 1 N Natronlauge versetzt. Das Gemisch wird 20 Stunden bei Raumtemperatur gerührt und dann bei 60°/14 Torr bis zur Trockne eingedampft. Der Rückstand wird in 100 ml Wasser aufgenommen und mit 6 N HCl auf pH 1 gestellt. Die ausgefallene 2-[4-(1-Naphthyl)-2-oxo-1-pyrrolidinyl]-essigsäure kristallisiert im Eisbad und wird

abgesaugt.
Nach dem Umkristallisieren aus 350 ml Methanol schmilzt sie bei 212-214°.

| | | | | |
|---|---|---|---|---|
| $C_{16}H_{15}NO_3$ (269,30) | Ber. | C 71,36 | H 5,62 | N 5,20 |
| | Gef. | C 71,2 | H 5,6 | N 5,1 |

Den Ausgangsstoff erhält man folgendermassen:
42,2 g (0,2 Mol) 4-(1-Naphthyl)-2-oxo-pyrrolidin werden in 300 ml Dimethylformamid gelöst und bei Raumtemperatur portionsweise mit 8,8 g (0,2 Mol) einer 55%igen Suspension von Natriumhydrid in Mineralöl versetzt. Nach vollendeter Zugabe wird noch 2 Stunden bei Raumtemperatur gerührt.
Zu diesem Gemisch wird eine Lösung von 22,4 ml (0,2 Mol) Bromessigsäureethylester in 120 ml Toluol innerhalb von 20 Minuten bei 15°C zugetropft. Das Reaktionsgemisch wird 18 Stunden bei Raumtemperatur und 8 Stunden bei 80° gerührt.
Das Reaktionsgemisch wird von Toluol und Dimethylformamid bei 70°/14 Torr befreit. Der ölige Rückstand wird mit Essigester verdünnt und mit gesättigter Natriumchloridlösung gewaschen. Nach dem Trocknen über Natriumsulfat und Eindampfen wird der ölige Rückstand zweimal mit 50 ml Hexan verrührt, um das Mineralöl zu entfernen. Der Rückstand wird im Hochvakuum destilliert. Die Fraktion bei 190° / 0,005 Torr ist ein gelbliches, klares Oel, bestehend aus 2-[4-(1-Naphthyl)-2-oxo-1-pyrrolidinyl]-essigsäure-ethylester.

| | | | | |
|---|---|---|---|---|
| $C_{18}H_{19}NO_3$ (297.35) | Ber. | C 72,71 | H 6,44 | N 4,71 |
| | Gef. | C 72,4 | H 6,7 | N 4,8 |

Beispiel 9: In 300 ml trockenes Dimethylformamid werden 11,7 g (30 mMol) 2-(2-Oxo-1-pyrrolidinyl)-essigsäure-pentachlorphenylester und 10,2 g (30 mMol) Glycyl-glycyl-glycyl-glycyl-glycinamid-hydrochlorid eingerührt und dann mit 4,6 ml (33 mMol) Triethylamin versetzt. Die Suspension wird innerthalb von 30 Minuten auf 70°C erwärmt und eine Stunde bei dieser Temperatur gerührt. Die weisse Suspension wird auf 10° abgekühlt und bei 13 Torr abgesaugt. Das

Filtergut wird mit Ethanol und Ether gewaschen und zweimal aus je 300 ml siedendem Wasser umkristallisiert, worauf man N-[(2-Oxo-1-pyrrolidinyl)-acetyl]-glycyl-glycyl-glycyl-glycyl-glycinamid mit Zersetzungsbereich > 300° erhält.

| | | | | | |
|---|---|---|---|---|---|
| $C_{16}H_{25}N_7O_7$ | (427,42) | Ber. | C 44,96 | H 5,90 | N 22,94 |
| | | Gef. | C 44,6 | H 5,8 | N 22,6 |

Das Ausgangsprodukt wird wie folgt hergestellt:
Zu einer Lösung von 23,2 g (0,1 Mol) N-tert.-Butyloxycarbonyl-glycyl-glycin mit 14 ml Triethylamin in 700 ml Dimethylformamid werden bei -10°C 13,7 g (0,1 Mol) Chlorameisensäureisobutylester zugetropft. Nach beendeter Zugabe rührt man noch 10 Minuten und rührt dann bei unveränderter Temperatur (-10°C) 22,5 g (0,1 Mol) Glycyl-glycyl-glycinamid-hydrochlorid ein. Nach Zugabe von 14 ml Triethylamin erwärmt man langsam auf Raumtemperatur und rührt dann 18 Stunden bei 40°C.
Zur Abspaltung der Schutzgruppe setzt man nun 20 ml 21%ige ethanolische Salzsäurelösung und 1 ml Wasser zu und rührt 1 Stunde bei 40°C. Das Reaktionsgemisch wird bei 70°/14 Torr bis zur Trockne eingedampft. Der Rückstand wird in 600 ml Ethanol aufgekocht und heiss abgesaugt. Der Filterrückstand wird in 250 ml Wasser auf 90° erwärmt. Man gibt wässrige 2 N Salzsäure bis zum Umschlagspunkt des Indikators Kongorot nach blau (pH < 4) zu, behandelt mit Aktivkohle und versetzt das Filtrat mit 700 ml Ethanol. Im Eisbad kristallisiert Glycyl-glycyl-glycyl-glycyl-glycinamid-hydrochlorid mit einem Zersetzungsbereich > 300.

| | | | | | |
|---|---|---|---|---|---|
| $C_{10}H_{18}N_6O_5 \cdot HCl$ | (338,75) | Ber. | C 35,46 | H 5,65 | N 24,81 |
| | | Gef. | C 35,6 | H 5,8 | N 24,4 |

Beispiel 10: Ein Gemisch von 5,6 g (0,02 Mol), Glycyl-glycyl-glycyl-glycinamid-hydrochlorid, 3,1 ml (0,022 Mol) Triethylamin und 11,7 g (0,02 Mol) 2-(2-Oxo-1-pyrrolidinyl)-essigsäure-pentachlorphenylester wird in 400 ml Dimethylformamid unter Stickstoffatmosphäre bei 60°

15 Stunden lang gerührt. Das Reaktionsgemisch wird auf 15° abgekühlt und abgesaugt. Die kristalline Masse auf dem Filter wird mit 96%igem Ethanol verrührt, abgesaugt und mit Ether gewaschen.
Der Filterrückstand wird in 100 ml Wasser bei 90° gelöst. Die leicht trübe Lösung wird filtriert und langsam auf Raumtemperatur abgekühlt. Das ausgefallene Produkt wird abgesaugt und mit Ethanol und Ether gewaschen, worauf man N-[(2-Oxo-1-pyrrolidinyl)-acetyl]-glycyl-glycyl-glycyl-glycinamid mit Zersetzungsbereich 268-272° erhält.

| | | | | | |
|---|---|---|---|---|---|
| $C_{14}H_{22}N_6O_6$ | (370,37) | Ber. | C 45,4 | H 6,0 | N 22,7 |
| | | Gef. | C 44,8 | H 6,2 | N 22,7 |
| | | Gef. | C 45,1 | H 5,7 | N 22,4 |

Das Ausgangsmaterial wird folgendermassen hergestellt:
In 700 ml Dimethylformamid werden 23,2 g (0,1 Mol) N-tert.Butyloxycarbonyl-glycyl-glycin eingerührt. Die Lösung wird auf -10°C abgekühlt und mit 14 ml (0,1 Mol) Triethylamin versetzt. Bei -10° werden 13,7 g (0,1 Mol) Chlorameisensäureisobutylester zugetropft. Nach 10 Minuten Rühren werden nochmals 14 ml (0,1 Mol) Triethylamin, und dann 16,8 g (0,1Mol) Glycyl-glycinamid-hydrochlorid als Feststoff zugegeben. Das Reaktionsgemisch wird 40 Stunden bei Raumtemperatur gerührt.
Zur Abspaltung der Schutzgruppe wird die trübe gelbliche Suspension mit 1 ml Wasser versetzt und mit ethanolischer Salzsäure (21%ig) auf pH 1 gestellt. Das Reaktionsgemisch wird bei 60°C/14 Torr bis zur Trockne eingedampft. Der erhaltene Feststoff wird in 200 ml Wasser bei 80° gelöst, mit Aktivkohle behandelt und abgesaugt. Das klare Filtrat wird mit 700 ml Ethanol verdünnt und im Eisbad gerührt. Das ausfallende Produkt wird abgesaugt, in 100 ml Wasser bei 80° gelöst und die Lösung mit 500 ml Ethanol versetzt. Nach dem Absaugen und Trocknen erhält man Glycyl-glycyl-glycyl-glycinamid-hydrochlorid in kristalliner Form, das oberhalb von 280° schmilzt.

| | | | | | |
|---|---|---|---|---|---|
| $C_8H_{14}N_5O_4 \cdot HCl$ | (281,70) | Ber. | C 34,1 | H 5,7 | N 24,9 |
| | | Gef. | C 34,0 | H 5,5 | N 24,3 |

Beispiel 11: 3,3 g (0,01 Mol) N-tert.Butyloxycarbonyl-L-prolyl-glycyl-glycinamid werden in 10 ml 1 N HCl während 2 Stunden bei 80° gerührt. Die klare Lösung wird bei 70°/13 Torr eingedampft und noch zweimal mit je 50 ml Toluol eingedampft. Der Rückstand wird in 60 ml Dimethylformamid aufgenommen, mit 3,91 g (10 mMol) 2-(2-Oxo-pyrrolidin-1-yl)-essigsäurepentachlorphenylester und 1,4 ml (10 mMol) Triethylamin versetzt. Das Reaktionsgemisch wird bei 50°C 20 Stunden gerührt und dann bei 70°/13 Torr bis zur Trockne eingedampft. Der viskose Rückstand wird mit 50 ml Essigsäureethylester in der Wärme verrührt. Die Essigesterschicht enthält Pentachlorphenol. Der glasige Rückstand wird an 100 g Silicagel (Kieselgel 60, 70-230 mesh, Merck AG) chromatographiert. Die mit Chloroform/Methanol = 20/1 eluierten Fraktionen werden vereinigt. Das nach Abdampfen des Lösungsmittels erhaltene Rohprodukt wird in 25 ml Wasser aufgenommen und durch eine kurze Säule mit 10 g Dowex 50 (Kationenaustauscher, $H^+$-Form) gewaschen. Das wässrige Eluat wird bis zur Trockne eingedampft, worauf man N-[(2-Oxo-1-pyrrolidinyl)-acetyl]-L-prolyl-glycyl-glycinamid in Form eines amorphen Feststoffes erhält.

| | | | | | |
|---|---|---|---|---|---|
| $C_{15}H_{23}N_5O_5$ | (353,38) | Ber. | C 50,99 | H 6,56 | N 19,82 |
| | | Gef. | C 50,8 | H 6,6 | N 19,8 |

100 MHz-$^1$H-NMR (Dimethylsulfoxid-$d_6$): $\delta$ = 1,7-2,4 (8H), 3,3-3,6 (4H), 3,8 (4H), 4,1 (2H), 4,35 (1H), 6,7 (1H), 6,95 (1H), 7,9 (1H) und 8,6 (1H).

Das Zwischenprodukt wird folgendermassen hergestellt:
Ein Gemisch von 23,2 g (0,05 Mol) N-tert.Butyloxycarbonyl-L-prolin-pentachlorphenylester (Smp. 113-115°), 5,8 g (0,05 Mol) Glycyl-glycinamid-hydrochlorid und 7 ml (0,05 Mol) Triethylamin in 250 ml Dimethylformamid wird bei 60° unter Stickstoffatmosphäre gerührt. Nach 15 Stunden Rühren wird das Reaktionsgemisch bei 70°/13 Torr bis 80 ml eingeengt und nochmals 20 Stunden bei 80° gerührt. Nach Abkühlen auf Raumtemperatur wird das ausgeschiedene Triethylamin-hydrochlorid abgesaugt. Das Filtrat wird bei 70°/15 Torr bis zur Trockne eingedampft. Das erhaltene viskose Oel wird mit 100 ml Toluol und 100 ml Ether verrührt. Nach 90 Minuten Rühren wird ein

amorpher Feststoff erhalten, der in heissem Dioxan löslich ist und aus Dioxan/Essigester/Ether=7/10/10 kristallisiert wird, worauf man N-tert.Butyloxycarbonyl-L-prolyl-glycyl-glycinamid mit Schmelzpunkt 171-172° erhält.

| | | | | |
|---|---|---|---|---|
| $C_{14}H_{24}N_4O_5$ (328,37) | Ber. | C 51,21 | H 7,37 | N 17,06 |
| | Gef. | C 51,3 | H 7,2 | N 16,8 |

Beispiel 12: In analoger Weise, wie in dieser Anmeldung beschrieben, erhält man
N-[4-Hydroxy-2-(2-oxo-1-pyrrolidinyl)-butyryl]-glycyl-glycinamid.

Beispiel 13: Rezept für eine Tablette:

| | |
|---|---|
| N-[(2-Oxo-1-pyrrolidinyl)-acetyl]-glycyl-(L)-alaninamid | 100 mg |
| Stärke | 15 mg |
| Polyvinylpyrrolidon | 2 mg |
| Talkum | 6 mg |
| Magnesiumstearat | 1 mg |

Beispiel 14: Herstellung von 1000 Tabletten, die je 100 mg N-[(2-Oxo-1-pyrrolidinyl)-acetyl]-glycyl-glycyl-glycinamid enthalten:

Zusammensetzung

| | |
|---|---|
| N-[(2-Oxo-1-pyrrolidinyl)-acetyl]-glycyl-glycyl-glycinamid | 100 g |
| Lactose | 100 g |
| Kartoffelstärke | 70 g |
| Gelatine | 1,6 g |
| Talk | 12 g |
| Magnesiumstearat | 2 g |
| Silicumdioxid (hochdispers) | 4 g |
| Ethanol | q.s. |

Der Wirkstoff wird mit der Lactose und 60 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Silicumdioxid zu und presst das Gemisch zu Tabletten von je 289,6 mg Gewicht und dem oben angegebenen Wirkstoffgehalt, die gewünschtenfalls mit Teilkerben zur feineren Anpassung der Dosierung versehen sein können.

Beispiel 15: 20,7 g (0,1 Mol) 2-(5,5-Dimethyl-2-oxo-1-pyrrolidinyl)-propionsäure-natriumsalz (beschrieben in der Deutschen Offenlegungsschrift 27 47 369) und 16,8 g (0,1 Mol) Glycyl-glycinamid-hydrochlorid werden in 150 ml Dimethylformamid während 10 Minuten gerührt und dann mit 32,1 g (0,105 Mol) Triphenylphosphit versetzt. Anschliessend wird unter Stickstoffatmosphäre und Rühren während 2 1/2 Stunden auf 90-95° erwärmt, wobei sich eine gelbe Suspension bildet. Zur Aufarbeitung wird auf 5° abgekühlt und mit 300 ml Diethylether versetzt. Das ausgefallene Rohprodukt wird abgenutscht, in Isopropanol erwärmt, durch Filtration von unlöslichen anorganischen Salzen befreit und dann bis zur beginnenden Kristallisation eingeengt. Das auskristallisierte N-[2-(5,5-Dimethyl-2-oxo-1-pyrrolidinyl)-propionyl]-glycyl-glycinamid wird abgenutscht und im Vakuum bei 80° getrocknet; Schmelzpunkt 218-220°.

Beispiel 16: 3,17 g (0,01 Mol) N-(3-Carboxy-propyl)-glycyl-L-prolyl-glycyl-glycinamid werden in 25 ml Hexamethyldisilazan suspendiert und unter Rühren zum Sieden erhitzt. Nach kurzer Zeit bildet sich eine klare Lösung. Man erhitzt während 24 Stunden unter Rückfluss und dampft dann das rohe Reaktionsgemisch im Hochvakuum ein. Der Rückstand wird in 30 ml Methanol gelöst und bei Raumtemperatur gerührt. Das ausgefallene, in Beispiel 11 beschriebene N-[(2-Oxo-1-pyrrolidinyl)-acetyl]-L-prolyl-glycyl-glycinamid wird abgenutscht und mit wenig kaltem Methanol nachgewaschen.

Das Ausgangsmaterial wird wie folgt hergestellt:

10,3 g (0,10 Mol) 4-Amino-buttersäure, 28 g (0,20 Mol) Kaliumcarbonat und 17,2 g (0,05 Mol) N-Bromacetyl-L-prolyl-glycyl-glycinamid werden in 300 ml Ethanol während 8 Stunden bei 50° gerührt. Hierauf wird mit 2N Salzsäure vorsichtig neutralisiert und dann im Wasserstrahlvakuum eingedampf. Der Rückstand wird mit 250 ml Isopropanol versetzt und zum Kochen erwärmt. Die unlöslichen Salze werden abgenutscht und das Filtrat am Rotationsverdampfer bis zur beginnenden Kristallisation eingeengt. Man erhält so das rohe N-(3-Carboxy-propyl)-glycyl-L-prolyl-glycyl-glycinamid, das direkt weiterverarbeitet wird.

Beispiel 17: 3,9 g (0,01 Mol) N-(4-Chlor-butyryl)-glycyl-L-prolyl-glycyl-glycinamid und 1,5 g (0,011 Mol) Kaliumcarbonat werden unter Rühren in 120 ml Ethanol während 5 Stunden zum Sieden erhitzt. Hierauf dampft man im Rotationsverdampfer ein, digeriert den Rückstand in 200 ml kochendem Isopropanol, filtriert von den unlöslichen anorganischen Salzen ab und engt im Wasserstrahlvakuum bis zur beginnenden Kristallisation ein. Das ausgefallene, in Beispiel 11 beschriebene N-[(2-Oxo-1-pyrrolidinyl)-acetyl]-L-prolyl-glycyl-glycinamid wird abgenutscht und mit wenig Isopropanol nachgewaschen.

Das Ausgangsmaterial wird wie folgt hergestellt:

Zu einer Mischung von 3,1 g (0,01 Mol) Glycyl-L-prolyl-glycyl-glycinamid-hydrochlorid und 2,0 g (0,02 Mol) Triethylamin in 50 ml Dimethylformamid lässt man unter Rühren bei einer Reaktionstemperatur von 5° 1,5 g (0,011 Mol) 4-Chlor-buttersäurechlorid zutropfen. Nach erfolgter Zugabe lässt man noch 1 Stunde bei 5-20° ausreagieren. Dann wird das Dimethylformamid im Hochvakuum abdestilliert. Der Rückstand wird mit 200 ml Ethanol aufgeschlämmt und das unlösliche N-(4-Chlorbutyryl)-glycyl-L-prolyl-glycyl-glycinamid abgenutscht.

Beispiel 18: Zu einer Lösung von 1,70 g (0,02 Mol) 2-Oxo-pyrrolidin in 50 ml Dimethylformamid gibt man unter Rühren und Stickstoffatmosphäre 0,86 g (0,02 Mol) einer 57proz. Dispersion von Natriumhydrid in Mineralöl, wobei eine starke Wasserstoffgasentwicklung stattfindet. Man lässt während 30 Minuten bei Raumtemperatur ausreagieren und versetzt dann mit 6,94 g (0,02 Mol) N-(Bromacetyl)-L-prolyl-glycyl-glycinamid. Das Reaktionsgemisch wird während 15 Stunden bei Raumtemperatur gerührt. Zur Aufarbeitung wird das Dimethylformamid im Hochvakuum eingedampft und der Rückstand in 250 ml heissem Ethanol digeriert. Man nutscht von den unlöslichen anorganischen Salzen ab und engt das Filtrat im Wasserstrahlvakuum bis zur beginnenden Kristallisation ein. Das ausgefallene, in Beispiel 11 beschriebene N-[(2-Oxo-1-pyrrolidinyl)-acetyl]-L-prolyl-glycyl-glycinamid wird abgenutscht und mit wenig Ethanol nachgewaschen.

Beispiel 19: 2,97 g (0,01 Mol) N-[(2-Oxo-1-pyrrolidinyl)-acetyl]-L-prolyl-glycin und 1,1 g (0,01 Mol) Glycinamid-hydrochlorid werden in 50 ml Dimethylformamid unter Rühren und Stickstoffatmosphäre nacheinander mit 1,1 g (0,011 Mol) Triethylamin und 3,26 g (0,0105 Mol) Triphenylphosphit versetzt. Hierauf wird das Reaktionsgemisch während 3 Stunden auf 85-90° erwärmt, wobei eine klare Lösung entsteht. Dann kühlt man auf 5° ab und versetzt mit 30 ml Diethylether. Der Niederschlag wird abgenutscht, zur Abtrennung von Triethylamin-hydrochlorid in 20 ml Ethanol aufgeschlämmt und dann wieder abgenutscht, worauf man das in Beispiel 11 beschriebene N-[(2-Oxo-1-pyrrolidinyl)-acetyl]-L-prolyl-glycyl-glycinamid erhält.

Beispiel 20: 3,67 g (0,01 Mol) N-[(2-Oxo-1-pyrrolidinyl)-acetyl]-L-prolyl-glycyl-glycin-methylester werden in 150 ml Methanol gelöst und unter Rühren wird Ammoniakgas eingeleitet. Man lässt insgesamt während 15 Stunden bei Raumtemperatur reagieren, nutscht dann das ausgefallene, in Beispiel 11 beschriebene N-[(2-Oxo-1-pyrrolidinyl)-acetyl]-L-prolyl-glycyl-glycinamid ab und kristallisiert dieses aus Methanol um.

Beispiel 21: In ein Chromatographie-Gefäss mit 2,5 cm Durchmesser werden 50 ml feucht-aufgeschlämmtes AMBERLITE® IRA-400 (starkbasisches Anionenaustauscherharz auf Polystyrol-Basis) in Chloridform eingetragen und mit einem Gemisch von 20 ml Triethylamin, 20 ml Ethanol und 60 ml Wasser überschichtet. Das Gemisch wird tropfenweise durchgelassen und dann mit destilliertem Wasser solange gespült, bis der Effluent neutral ist.

Das auf diese Weise aktivierte und gewaschene Amberlite® IRA 400 wird in einen Rundkolben transferiert und das Wasser abdekantiert. Eine wässrige Lösung (150 ml) von 3,35 g (0,01 Mol) N-[(2-Oxo-1-pyrrolidinyl)-acetyl]-L-prolyl-glycin-N-cyanomethyl-amid wird mit dem aktivierten Anionenaustauscher unter magnetischem Rühren 5 Stunden lang unter Rückfluss gekocht. Bei Raumtemperatur wird der Ionenaustauscher auf einer Nutsche abgesaugt und das klare wässrige Filtrat bei 60°/14 Torr bis zur Trockene eingedampft. Das im Rückstand zurückbleibende, in Beispiel 11 beschriebene N-[(2-Oxo-1-pyrrolidinyl)-acetyl]-L-prolyl-glycyl-glycinamid wird durch Umkristallisation aus Methanol gereinigt.

Beispiel 22: 8,6 g (20,2 mMol) N-tert.Butyloxycarbonyl-L-prolyl-L-prolyl-glycyl-glycinamid werden 2 Stunden bei 60° in 20,2 ml 1N wässriger Salzsäure gerührt. Die Lösung wird bei 80°/14 Torr bis zur Trockene eingedampft, zweimal mit 20 ml abs. Ethanol eingedampft und über Nacht bei 90°/14 Torr über Calciumchlorid getrocknet. Der glasige Rückstand wird mit 100 ml Dimethylformamid (DMF) und 2,82 ml Triethylamin versetzt und bei 40° gerührt bis eine klare Lösung entsteht. Zu dieser Lösung wird tropfenweise eine Lösung von 9,0 g (23 mMol) 2-(2-Oxo-1-pyrrolidinyl)-essigsäure-pentachlorphenylester in 60 ml DMF zugetropft. Die Zugabe dauert 3 Stunden bei einer Innentemperatur von 50°. Das Lösungsmittel wird bei 60°/1 Torr abdestilliert. Der Rückstand wird zwischen 50 ml Wasser und 50 ml Diethylether verteilt und die wässrige Schicht noch zweimal mit Diethylether gewaschen, um alles Pentachlorphenol zu entfernen.

Die wässrige Schicht wird mit Aktivkohle geklärt und filtriert. Die klare, farblose, wässrige Lösung wird durch eine Säule von 20 ml neutralgewaschenem Dowex® 50 (Kationenaustauscher), $H^+$-Form, passieren lassen und mit 50 ml Wasser nachgewaschen. Das wässrige Eluat wird bei 60°/14 Torr bis zur Trockene eingedampft.

Nach längerem Stehen bei Raumtemperatur (20°) erfolgt spontane Kristallisation. Die Kristalle werden mit Diethylether überschichtet, verrieben und abgesaugt, worauf man N-[(2-Oxo-1-pyrrolidinyl)-acetyl]-L-prolyl-L-prolyl-glycyl-glycinamid mit Schmelzpunkt 135-138° (Wasserverlust bei 98-120°) erhält.

| | | | | | |
|---|---|---|---|---|---|
| $C_{20}H_{30}N_6O_6 \cdot 2H_2O$ (486,53) | Ber. | C 49,37 | H 7,04 | N 17,27 | $H_2O$ 7,4 |
| | Gef. | C 49,7 | H 6,9 | N 17,4 | $H_2O$ 6,6 |

Das Ausgangsmaterial, N-tert.Butyloxycarbonyl-L-prolyl-L-prolyl-glycyl-glycinamid erhält man aus 19,3 g (34 mMol) N-tert.Butyloxycarbonyl-L-prolyl-L-prolyl-pentachlorphenylester (Schmelzpunkt 153-154°) und 3,9 g (34 mMol) Glycyl-glycinamid-hydrochlorid in 150 ml DMF in Gegenwart von 4,7 ml (34 mMol) Triethylamin.

Patentansprüche für die Vertragsstaaten BE, CH, DE, FR, GB, IT, LU, NL und SE:

1. Lactampeptide der Formel I,

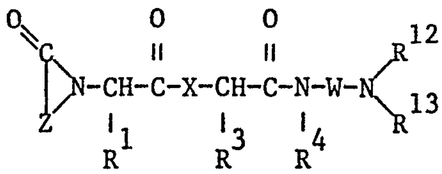

(I)

worin Z einen Alkylenrest bedeutet, der unsubstituiert oder durch freies oder funktionell abgewandeltes Hydroxy und/oder durch Aryl substituiert ist und der zusammen mit der Amidgruppierung einen fünfgliedrigen Ring bildet,

$R^1$ Wasserstoff, unsubstituiertes oder durch Aryl oder durch freies oder funktionell abgewandeltes Hydroxy substituiertes Niederalkyl oder Aryl,

X Sauerstoff oder die Gruppe $NR^2$, worin $R^2$ für Wasserstoff oder Niederalkyl steht,

$R^3$ Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl oder Aryl oder $R^2$ und $R^3$ zusammen unsubstituiertes oder durch freies oder funktionell abgewandeltes Hydroxy substituiertes Trimethylen,

$R^4$ Wasserstoff oder Niederalkyl,

W einen jeweils mit dem Carbonyl-C-Atom an den Rest der Formel II

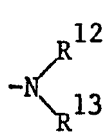

(II)

gebundenen Rest der Formeln III, IV, V oder VI,

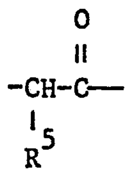

(III)

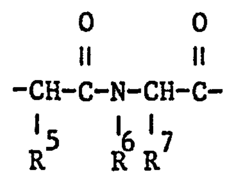

(IV)

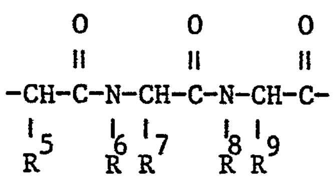

(V)

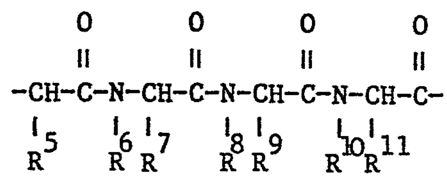

(VI)

worin $R^6$, $R^8$ und $R^{10}$ unabhängig voneinander für Wasserstoff oder Niederalkyl, $R^5$, $R^7$, $R^9$ und $R^{11}$ unabhängig voneinander für Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl, oder Aryl oder $R^4$ und $R^5$ zusammen, $R^6$ und $R^7$ zusammen, $R^8$ und $R^9$ zusammen und/oder $R^{10}$ und $R^{11}$ zusammen jeweils unabhängig voneinander für unsubstituiertes oder durch freies oder funktionell abgewandeltes Hydroxy substituiertes Trimethylen stehen, und
$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, und Salze von solchen Verbindungen mit mindestens einer salzbildenden Gruppe, mit Ausnahme von solchen Verbindungen der Formel I, worin

Z unsubstituiertes Alkylen mit 3 C-Atomen und zugleich
$R^1$ Wasserstoff, unsubstituiertes Alkyl mit 1-4 C-Atomen, Aryl oder Arylniederalkyl,

X den Rest NH,
$R^3$ Aryl, unsubstituiertes Alkyl oder Wasserstoff,
$R^4$ Wasserstoff,
W einen Rest der Formel III, worin $R^5$ eine mit dem Rest $R^3$ identische Bedeutung hat, und
$R^{12}$ und $R^{13}$ Wasserstoff
bedeuten, und von Salzen von diesen Verbindungen mit mindestens einer salzbildenden Gruppe.

2. Verbindungen der Formel I nach Anspruch 1, worin
Z einen Alkylenrest mit 3 bis 17 C-Atomen bedeutet, der unsubstituiert oder durch Hydroxy, Niederalkoxy, Niederalkanoyloxy und/oder einen unsubstituierten oder durch Halogen, Niederalkyl, Halogenphenoxy, Hydroxy oder Niederalkoxy substituierten Phenyl- oder Naphthylrest substituiert ist und der zusammen mit der Amidgruppierung einen fünfgliedrigen Ring bildet,
$R^1$ Wasserstoff, unsubstituiertes oder durch einen Phenylrest, Hydroxy, Niederalkoxy oder Niederalkanoyloxy substituiertes Niederalkyl oder Phenyl, X Sauerstoff oder die Gruppe $NR^2$, worin $R^2$ für Wasserstoff oder Niederalkyl steht,
$R^3$ Wasserstoff, unsubstituiertes oder durch Hydroxy, Niederalkanoyloxy, einen Phenylrest, Aminocarbonyl, Mercapto, Methylthio, Amino, Di- oder Mononiederalkylamino, Niederalkanoylamino, Guanidino, Carboxy, Niederalkoxycarbonyl oder Imidazol-4-yl substituiertes Niederalkyl oder einen Phenylrest, oder $R^2$ und $R^3$ zusammen unsubstituiertes oder durch Hydroxy, Niederalkoxy oder Niederalkanoyloxy substituiertes Trimethylen,
$R^4$ Wasserstoff oder Niederalkyl,
W einen jeweils mit dem Carbonyl-C-Atom an den Rest der Formel II gebundenen Rest der Formeln III, IV, V oder VI, worin
$R^6$, $R^8$ und $R^{10}$ unabhängig voneinander für Wasserstoff oder Niederalkyl,
$R^5$, $R^7$, $R^9$ und $R^{11}$ unabhängig voneinander für Wasserstoff, unsubstituiertes oder durch Hydroxy, Niederalkoxy, Niederalkanoyloxy, einen Phenylrest, Aminocarbonyl, Mercapto, Methylthio, Amino, Di- oder Mononiederalkylamino, Niederalkanoylamino, Guanidino, Carboxy, Niederalkoxycarbonyl oder Imidazol-4-yl substituiertes Niederalkyl oder einen Phenylrest, oder $R^4$ und $R^5$ zusammen, $R^6$ und $R^7$ zusammen, $R^8$ und $R^9$ zusammen oder $R^{10}$ und $R^{11}$ zusammen jeweils unabhängig voneinander für unsubstituiertes oder durch Hydroxy, Niederalkoxy oder Niederalkanoyloxy substituiertes Trimethylen stehen, und
$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, und Salze von solchen Verbindungen mit mindestens einer salzbildenden Gruppe.

3. Verbindungen der Formel I nach Anspruch 1, worin
Z einen Alkylenrest mit 3 bis 14 C-Atomen bedeutet, der unsubstituiert oder durch Hydroxy, Niederalkanoyloxy und/oder Naphthyl oder einen unsubstituierten oder durch Halogen oder Halogenphenoxy substituierten Phenylrest substituiert ist, und der zusammen mit der Amidgruppierung einen fünfgliedrigen Ring bildet,
$R^1$ Wasserstoff oder unsubstituiertes oder durch Hydroxy oder Niederalkanoyloxy substituiertes Niederalkyl,
X Sauerstoff oder die Gruppe $NR^2$, worin $R^2$ für Wasserstoff oder $C_{1-4}$-Alkyl steht,
$R^3$ Wasserstoff, unsubstituiertes oder durch Hydroxy, Niederalkanoyloxy, Phenyl, 4-Hydroxy-phenyl, Aminocarbonyl, Amino, Niederalkanoylamino, Carboxy, Niederalkoxycarbonyl oder Imidazol-4-yl substituiertes Niederalkyl oder Phenyl, oder
$R^2$ und $R^3$ zusammen unsubstituiertes oder durch Hydroxy substituiertes Trimethylen,
$R^4$ Wasserstoff oder $C_{1-4}$-Alkyl,
W einen jeweils mit dem Carbonyl-C-Atom an den Rest der Formel II gebundenen Rest der Formeln III, IV, V oder VI, worin
$R^6$, $R^8$ und $R^{10}$ unabhängig voneinander für Wasserstoff oder $C_{1-4}$-Alkyl,
$R^5$, $R^7$, $R^9$ und $R^{11}$ unabhängig voneinander für Wasserstoff, unsubstituiertes oder durch Hydroxy, Niederalkanoyloxy, Phenyl, 4-Hydroxyphenyl,

Aminocarbonyl, Amino, Niederalkanoylamino, Carboxy, Niederalkoxycarbonyl oder Imidazol-4-yl substituiertes Niederalkyl oder Phenyl, oder $R^4$ und $R^5$ zusammen, $R^6$ und $R^7$ zusammen, $R^8$ und $R^9$ zusammen und/oder $R^{10}$ und $R^{11}$ zusammen jeweils unabhängig voneinander für unsubstituiertes oder durch Hydroxy oder Niederalkanoyloxy substituiertes Trimethylen stehen, und

$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, und Salze von solchen Verbindungen mit mindestens einer salzbildenden Gruppe.

4. Verbindung der Formel I nach Anspruch 1, worin Z einen Rest der Formel VII bedeutet,

$$-\underset{R^{15}}{\overset{R^{14}}{\underset{|}{\overset{|}{C}}}}-\underset{H}{\overset{R^{16}}{\underset{|}{\overset{|}{C}}}}-\underset{H}{\overset{R^{17}}{\underset{|}{\overset{|}{C}}}}- \qquad \text{(VII)}$$

der mit dem $C(R^{14})$-Atom an das Stickstoffatom gebunden ist, und worin

$R^{14}$ und $R^{15}$ unabhängig voneinander Wasserstoff oder $C_{1-4}$-Alkyl,

$R^{16}$ Wasserstoff, Naphthyl, unsubstituiertes oder durch Fluor, Chlor, Fluorphenoxy oder Chlorphenoxy substituiertes Phenyl, Hydroxy oder Niederalkanoyloxy und

$R^{17}$ Wasserstoff, Hydroxy oder Niederalkanoyloxy bedeuten,

$R^1$ Wasserstoff, unsubstituiertes oder durch Hydroxy substituiertes $C_{1-4}$-Alkyl,

X Sauerstoff oder die Gruppe $NR^2$, worin $R^2$ für Wasserstoff oder geradkettiges $C_{1-3}$-Alkyl steht,

$R^3$ Wasserstoff oder unsubstituiertes oder durch Hydroxy, Phenyl, p-Hydroxy-phenyl, Aminocarbonyl oder Imidazol-4-yl substituiertes Niederalkyl,

$R^4$ Wasserstoff oder geradkettiges $C_{1-3}$-Alkyl,
W einen jeweils mit dem Carbonyl-C-Atom an den Rest der Formel II gebundenen Rest der Formeln III, IV, V oder VI,
worin
$R^6$, $R^8$ und $R^{10}$ unabhängig voneinander für Wasserstoff oder geradkettiges $C_{1-3}$-Alkyl,
$R^5$, $R^7$, $R^9$ und $R^{11}$ unabhängig voneinander für Wasserstoff oder unsubstituiertes oder durch Hydroxy, Phenyl, 4-Hydroxy-phenyl, Aminocarbonyl oder Imidazol-4-yl substituiertes Niederalkyl, oder, wenn W für Formel III steht, $R^4$ und $R^5$ zusammen, oder, wenn W für Formel IV steht, $R^6$ und $R^7$ zusammen, oder, wenn W für Formel V steht, $R^8$ und $R^9$ zusammen, oder, wenn W für Formel VI steht, $R^{10}$ und $R^{11}$ zusammen jeweils für einen unsubstituierten oder durch Hydroxy oder Niederalkanoyloxy substituierten Trimethylenrest stehen, und
$R^{12}$ und $R^{13}$ unabhängig voneinander Wasserstoff oder $C_{1-4}$-Alkyl oder $R^{12}$ und $R^{13}$ zusammen 3-Oxa-1,5-pentylen oder 3-Aza-3-methyl-1,5-pentylen bedeuten, und Salze von solchen Verbindungen mit mindestens einer salzbildenden Gruppe.

5. Verbindungen der Formel I nach Anspruch 1, worin
Z einen mit dem $C(R^{14})$-Atom an das Stickstoffatom gebundenen Rest der Formel VII, worin $R^{14}$ und $R^{15}$ unabhängig voneinander Wasserstoff oder $C_{1-4}$-Alkyl, $R^{16}$ Wasserstoff, Naphthyl, 4-Fluor-phenyl, 4-Chlorphenoxyphenyl oder Hydroxy und $R^{17}$ Wasserstoff bedeuten,
$R^1$ Wasserstoff,
X Sauerstoff oder die Gruppe NH,
$R^3$ Wasserstoff oder $C_1$-$C_4$-Alkyl,
$R^4$ Wasserstoff,
W einen Rest der Formeln III, IV, V oder VI, worin
$R^5$, $R^7$, $R^9$ und $R^{11}$ unabhängig voneinander für Wasserstoff oder $C_{1-4}$-Alkyl,

$R^6$, $R^8$ und $R^{10}$ für Wasserstoff oder $R_{10}$ und $R_{11}$ zusammen für Trimethylen stehen, $R^{12}$ Wasserstoff und $R^{13}$ $C_{1-4}$-Alkyl bedeuten.

6. Verbindungen der Formel I nach einem der Ansprüche 1-5, worin X die Gruppe NH bedeutet, und Salze von solchen Verbindungen mit mindestens einer salzbildenden Gruppe.

7. Verbindungen der Formel I nach einem der Ansprüche 1-6, worin $R^5$ Wasserstoff oder $C_{1-4}$-Alkyl bedeutet, und Salze von solchen Verbindungen mit mindestens einer salzbildenden Gruppe.

8. Verbindungen der Formel I nach einem der Ansprüche 1-7, worin, wenn W für einen Rest der Formel IV steht, $R^7$, wenn W für einen Rest der Formel V steht, $R^7$ und $R^9$, und, wenn W für einen Rest der Formel VI steht, $R^7$, $R^9$ und $R^{11}$ unabhängig voneinander Wasserstoff oder $C_{1-4}$-Alkyl bedeuten, und Salze von solchen Verbindungen mit mindestens einer salzbildenden Gruppe.

9. Verbindungen der Formel I nach einem der Ansprüche 1-8, worin $R^2$, $R^4$ und, wenn W für einen Rest der Formel IV steht, $R^6$, und, wenn W für einen Rest der Formel V steht, auch $R^8$ und, wenn W für einen Rest der Formel VI steht, auch $R^{10}$ Wasserstoff bedeuten, und Salze von solchen Verbindungen mit mindestens einer salzbildenden Gruppe.

10. Verbindungen der Formel I nach einem der Ansprüche 1-7 und 9, worin W einen Rest der Formel III bedeutet, und Salze von solchen Verbindungen mit mindestens einer salzbildenden Gruppe.

11. Verbindungen der Formel I nach einem der Ansprüche 1-9, worin W einen Rest der Formel IV bedeutet, und Salze von solchen Verbindungen mit mindestens einer salzbildenden Gruppe.

12. Verbindungen der Formel I nach einem der Ansprüche 1-9, worin W einen Rest der Formeln V oder VI bedeutet, und Salze von solchen Verbindungen mit mindestens einer salzbildenden Gruppe.

13. Verbindungen der Formel I nach einem der Ansprüche 1-12, die an einem asymmetrisch substituierten C-$R^3$-Atom und an allen Asymmetriezentren im Rest W die (L)-Konfiguration, am C-$R^5$-Atom alternativ auch die (D)-Konfiguration aufweisen, und Salze von solchen Verbindungen mit mindestens einer salzbildenden Gruppe.

14. Verbindungen der Formel I nach Anspruch 1, worin Z Trimethylen oder 2-Hydroxy-trimethylen, $R^1$, $R^3$, $R^4$ und $R^{12}$ Wasserstoff, X die Gruppe NH, W einen Rest der Formeln III oder IV, worin $R^5$ für Wasserstoff oder Methyl und $R^6$ und $R^7$ für Wasserstoff stehen, und $R^{13}$ Wasserstoff oder $C_{1-3}$-Alkyl bedeuten, wobei die Konfiguration am C-$R^5$, wenn $R^5$ für Methyl steht, (D) oder (L) sein kann.

15. Verbindungen der Formel I nach einem der Ansprüche 1-14, worin Z Trimethylen bedeutet, und Salze von solchen Verbindungen mit mindestens einer salzbildenden Gruppe.

16. Verbindungen der Formel I nach Anspruch 1, worin Z Trimethylen, $R^1$ Wasserstoff, X die Gruppe $NR^2$, $R^2$ und $R^3$ Wasserstoff oder $R^2$ und $R^3$ zusammen Trimethylen, $R^4$ Wasserstoff, W einen Rest der Formeln III, IV, V oder VI, worin $R^5$ für Wasserstoff oder $C_{1-3}$-Alkyl oder $R^4$ und $R^5$ zusammen für Trimethylen, $R^6$, $R^7$, $R^8$, $R^9$, $R^{10}$ und $R^{11}$ für Wasserstoff oder $R^6$ und $R^7$ zusammen für Trimethylen stehen, $R^{12}$ Wasserstoff oder $C_{1-3}$-Alkyl und $R^{13}$ Wasserstoff bedeuten.

17. N-[(2-Oxo-1-pyrrolidinyl)-acetyl]-glycyl-(L)-alaninamid nach Anspruch 1.

18. N-[(2-Oxo-1-pyrrolidinyl)-acetyl]-L-prolyl-glycyl-glycinamid nach Anspruch 1.

19. N-[(2-Oxo-1-pyrrolidinyl)-acetyl]-glycyl-(L)-alanin-N-monoethylamid nach Anspruch 1.

20. Pharmazeutische Präparate, die eine Verbindung gemäss einem der Ansprüche 1-19 zusammen mit einem pharmazeutisch verwendbaren Trägermaterial enthalten.

21. Eine Verbindung gemäss einem der Ansprüche 1-19 zur Verwendung in einem Verfahren zur therapeutischen Behandlung des menschlichen oder tierischen Körpers.

22. Verwendung einer Verbindung gemäss einem der Ansprüche 1-19 zur Herstellung von pharmazeutischen Präparaten.

23. Verwendung einer Verbindung gemäss einem der Ansprüche 1-19 zur Behandlung von zerebraler Leistungsinsuffizienz.

24. Verwendung einer Verbindung gemäss einem der Ansprüche 1-19 zur Therapie von Tumorerkrankungen.

25. Verfahren zur Herstellung von Lactampeptiden der Formel I

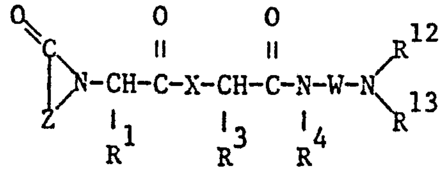

(I)

worin Z einen Alkylenrest bedeutet, der unsubstituiert oder durch freies oder funktionell abgewandeltes Hydroxy und/oder durch Aryl substituiert ist und der zusammen mit der Amidgruppierung einen fünfgliedrigen Ring bildet,

$R^1$ Wasserstoff, unsubstituiertes oder durch Aryl oder durch freies

oder funktionell abgewandeltes Hydroxy substituiertes Niederalkyl oder Aryl,

X Sauerstoff oder die Gruppe $NR^2$, worin $R^2$ für Wasserstoff oder Niederalkyl steht,

$R^3$ Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl oder Aryl oder $R^2$ und $R^3$ zusammen unsubstituiertes oder durch freies oder funktionell abgewandeltes Hydroxy substituiertes Trimethylen,

$R^4$ Wasserstoff oder Niederalkyl,

W einen jeweils mit dem Carbonyl-C-Atom an den Rest der Formel II

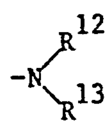

(II)

gebundenen Rest der Formeln III, IV, V oder VI,

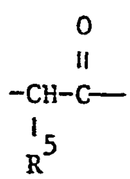

(III)

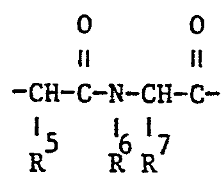

(IV)

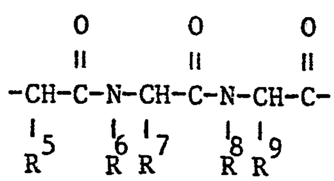

(V)

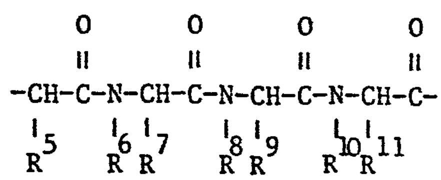

(VI)

worin $R^6$, $R^8$ und $R^{10}$ unabhängig voneinander für Wasserstoff oder Niederalkyl, $R^5$, $R^7$, $R^9$ und $R^{11}$ unabhängig voneinander für Wasserstoff, unsubstituiertes oder durch freies oder funktionell abgewandeltes Hydroxy, Aryl, Aminocarbonyl, Mercapto, Methylthio,

freies, alkyliertes oder acyliertes Amino, Guanidino, freies, verestertes oder amidiertes Carboxy oder Imidazol-4-yl substituiertes Niederalkyl, oder Aryl oder $R^4$ und $R^5$ zusammen, $R^6$ und $R^7$ zusammen, $R^8$ und $R^9$ zusammen und/oder $R^{10}$ und $R^{11}$ zusammen jeweils unabhängig voneinander für unsubstituiertes oder durch freies oder funktionell abgewandeltes Hydroxy substituiertes Trimethylen stehen, und
$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, und von Salzen von solchen Verbindungen, die mindestens eine salzbildende Gruppe enthalten, mit Ausnahme von solchen Verbindungen der Formel I, worin
Z unsubstituiertes Alkylen mit 3 C-Atomen und zugleich
$R^1$ Wasserstoff, unsubstituiertes Alkyl mit 1-4 C-Atomen, Aryl oder Arylniederalkyl,
X den Rest NH,
$R^3$ Aryl, unsubstituiertes Alkyl oder Wasserstoff,
$R^4$ Wasserstoff,
W einen Rest der Formel III, worin $R^5$ eine mit dem Rest $R^3$ identische Bedeutung hat, und
$R^{12}$ und $R^{13}$ Wasserstoff
bedeuten, und von Salzen von diesen Verbindungen mit mindestens einer salzbildenden Gruppe, dadurch gekennzeichnet, dass man

a) eine Verbindung der Formel VIII,

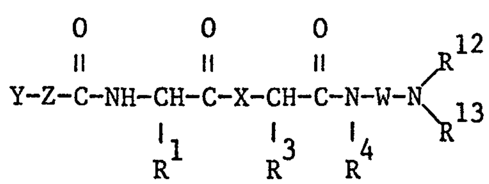

(VIII)

worin Y eine nucleophile Abgangsgruppe bedeutet und die übrigen Substituenten die obengenannten Bedeutungen haben, mit der Massgabe, dass in den Resten Z, $R^1$, $R^3$ oder W gegebenenfalls vorhandene funktionelle Gruppen gegebenenfalls durch leicht abspaltbare Schutzgruppen geschützt sind, intramolekular unter Ausbildung des Pyrrolidonringes zyklisiert, oder

b) eine Verbindung der Formel IX,

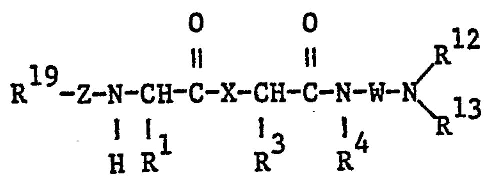 (IX)

worin $R^{19}$ eine Carboxylgruppe oder ein aktiviertes Derivat derselben bedeutet und auch die an der Reaktion teilnehmende Aminogruppe in aktivierter Form vorliegen kann und die übrigen Substituenten die obengenannten Bedeutungen haben, mit der Massgabe, dass in den Resten Z, $R^1$, $R^3$ oder W gegebenenfalls vorhandene funktionelle Gruppen gegebenenfalls durch leicht abspaltbare Schutzgruppen geschützt sind, intramolekular unter Ausbildung des Pyrrolidonringes zyklisiert oder

c) eine Verbindung der Formel X,

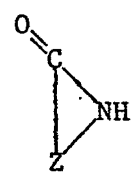 (X)

worin Z die obengenannte Bedeutung hat, mit der Massgabe, dass darin vorhandene funktionelle Gruppen gegebenenfalls durch leicht abspaltbare Schutzgruppen geschützt sind, oder eine reaktionsfähige Form dieser Verbindung mit einer Verbindung der Formel XI,

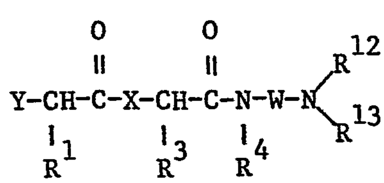 (XI)

worin Y eine nucleophile Abgangsgruppe bedeutet und die übrigen Substituenten die obengenannten Bedeutungen haben, mit der Massgabe,

dass in den Resten Z, $R^1$, $R^3$ oder W vorhandene funktionelle Gruppen gegebenenfalls durch leicht abspaltbare Schutzgruppen geschützt sind, umsetzt, oder

d) eine Verbindung der Formel XII,

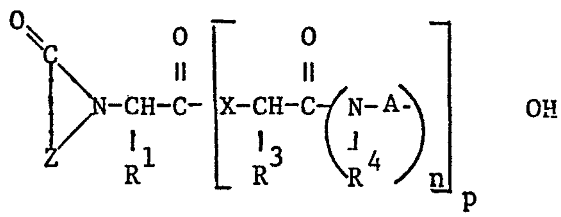

(XII)

worin n und p unabhängig voneinander die Zahl 0 oder 1 und A den Rest W oder $W_a$ bedeuten, wobei $W_a$ einen Rest der Formeln III, IV oder V bedeutet und die übrigen Substituenten die obengenannten Bedeutungen haben, mit der Massgabe, dass in den Resten Z, $R^1$, $R^3$ und A gegebenenfalls vorhandene funktionelle Gruppen gegebenenfalls durch leicht abspaltbare Schutzgruppen geschützt sind, oder ein reaktionsfähiges Carbonsäurederivat der Verbindung der Formel XII mit einer Verbindung der Formel XIII

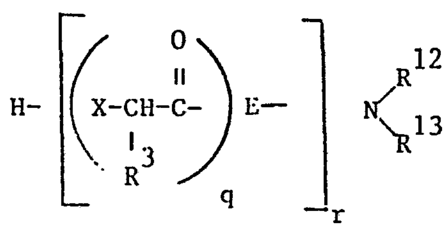

(XIII)

worin E für den Rest der Formel $-\underset{R^4}{N}-W-$ oder für den Rest $W_b$, der so definiert ist, dass bei Verknüpfung von $W_a$ und $W_b$ der Rest W resultiert, und q und r unabhängig voneinander für die Zahl 0 oder 1 stehen, mit der Massgabe, dass r und q jeweils für 1 und E für den Rest $-\underset{R^4}{N}-W-$ stehen, wenn im Reaktionspartner der Formel XII p für 0

steht, oder dass r für 1, q für 0 und E für den Rest $-\underset{R^4}{N}-W-$ stehen, wenn p für 1 und n für 0 stehen, oder dass r für 1, q für 0 und E für $W_b$ stehen, wenn p für 1, n für 1 und A für $W_a$ stehen, oder dass r für 0 steht, wenn p und n jeweils für 1 und A für W stehen, und worin die übrigen Substituenten die obengenannten Bedeutungen haben, mit der Massgabe, dass in den Resten $R^3$ und E gegebenenfalls vorhandene funktionelle Gruppen gegebenenfalls durch leicht abspaltbare Schutzgruppen geschützt sind, oder einem Derivat der Verbindung der Formel XIII, in dem die Gruppe H-X-, H-E- oder $H-\underset{R^{12}}{N}-$ in reaktionsfähiger Form vorliegt, umsetzt, oder

e) eine Verbindung der Formel I, worin mindestens einer der Reste $R^2$, $R^4$, $R^6$, $R^8$, $R^{10}$, $R^{12}$ und $R^{13}$ für Wasserstoff steht und die übrigen Substituenten die obengenannten Bedeutungen haben, mit der Massgabe, dass in einer Verbindung der Formel I vorhandene funktionelle Gruppen mit Ausnahme der reagierenden Amidgruppe(n) gegebenenfalls durch leicht abspaltbare Schutzgruppen geschützt sind, oder ein reaktionsfähiges Derivat dieser Verbindung mit einem die obengenannten Reste $R^2$, $R^4$, $R^6$, $R^8$, $R^{10}$, $R^{12}$ und/oder $R^{13}$ einführenden Alkylierungsmittel umsetzt, oder

f) zur Herstellung einer Verbindung der Formel I, worin Z 1- oder 2-Hydroxy-1,3-alkylen bedeutet, in einer Verbindung der Formel I, worin Z für einen mit dem $C(R^{14})$-Atom an das Stickstoffatom gebundenen Rest der Formel XV oder XVI steht,

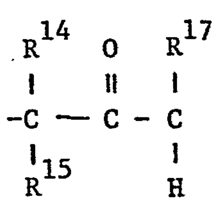

(XV)

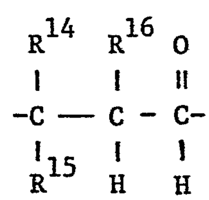

(XVI)

wobei alle Substituenten die obengenannten Bedeutungen haben, mit der Massgabe, dass in den Resten $R^1$, $R^3$, W, $R^{16}$ und $R^{17}$ gegebenenfalls

vorhandene funktionelle Gruppen gegebenenfalls durch leicht abspaltbare Schutzgruppen geschützt sind, die Ketogruppe in den Formeln XV oder XVI, mit einem regioselektiven Reduktionsmittel in eine Hydroxygruppe überführt, oder

g) die Cyanogruppe in einer Verbindung der Formel XVII,

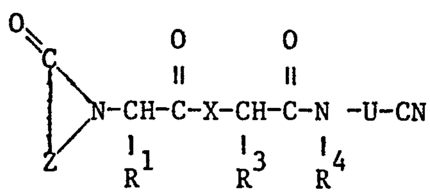

(XVII)

worin U einen Rest der Formel XVIII, XIX, XX oder XXI bedeutet

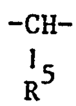

(XVIII)

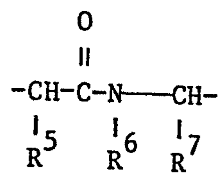

(XIX)

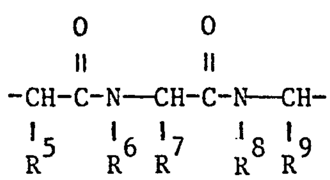

(XX)

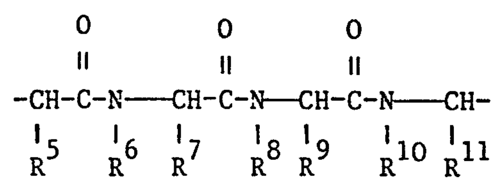

(XXI)

und worin alle Substituenten die obengenannten Bedeutungen haben, in eine Amidgruppe überführt oder

h) zur Herstellung einer Verbindung der Formel I, worin $R^{12}$ für Wasserstoff steht, die Oximgruppe in einer Verbindung der Formel XXII,

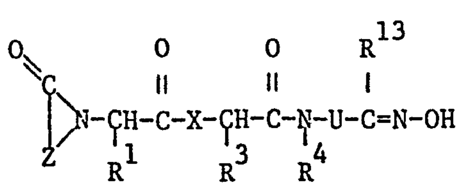

(XXII)

worin die Substituenten die obengenannten Bedeutungen haben, durch eine Beckmann-Umlagerung in eine Amidgruppe überführt, oder

i) in einer Verbindung der Formel I, die mindestens eine freie Hydroxy-, Carboxy- oder Aminogruppe aufweist, freies Hydroxy verestert oder verethert, freies Carboxy verestert oder amidiert oder freies Amino alkyliert oder acyliert und nach Ausführung der unter a) bis h) beschriebenen Verfahren gegebenenfalls vorhandene Schutzgruppen abspaltet und, wenn erwünscht, erhaltene Stereoisomerengemische auftrennt, und, wenn erwünscht, eine erhaltene Verbindung mit mindestens einer salzbildenden Gruppe in ein Salz oder ein erhaltenes Salz in die freie Verbindung überführt.

26. Die nach den Verfahren des Anspruchs 25 erhältlichen Verbindungen.

Patentansprüche für den Vertragsstaat AT:

1. Verfahren zur Herstellung von Lactampeptiden der Formel I

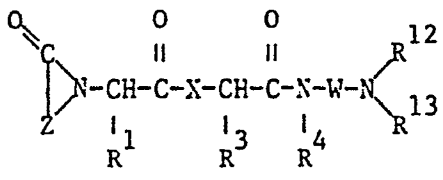

(I)

worin Z einen Alkylenrest bedeutet, der unsubstituiert oder durch freies oder funktionell abgewandeltes Hydroxy und/oder durch Aryl substituiert ist und der zusammen mit der Amidgruppierung einen fünfgliedrigen Ring bildet,

$R^1$ Wasserstoff, unsubstituiertes oder durch Aryl oder durch freies oder funktionell abgewandeltes Hydroxy substituiertes Niederalkyl oder Aryl,

X Sauerstoff oder die Gruppe $NR^2$, worin $R^2$ für Wasserstoff oder Niederalkyl steht,

$R^3$ Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl oder Aryl oder $R^2$ und $R^3$ zusammen unsubstituiertes oder durch freies oder funktionell abgewandeltes Hydroxy substituiertes Trimethylen,

$R^4$ Wasserstoff oder Niederalkyl,

W einen jeweils mit dem Carbonyl-C-Atom an den Rest der Formel II

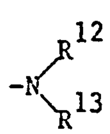

(II)

gebundenen Rest der Formeln III, IV, V oder VI,

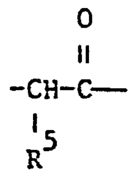

(III)

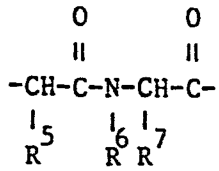

(IV)

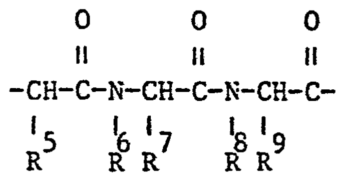

(V)

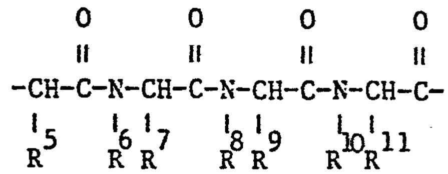

(VI)

worin $R^6$, $R^8$ und $R^{10}$ unabhängig voneinander für Wasserstoff oder Niederalkyl, $R^5$, $R^7$, $R^9$ und $R^{11}$ unabhängig voneinander für Wasserstoff, unsubstituiertes oder durch freies oder funktionell abgewandeltes Hydroxy, Aryl, Aminocarbonyl, Mercapto, Methylthio, freies, alkyliertes oder acyliertes Amino, Guanidino, freies, verestertes oder amidiertes Carboxy oder Imidazol-4-yl substituiertes Niederalkyl, oder Aryl oder $R^4$ und $R^5$ zusammen, $R^6$ und $R^7$ zusammen, $R^8$ und $R^9$ zusammen und/oder $R^{10}$ und $R^{11}$ zusammen jeweils unabhängig voneinander für unsubstituiertes oder durch freies oder funktionell abgewandeltes Hydroxy substituiertes Trimethylen stehen, und
$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, und von Salzen von solchen Verbindungen, die mindestens eine salzbildende Gruppe enthalten, mit Ausnahme von solchen Verbindungen der Formel I, worin

Z unsubstituiertes Alkylen mit 3 C-Atomen und zugleich

$R^1$ Wasserstoff, unsubstituiertes Alkyl mit 1-4 C-Atomen, Aryl oder Arylniederalkyl,

X den Rest NH,

$R^3$ Aryl, unsubstituiertes Alkyl oder Wasserstoff,

$R^4$ Wasserstoff,

W einen Rest der Formel III, worin $R^5$ eine mit dem Rest $R^3$ identische Bedeutung hat, und

$R^{12}$ und $R^{13}$ Wasserstoff
bedeuten, und von Salzen von diesen Verbindungen mit mindestens einer salzbildenden Gruppe, dadurch gekennzeichnet, dass man

a) eine Verbindung der Formel VIII,

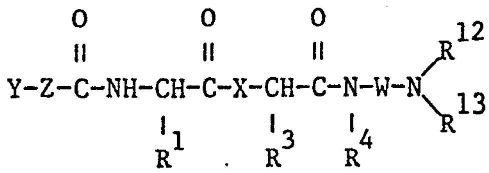

(VIII)

worin Y eine nucleophile Abgangsgruppe bedeutet und die übrigen Substituenten die obengenannten Bedeutungen haben, mit der Massgabe, dass in den Resten Z, $R^1$, $R^3$ oder W gegebenenfalls vorhandene funktionelle Gruppen gegebenenfalls durch leicht abspaltbare Schutzgruppen geschützt sind, intramolekular unter Ausbildung des Pyrrolidonringes zyklisiert, oder

b) eine Verbindung der Formel IX,

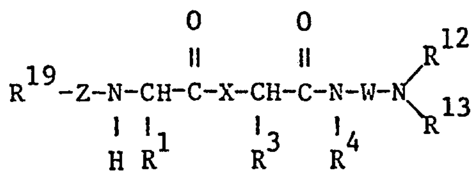

(IX)

worin $R^{19}$ eine Carboxylgruppe oder ein aktiviertes Derivat derselben bedeutet und auch die an der Reaktion teilnehmende Aminogruppe in aktivierter Form vorliegen kann und die übrigen Substituenten die obengenannten Bedeutungen haben, mit der Massgabe, dass in den Resten Z, $R^1$, $R^3$ oder W gegebenenfalls vorhandene funktionelle Gruppen gegebenenfalls durch leicht abspaltbare Schutzgruppen geschützt sind, intramolekular unter Ausbildung des Pyrrolidonringes zyklisiert oder

c) eine Verbindung der Formel X,

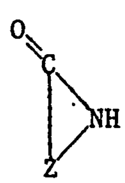

(X)

worin Z die obengenannte Bedeutung hat, mit der Massgabe, dass darin vorhandene funktionelle Gruppen gegebenenfalls durch leicht abspaltbare Schutzgruppen geschützt sind, oder eine reaktionsfähige Form dieser Verbindung mit einer Verbindung der Formel XI,

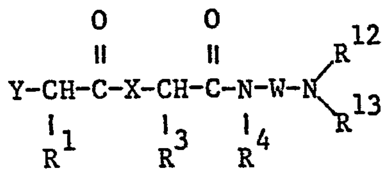

(XI)

worin Y eine nucleophile Abgangsgruppe bedeutet und die übrigen Substituenten die obengenannten Bedeutungen haben, mit der Massgabe, dass in den Resten Z, $R^1$, $R^3$ oder W vorhandene funktionelle Gruppen gegebenenfalls durch leicht abspaltbare Schutzgruppen geschützt sind, umsetzt, oder

d) eine Verbindung der Formel XII,

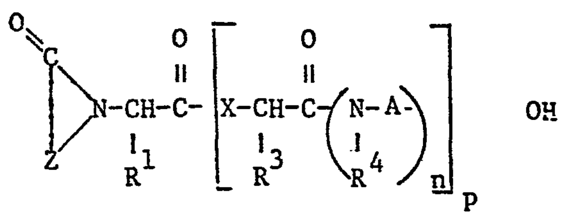

(XII)

worin n und p unabhängig voneinander die Zahl 0 oder 1 und A den Rest W oder $W_a$ bedeuten, wobei $W_a$ einen Rest der Formeln III, IV oder V bedeutet und die übrigen Substituenten die obengenannten Bedeutungen haben, mit der Massgabe, dass in den Resten Z, $R^1$, $R^3$ und A gegebenenfalls vorhandene funktionelle Gruppen gegebenenfalls durch leicht abspaltbare Schutzgruppen geschützt sind, oder ein reaktionsfähiges Carbonsäurederivat der Verbindung der Formel XII mit einer Verbindung der Formel XIII

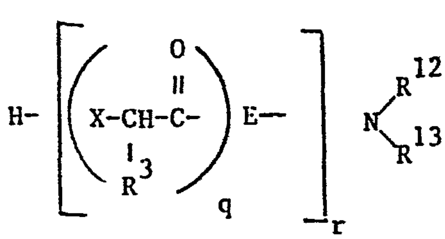

(XIII)

worin E für den Rest der Formel $-\underset{R^4}{N}-W-$ oder für den Rest $W_b$, der so definiert ist, dass bei Verknüpfung von $W_a$ und $W_b$ der Rest W resultiert, und q und r unabhängig voneinander für die Zahl 0 oder 1 stehen, mit der Massgabe, dass r und q jeweils für 1 und E für den Rest $-\underset{R^4}{N}-W-$ stehen, wenn im Reaktionspartner der Formel XII p für 0 steht, oder dass r für 1, q für 0 und E für den Rest $-\underset{R^4}{N}-W-$ stehen, wenn p für 1 und n für 0 stehen, oder dass r für 1, q für 0 und E für $W_b$ stehen, wenn p für 1, n für 1 und A für $W_a$ stehen, oder dass r für 0 steht, wenn p und n jeweils für 1 und A für W stehen, und worin die übrigen Substituenten die obengenannten Bedeutungen haben, mit der Massgabe, dass in den Resten $R^3$ und E gegebenenfalls vorhandene funktionelle Gruppen gegebenenfalls durch leicht abspaltbare Schutzgruppen geschützt sind, oder einem Derivat der Verbindung der Formel XIII, in dem die Gruppe H-X-, H-E- oder $H-\underset{R^{12}}{N}-$ in reaktionsfähiger Form vorliegt, umsetzt, oder

e) eine Verbindung der Formel I, worin mindestens einer der Reste $R^2$, $R^4$, $R^6$, $R^8$, $R^{10}$, $R^{12}$ und $R^{13}$ für Wasserstoff steht und die übrigen Substituenten die obengenannten Bedeutungen haben, mit der Massgabe, dass in einer Verbindung der Formel I vorhandene funktionelle Gruppen mit Ausnahme der reagierenden Amidgruppe(n) gegebenenfalls durch leicht abspaltbare Schutzgruppen geschützt sind, oder ein reaktionsfähiges Derivat dieser Verbindung mit einem die obengenannten Reste $R^2$, $R^4$, $R^6$, $R^8$, $R^{10}$, $R^{12}$ und/oder $R^{13}$ einführenden Alkylierungsmittel umsetzt, oder

f) zur Herstellung einer Verbindung der Formel I, worin Z 1- oder 2-Hydroxy-1,3-alkylen bedeutet, in einer Verbindung der Formel I, worin Z für einen mit dem $C(R^{14})$-Atom an das Stickstoffatom gebundenen Rest der Formel XV oder XVI steht,

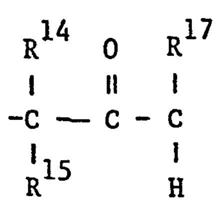

(XV)

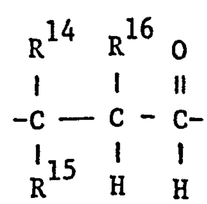

(XVI)

wobei alle Substituenten die obengenannten Bedeutungen haben, mit der Massgabe, dass in den Resten $R^1$, $R^3$, W, $R^{16}$ und $R^{17}$ gegebenenfalls vorhandene funktionelle Gruppen gegebenenfalls durch leicht abspaltbare Schutzgruppen geschützt sind, die Ketogruppe in den Formeln XV oder XVI, mit einem regioselektiven Reduktionsmittel in eine Hydroxygruppe überführt, oder

g) die Cyanogruppe in einer Verbindung der Formel XVII,

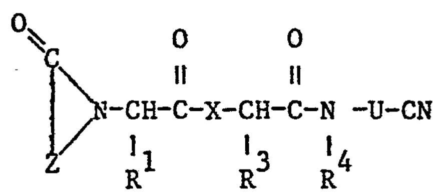

(XVII)

worin U einen Rest der Formel XVIII, XIX, XX oder XXI bedeutet

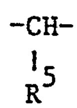

(XVIII)

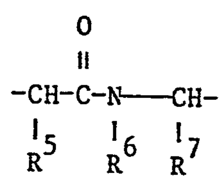

(XIX)

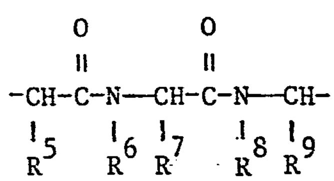

(XX)

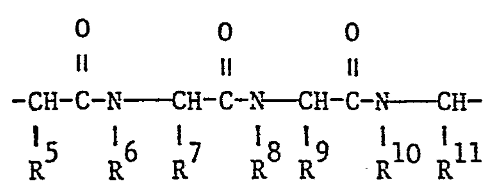

(XXI)

und worin alle Substituenten die obengenannten Bedeutungen haben, in eine Amidgruppe überführt oder

h) zur Herstellung einer Verbindung der Formel I, worin $R^{12}$ für Wasserstoff steht, die Oximgruppe in einer Verbindung der Formel XXII,

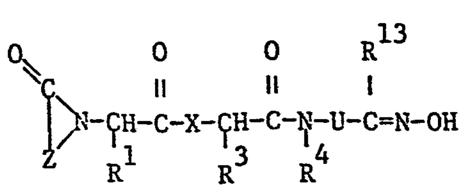

(XXII)

worin die Substituenten die obengenannten Bedeutungen haben, durch eine Beckmann-Umlagerung in eine Amidgruppe überführt, oder

i) in einer Verbindung der Formel I, die mindestens eine freie Hydroxy-, Carboxy- oder Aminogruppe aufweist, freies Hydroxy verestert oder verethert, freies Carboxy verestert oder amidiert oder freies Amino alkyliert oder acyliert und nach Ausführung der unter a) bis h) beschriebenen Verfahren gegebenenfalls vorhandene Schutzgruppen abspaltet und, wenn erwünscht, erhaltene Stereoisomerengemische auftrennt, und, wenn erwünscht, eine erhaltene Verbindung mit mindestens einer salzbildenden Gruppe in ein Salz oder ein erhaltenes Salz in die freie Verbindung überführt.

2. Verfahren nach Anspruch 1, dadurch gekennzeichnet, dass man die Ausgangsstoffe so wählt, dass man eine Verbindung der Formel I, worin Z einen Alkylenrest mit 3 bis 17 C-Atomen bedeutet, der unsubstituiert oder durch Hydroxy, Niederalkoxy, Niederalkanoyloxy und/oder einen unsubstituierten oder durch Halogen, Niederalkyl, Halogenphenoxy, Hydroxy oder Niederalkoxy substituierten Phenyl- oder Naphthylrest substituiert ist und der zusammen mit der Amidgruppierung einen fünfgliedrigen Ring bildet,
$R^1$ Wasserstoff, unsubstituiertes oder durch einen Phenylrest, Hydroxy, Niederalkoxy oder Niederalkanoyloxy substituiertes Niederalkyl oder Phenyl, X Sauerstoff oder die Gruppe $NR^2$, worin $R^2$ für Wasserstoff oder Niederalkyl steht,
$R^3$ Wasserstoff, unsubstituiertes oder durch Hydroxy, Niederalkanoyloxy, einen Phenylrest, Aminocarbonyl, Mercapto, Methylthio, Amino, Di- oder Mononiederalkylamino, Niederalkanoylamino, Guanidino, Carboxy, Niederalkoxycarbonyl oder Imidazol-4-yl substituiertes Niederalkyl oder einen Phenylrest, oder $R^2$ und $R^3$ zusammen unsubstituiertes oder durch Hydroxy, Niederalkoxy oder Niederalkanoyloxy substituiertes Trimethylen,
$R^4$ Wasserstoff oder Niederalkyl,
W einen jeweils mit dem Carbonyl-C-Atom an den Rest der Formel II gebundenen Rest der Formeln III, IV, V oder VI, worin
$R^6$, $R^8$ und $R^{10}$ unabhängig voneinander für Wasserstoff oder Niederalkyl,

$R^5$, $R^7$, $R^9$ und $R^{11}$ unabhängig voneinander für Wasserstoff, unsubstituiertes oder durch Hydroxy, Niederalkoxy, Niederalkanoyloxy, einen Phenylrest, Aminocarbonyl, Mercapto, Methylthio, Amino, Di- oder Mononiederalkylamino, Niederalkanoylamino, Guanidino, Carboxy, Niederalkoxycarbonyl oder Imidazol-4-yl substituiertes Niederalkyl oder einen Phenylrest, oder $R^4$ und $R^5$ zusammen, $R^6$ und $R^7$ zusammen, $R^8$ und $R^9$ zusammen oder $R^{10}$ und $R^{11}$ zusammen jeweils unabhängig voneinander für unsubstituiertes oder durch Hydroxy, Niederalkoxy oder Niederalkanoyloxy substituiertes Trimethylen stehen, und
$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, oder ein Salz einer solchen Verbindung mit mindestens einer salzbildenden Gruppe erhält.

3. Verfahren nach Anspruch 1, dadurch gekennzeichnet, dass man die Ausgangsstoffe so wählt, dass man eine Verbindung der Formel I, worin
Z einen Alkylenrest mit 3 bis 14 C-Atomen bedeutet, der unsubstituiert oder durch Hydroxy, Niederalkanoyloxy und/oder Naphthyl oder einen unsubstituierten oder durch Halogen oder Halogenphenoxy substituierten Phenylrest substituiert ist, und der zusammen mit der Amidgruppierung einen fünfgliedrigen Ring bildet,
$R^1$ Wasserstoff oder unsubstituiertes oder durch Hydroxy oder Niederalkanoyloxy substituiertes Niederalkyl,
X Sauerstoff oder die Gruppe $NR^2$, worin $R^2$ für Wasserstoff oder $C_{1-4}$-Alkyl steht,
$R^3$ Wasserstoff, unsubstituiertes oder durch Hydroxy, Niederalkanoyloxy, Phenyl, 4-Hydroxy-phenyl, Aminocarbonyl, Amino, Niederalkanoylamino, Carboxy, Niederalkoxycarbonyl oder Imidazol-4-yl substituiertes Niederalkyl oder Phenyl, oder
$R^2$ und $R^3$ zusammen unsubstituiertes oder durch Hydroxy substituiertes Trimethylen,
$R^4$ Wasserstoff oder $C_{1-4}$-Alkyl,
W einen jeweils mit dem Carbonyl-C-Atom an den Rest der Formel II gebundenen Rest der Formeln III, IV, V oder VI, worin

$R^6$, $R^8$ und $R^{10}$ unabhängig voneinander für Wasserstoff oder $C_{1-4}$-Alkyl, $R^5$, $R^7$, $R^9$ und $R^{11}$ unabhängig voneinander für Wasserstoff, unsubstituiertes oder durch Hydroxy, Niederalkanoyloxy, Phenyl, 4-Hydroxyphenyl, Aminocarbonyl, Amino, Niederalkanoylamino, Carboxy, Niederalkoxycarbonyl oder Imidazol-4-yl substituiertes Niederalkyl oder Phenyl, oder $R^4$ und $R^5$ zusammen, $R^6$ und $R^7$ zusammen, $R^8$ und $R^9$ zusammen und/oder $R^{10}$ und $R^{11}$ zusammen jeweils unabhängig voneinander für unsubstituiertes oder durch Hydroxy oder Niederalkanoyloxy substituiertes Trimethylen stehen, und

$R^{12}$ und $R^{13}$ unabhängig voneinander Wasserstoff oder Niederalkyl oder $R^{12}$ und $R^{13}$ zusammen Tetra- oder Pentamethylen, 3-Oxa-1,5-pentylen oder unsubstituiertes oder durch Oxo oder Methyl substituiertes 3-Aza-3-methyl-1,5-pentylen bedeuten, oder ein Salz einer solchen Verbindung mit mindestens einer salzbildenden Gruppe erhält.

4. Verfahren nach Anspruch 1, dadurch gekennzeichnet, dass man die Ausgangsstoffe so wählt, dass man eine Verbindung der Formel I, worin Z einen Rest der Formel VII bedeutet,

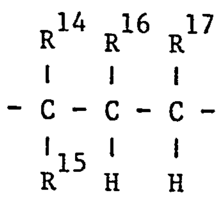

(VII)

der mit dem $C(R^{14})$-Atom an das Stickstoffatom gebunden ist, und worin

$R^{14}$ und $R^{15}$ unabhängig voneinander Wasserstoff oder $C_{1-4}$-Alkyl,
$R^{16}$ Wasserstoff, Naphthyl, unsubstituiertes oder durch Fluor, Chlor, Fluorphenoxy oder Chlorphenoxy substituiertes Phenyl, Hydroxy oder Niederalkanoyloxy und
$R^{17}$ Wasserstoff, Hydroxy oder Niederalkanoyloxy bedeuten,
$R^1$ Wasserstoff, unsubstituiertes oder durch Hydroxy substituiertes $C_{1-4}$-Alkyl,
X Sauerstoff oder die Gruppe $NR^2$, worin $R^2$ für Wasserstoff oder geradkettiges $C_{1-3}$-Alkyl steht,

$R^3$ Wasserstoff oder unsubstituiertes oder durch Hydroxy, Phenyl, p-Hydroxy-phenyl, Aminocarbonyl oder Imidazol-4-yl substituiertes Niederalkyl,
$R^4$ Wasserstoff oder geradkettiges $C_{1-3}$-Alkyl,
W einen jeweils mit dem Carbonyl-C-Atom an den Rest der Formel II gebundenen Rest der Formeln III, IV, V oder VI,
worin
$R^6$, $R^8$ und $R^{10}$ unabhängig voneinander für Wasserstoff oder geradkettiges $C_{1-3}$-Alkyl,
$R^5$, $R^7$, $R^9$ und $R^{11}$ unabhängig voneinander für Wasserstoff oder unsubstituiertes oder durch Hydroxy, Phenyl, 4-Hydroxy-phenyl, Aminocarbonyl oder Imidazol-4-yl substituiertes Niederalkyl, oder, wenn W für Formel III steht, $R^4$ und $R^5$ zusammen, oder, wenn W für Formel IV steht, $R^6$ und $R^7$ zusammen, oder, wenn W für Formel V steht, $R^8$ und $R^9$ zusammen, oder, wenn W für Formel VI steht, $R^{10}$ und $R^{11}$ zusammen jeweils für einen unsubstituierten oder durch Hydroxy oder Niederalkanoyloxy substituierten Trimethylenrest stehen, und
$R^{12}$ und $R^{13}$ unabhängig voneinander Wasserstoff oder $C_{1-4}$-Alkyl oder $R^{12}$ und $R^{13}$ zusammen 3-Oxa-1,5-pentylen oder 3-Aza-3-methyl-1,5-pentylen bedeuten, oder ein Salz einer solchen Verbindung mit mindestens einer salzbildenden Gruppe erhält.

5. Verfahren nach Anspruch 1, dadurch gekennzeichnet, dass man die Ausgangsstoffe so wählt, dass man eine Verbindung der Formel I, worin
Z einen mit dem $C(R^{14})$-Atom an das Stickstoffatom gebundenen Rest der Formel VII, worin $R^{14}$ und $R^{15}$ unabhängig voneinander Wasserstoff oder $C_{1-4}$-Alkyl, $R^{16}$ Wasserstoff, Naphthyl, 4-Fluor-phenyl, 4-Chlorphenoxyphenyl oder Hydroxy und $R^{17}$ Wasserstoff bedeuten,
$R^1$ Wasserstoff,
X Sauerstoff oder die Gruppe NH,
$R^3$ Wasserstoff oder $C_1$-$C_4$-Alkyl,
$R^4$ Wasserstoff,
W einen Rest der Formeln III, IV, V oder VI, worin
$R^5$, $R^7$, $R^9$ und $R^{11}$ unabhängig voneinander für Wasserstoff oder $C_{1-4}$-Alkyl,

$R^6$, $R^8$ und $R^{10}$ für Wasserstoff oder $R_{10}$ und $R_{11}$ zusammen für Trimethylen stehen, $R^{12}$ Wasserstoff und $R^{13}$ $C_{1-4}$-Alkyl bedeuten, erhält.

6. Verfahren nach einem der Ansprüche 1-5, dadurch gekennzeichnet, dass man die Ausgangsstoffe so wählt, dass man eine Verbindung der Formel I, worin X die Gruppe NH bedeutet, oder ein Salz einer solchen Verbindung mit mindestens einer salzbildenden Gruppe erhält.

7. Verfahren nach einem der Ansprüche 1-6, dadurch gekennzeichnet, dass man die Ausgangsstoffe so wählt, dass man eine Verbindung der Formel I, worin $R^5$ Wasserstoff oder $C_{1-4}$-Alkyl bedeutet, oder ein Salz einer solchen Verbindung mit mindestens einer salzbildenden Gruppe erhält.

8. Verfahren nach einem der Ansprüche 1-7, dadurch gekennzeichnet, dass man die Ausgangsstoffe so wählt, dass man eine Verbindung der Formel I, worin, wenn W für einen Rest der Formel IV steht, $R^7$, wenn W für einen Rest der Formel V steht, $R^7$ und $R^9$, und, wenn W für einen Rest der Formel VI steht, $R^7$, $R^9$ und $R^{11}$ unabhängig voneinander Wasserstoff oder $C_{1-4}$-Alkyl bedeuten, oder ein Salz einer solchen Verbindung mit mindestens einer salzbildenden Gruppe erhält.

9. Verfahren nach einem der Ansprüche 1-8, dadurch gekennzeichnet, dass man die Ausgangsstoffe so wählt, dass man eine Verbindung der Formel I, worin $R^2$, $R^4$ und, wenn W für einen Rest der Formel IV steht, $R^6$, und, wenn W für einen Rest der Formel V steht, auch $R^8$ und, wenn W für einen Rest der Formel VI steht, auch $R^{10}$ Wasserstoff bedeuten, oder ein Salz einer solchen Verbindung mit mindestens einer salzbildenden Gruppe erhält.

10. Verfahren nach einem der Ansprüche 1-7 und 9, dadurch gekennzeichnet, dass man die Ausgangsstoffe so wählt, dass man eine Verbindung der Formel I, worin W einen Rest der Formel III bedeutet, oder ein Salz einer solchen Verbindung mit mindestens einer salzbildenden Gruppe erhält.

11. Verfahren nach einem der Ansprüche 1-9, dadurch gekennzeichnet, dass man die Ausgangsstoffe so wählt, dass man eine Verbindung der Formel I, worin W einen Rest der Formel IV bedeutet, oder ein Salz einer solchen Verbindung mit mindestens einer salzbildenden Gruppe erhält.

12. Verfahren nach einem der Ansprüche 1-9, dadurch gekennzeichnet, dass man die Ausgangsstoffe so wählt, dass man eine Verbindung der Formel I, worin W einen Rest der Formeln V oder VI bedeutet, oder ein Salz einer solchen Verbindung mit mindestens einer salzbildenden Gruppe erhält.

13. Verfahren nach einem der Ansprüche 1-12, dadurch gekennzeichnet, dass man die Ausgangsstoffe so wählt, dass man eine Verbindung der Formel I, die an einem asymmetrisch substituierten $\underline{C}$-$R^3$-Atom und an allen Asymmetriezentren im Rest W die (L)-Konfiguration, am C-$R^5$-Atom alternativ auch die (D)-Konfiguration aufweist, oder ein Salz einer solchen Verbindung mit mindestens einer salzbildenden Gruppe erhält.

14. Verfahren nach Anspruch 1, dadurch gekennzeichnet, dass man die Ausgangsstoffe so wählt, dass man eine Verbindung der Formel I, worin Z Trimethylen oder 2-Hydroxy-trimethylen, $R^1$, $R^3$, $R^4$ und $R^{12}$ Wasserstoff, X die Gruppe NH, W einen Rest der Formeln III oder IV, worin $R^5$ für Wasserstoff oder Methyl und $R^6$ und $R^7$ für Wasserstoff stehen, und $R^{13}$ Wasserstoff oder $C_{1-3}$-Alkyl bedeuten, wobei die Konfiguration am $\underline{C}$-$R^5$, wenn $R^5$ für Methyl steht, (D) oder (L) sein kann, erhält.

15. Verfahren nach einem der Ansprüche 1-14, dadurch gekennzeichnet, dass man die Ausgangsstoffe so wählt, dass man eine Verbindung der Formel I, worin Z Trimethylen bedeutet, oder ein Salz einer solchen Verbindung mit mindestens einer salzbildenden Gruppe erhält.

16. Verfahren nach Anspruch 1, dadurch gekennzeichnet, dass man die Ausgangsstoffe so wählt, dass man eine Verbindung der Formel I, worin

Z Trimethylen, $R^1$ Wasserstoff, X die Gruppe $NR^2$, $R^2$ und $R^3$ Wasserstoff oder $R^2$ und $R^3$ zusammen Trimethylen, $R^4$ Wasserstoff, W einen Rest der Formeln III, IV, V oder VI, worin $R^5$ für Wasserstoff oder $C_{1-3}$-Alkyl oder $R^4$ und $R^5$ zusammen für Trimethylen, $R^6$, $R^7$, $R^8$, $R^9$, $R^{10}$ und $R^{11}$ für Wasserstoff oder $R^6$ und $R^7$ zusammen für Trimethylen stehen, $R^{12}$ Wasserstoff oder $C_{1-3}$-Alkyl und $R^{13}$ Wasserstoff bedeuten, erhält.

17. Verfahren nach Anspruch 1, dadurch gekennzeichnet, dass man die Ausgangsstoffe so wählt, dass man N-[(2-Oxo-1-pyrrolidinyl)-acetyl]-glycyl-(L)-alaninamid erhält.

18. Verfahren nach Anspruch 1, dadurch gekennzeichnet, dass man die Ausgangsstoffe so wählt, dass man N-[(2-Oxo-1-pyrrolidinyl)-acetyl]-L-prolyl-glycyl-glycinamid erhält.

19. Verfahren nach Anspruch 1, dadurch gekennzeichnet, dass man die Augsgangsstoffe so wählt, dass man N-[(2-Oxo-1-pyrrolidinyl)-acetyl]-glycyl-(L)-alanin-N-monoethylamid erhält.